# EUROPEAN PATENT APPLICATION

(11) **EP 4 082 611 A1**
(43) Date of publication of application: **02.11.2022**
(21) Application number: 20895331.5
(22) Date of filing: 04.12.2020
(51) Int. Cl.: A61P 1/04, A61P 9/00, A61P 11/00, A61P 13/12, A61P 21/00, A61P 25/00, A61P 29/00, A61P 37/06, C07D 401/04, C07D 401/12, C07D 403/12, C07D 405/12, C07F 5/02, A61K 31/397, A61K 31/416, A61K 31/4427, A61K 31/45, A61K 31/472, A61K 31/498

(54) **AZETIDINE SULFONAMIDE COMPOUND**

(30) Priority: 06.12.2019 JP 2019220999
(71) Applicant: Daiichi Sankyo Company, Limited, Tokyo 103-8426 (JP)
(72) Inventor: TSUJI Takashi, Tokyo 103-8426 (JP); NAKAO Akira, Tokyo 103-8426 (JP); NAKAMURA Yuji, Tokyo 103-8426 (JP); ZHANG Zhengyu, Tokyo 103-8426 (JP); YOKOYAMA Mika, Tokyo 103-8426 (JP); IWANAMI Makoto, Tokyo 103-8426 (JP)
(74) Representative: Marks & Clerk LLP
(86) International application number: PCT/JP2020/045344
(87) International publication number: WO 2021/112251

(57) **Abstract**

An object of the present invention is to provide a compound useful as a therapeutic drug for an autoimmune disease such as systemic lupus erythematosus (SLE) and lupus nephritis of SLE patients by suppressing immune cell function by inhibiting proliferation of activated T cells and production of interferon alpha (IFN-α).

The present invention provides a compound represented by formula (I): 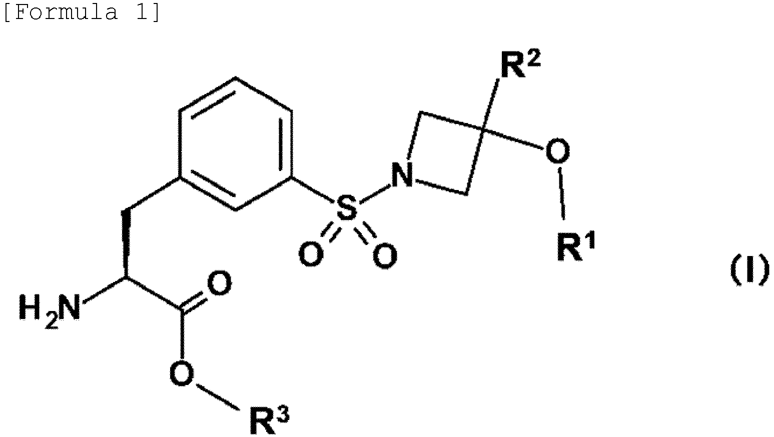 wherein R¹, R² and R³ are the same as defined in the specification,
or a pharmaceutically acceptable salt thereof.

## Description

### Technical Field

The present invention relates to a compound or a pharmaceutically acceptable salt thereof useful as a therapeutic or prophylactic drug for a disease involving the immune system, in particular, an autoimmune disease such as systemic lupus erythematosus (sometimes abbreviated herein as SLE) and lupus nephritis in SLE patients, by suppressing immune cell function by suppressing proliferation of activated T cells or production of interferon alpha (sometimes abbreviated herein as IFN-α or IFNa) by activated plasmacytoid dendritic cells (sometimes abbreviated herein as pDC).

### Background Art

SLE is a systemic autoimmune disease. There are about 1.4 million SLE patients in the world. Of these, colored races such as Asians and Hispanics are highly frequently affected. In Japan, morbidity is about 50 per 100,000 and the male-female ratio is 1:9. The numbers of patients are overwhelmingly female. The peak age of onset is 20s to 30s. Early-age onset is a characteristic feature of SLE. In identical twins, the rate of both of them developing SLE is about 30%. From these facts, it is presumed that SLE is caused by environmental factors such as ultraviolet rays, viral infection, stress and sex hormones, in addition to some underlying genetic predisposition.

SLE is classified based on the SLICC classification criteria (the Systemic Lupus International Collaborating Clinics classification criteria for systemic lupus erythematosus) published by the American College of Rheumatology in 2012. SLE is diagnosed if a total of four criteria including at least one of 11 clinical criteria and at least one of 6 immunity criteria are satisfied. Main clinical symptoms are, for example, acute and chronic skin lupus, synovitis (painful and swollen joints and stiffness), neurological symptoms and nephropathy. Main immunity criteria are, for example, increased blood levels of autoantibodies (such as antinuclear antibody, anti-dsDNA antibody, and anti-Sm antibody) and decreased blood levels of complements.

SLE is caused by the following mechanism: an immune complex formed of an autoantibody and an autoantigen is produced in the blood, and then deposits on vessel walls. Production of an autoantibody requires activation of B cells with the assistance of T cell function. In addition, it is known that interferon alpha (sometimes abbreviated herein as IFN-α or IFNa) produced from plasmacytoid dendritic cells (sometimes abbreviated herein pDC) plays an important role in the pathology of SLE. An intricate network involving various immune cells is considered to cause SLE.

Disorders of vital organs observed in SLE patients are disorders of the kidney, central nervous system and lung. Due to these disorders, life and functional prognosis are highly likely to be poor. Among them, a disorder of the kidney (nephropathy) occurs in 50% or more of SLE patients and the possibility of the patients having renal failure and resulting in death is still high. In this sense, nephropathy is an organ disorder having extremely high unmet medical needs. Patients with nephropathy are examined and diagnosed to have lupus nephritis according to the tissue classification specified by the International Society of Nephrology. If the SLE classification criteria are satisfied; and a urinary protein level persists > 0.5 g/day or 3+ or more; and/or urinary casts are observed, nephropathy is defined as lupus nephritis. Lupus nephritis is classified into types I to VI based on pathological diagnosis. Type III, IV and V lupus nephritis (active lupus nephritis) must be intensively treated by applying high-dose steroids and immunosuppressive agents in combination to achieve remission as early as possible. A first-choice drug for lupus nephritis to achieve remission is mycophenolate mofetil (sometimes abbreviated herein as MMF), which is used for about 70% of patients. To remaining patients (20 to 30%), cyclophosphamide pulse therapy is applied. MMF is an inhibitor of inosine monophosphate dehydrogenase, principally acting on lymphocytes such as T cells and B cells by suppressing purine synthesis in these cells (where purine synthesis is dependent on inosine monophosphate dehydrogenase). Cyclophosphamide inhibits DNA synthesis of lymphocytes by a similar mechanism. However, since they act by inhibiting nucleic acid synthesis, these drugs have serious side effects. MMF has serious side effects such as teratogenicity (contraindicated for pregnant women), gastrointestinal disorders and susceptibility to infection; whereas, cyclophosphamide has side effects such as carcinogenicity, bone marrow disorder, nausea/vomiting and reproductive dysfunction. As described in the above, the existing drugs have dose and use limitations due to their side effects and treatments with the existing drugs are presently not always satisfactory. Even if a sufficient treatment can be made by these drugs, the rate of response is not always high. In these circumstances, drugs acting through a new mechanism of action and drugs with higher safety are desired by patients and in medical settings.

One of the drugs developed for SLE is an antibody against IFN-α-receptor. When the antibody drug was used in Phase II trials, results showed a high therapeutic response rate (Non Patent Literature 1). IFN-α is a cytokine that has a characteristically elevated level in SLE patients, and is considered an important factor for serving as a therapeutic target. A drug that is more effective for suppressing IFN-α is desired. In Phase II clinical trials for patients with lupus nephritis, voclosporin, a drug acting as both a calcineurin inhibitor (CNI) and a T-cell-suppressor, showed a significant remission induction rate compared to a control group. This supports the validity of targeting T cells as a therapy (Non Patent Literature 2).

### Citation List

### Non Patent Literature

Non Patent Literature 1: Arthritis Rheumatol. 2017 Feb; 69(2): 376-386
Non Patent Literature 2: Dobronravov V, Dooley M, Haq S, et al. LB0002 48 week complete remission of active lupus nephritis with voclosporin. Annals of the Rheumatic Diseases 2017; 76: 153.

### Summary of Invention

### Technical Problem

The present inventors have found compounds that provide not only an action to suppress proliferation of activated T cells but also an action to suppress production of IFN-α. They considered that this profile of the compounds is highly ideal for a therapeutic drug for an immune disease, particularly an autoimmune disease, such as SLE and lupus nephritis of SLE patients. As a result of intensive studies, they found that azetidine sulfonamide derivatives having a specific chemical structure exhibit an excellent action to suppress proliferation of activated T cells and production of IFN-α, and are effective for treating immune diseases, particularly autoimmune diseases, such as SLE and lupus nephritis of SLE patients. Based on this finding, the present invention was accomplished.

### Solution to Problem

The present invention provides the following:
[1] A compound represented by formula (I): 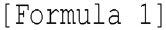 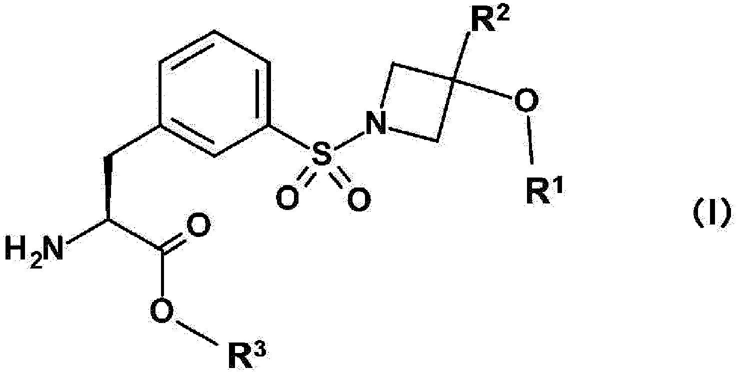 wherein
   R¹ represents a phenyl group, a monocyclic aromatic heterocyclic group or a dicyclic heterocyclic group, wherein
   the monocyclic aromatic heterocycle is a 5 to 6-membered aromatic heterocycle having 1 to 4 heteroatoms in the ring independently selected from oxygen atoms, sulfur atoms and nitrogen atoms,
   the dicyclic heterocyclic group is represented by any one of the following formulas (R¹-1A) to (R¹-1F): 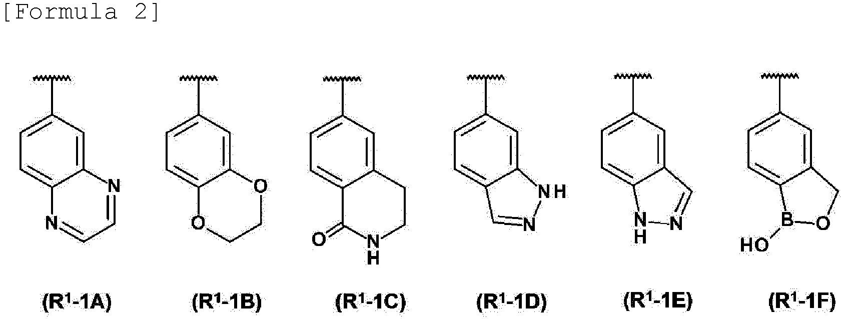 and
   the phenyl group, monocyclic aromatic heterocyclic group or dicyclic heterocyclic group optionally has 1 to 5 substituents independently selected from substituent group a;
   R² represents a phenyl group or a monocyclic aromatic heterocyclic group, wherein
   the monocyclic aromatic heterocycle is a 5 to 6-membered aromatic heterocycle having 1 to 4 heteroatoms in the ring independently selected from oxygen atoms, sulfur atoms and nitrogen atoms, and
   the phenyl group or monocyclic aromatic heterocyclic group optionally has 1 to 5 substituents independently selected from substituent group b;
   R³ represents a hydrogen atom,
   a group represented by formula (A): 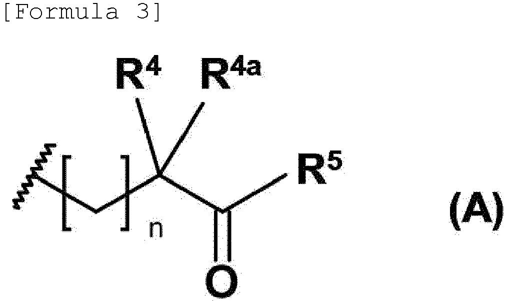 (wherein
      n represents an integer 0 or 1,
      R⁴ represents a hydrogen atom, a C₁-C₆ alkyl group or a C₃-C₆ cycloalkyl group,
      R^{4a} represents a hydrogen atom, a C₁-C₆ alkyl group or a C₃-C₆ cycloalkyl group,
      R⁵ represents a C₁-C₆ alkyl group, an amino group, a mono(C₁-C₆ alkyl)amino group, a di(C₁-C₆ alkyl)amino group or a morpholino group, wherein the C₁-C₆ alkyl group, amino group, mono (C₁-C₆ alkyl)amino group, di(C₁-C₆ alkyl)amino group or morpholino group optionally has one or two substituents independently selected from substituent group c),
      a group represented by formula (B): 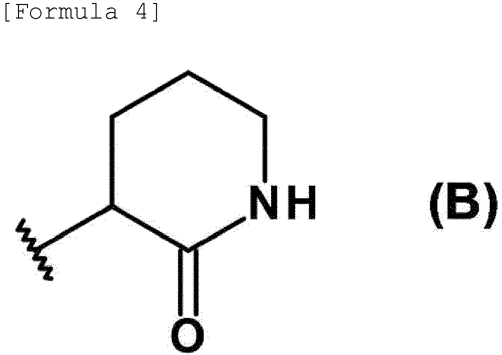 or
      a C₁-C₆ alkyl group substituted with one pyrimidinyl group;
      Substituent group a: a halogen atom, a C₁-C₆ alkyl group, a C₁-C₆ alkyl group substituted with one to three halogen atoms, a C₁-C₆ alkyl group substituted with one carbamoyl group, a C₁-C₆ alkyl group substituted with one hydroxy group, a cyano group, a carbamoyl group, a deuterium atom, a phenyl group (wherein the phenyl group is optionally substituted with one to three substituents independently selected from the group consisting of C₁-C₆ alkyl groups and halogen atoms), a hydroxy group, a C₁-C₆ alkoxy group, a C₁-C₆ alkoxy group substituted with one to three halogen atoms, a C₁-C₇ acyl group, a mono(C₁-C₆ alkyl)carbamoyl group, a di(C₁-C₆ alkyl)carbamoyl group, a (C₁-C₆ alkoxy)carbonyl group, a carboxy group, a mono (C₁-C₆ alkyl)amino group, di (C₁-C₆ alkyl)amino group and a B(OH)₂ group;
      Substituent group b: a halogen atom, a deuterium atom and a C₁-C₆ alkyl group; and
      Substituent group c: a C₁-C₆ alkoxy group and a hydroxy group;
      or a pharmaceutically acceptable salt thereof.
[2] The compound or a pharmaceutically acceptable salt thereof according to [1], wherein, in the formula (I),
   R¹ is a phenyl group or a pyridyl group, wherein
   the phenyl group or pyridyl group optionally has 1 to 5 substituents independently selected from substituent group a1;
   R² is a phenyl group or a pyridyl group, wherein
   the phenyl group or pyridyl group optionally has one or two substituents independently selected from substituent group b1; and
   R³ is a hydrogen atom or a group represented by formula (A), wherein
   n represents an integer of 0,
   R⁴ represents a hydrogen atom or a C₁-C₆ alkyl group,
   R^{4a} represents a hydrogen atom, and
   R⁵ represents a C₁-C₆ alkyl group, an amino group, a mono(C₁-C₆ alkyl)amino group or a di (C₁-C₆ alkyl)amino group, wherein the C₁-C₆ alkyl group, amino group, mono(C₁-C₆ alkyl)amino group or di(C₁-C₆ alkyl)amino group has no substituents;
   Substituent group a1: a halogen atom, a C₁-C₃ alkyl group, a cyano group, a carbamoyl group and a deuterium atom; and
   Substituent group b1: a halogen atom and a C₁-C₃ alkyl group.
[3] The compound or a pharmaceutically acceptable salt thereof according to [2], wherein, in the formula (I), R³ is a group represented by formula (A), wherein
   n represents an integer of 0,
   R⁴ represents a hydrogen atom or a C₁-C₆ alkyl group,
   R^{4a} represents a hydrogen atom, and
   R⁵ represents a C₁-C₆ alkyl group, an amino group, a mono (C₁-C₆ alkyl)amino group or a di(C₁-C₆ alkyl)amino group, wherein the C₁-C₆ alkyl group, amino group, mono(C₁-C₆ alkyl)amino group or di(C₁-C₆ alkyl)amino group has no substituents.
[4] The compound or a pharmaceutically acceptable salt thereof according to [2], wherein, in the formula (I), R³ is a group represented by formula (A), wherein
   n represents an integer of 0,
   R⁴ represents a C₃-C₆ alkyl group,
   R^{4a} represents a hydrogen atom, and
   R⁵ represents a mono(C₁-C₆ alkyl)amino group or a di(C₁-C₆ alkyl)amino group, wherein the mono(C₁-C₆ alkyl)amino group or di(C₁-C₆ alkyl)amino group has no substituents.
[5] The compound or a pharmaceutically acceptable salt thereof according to [1], wherein, in the formula (I),
   R¹ is a phenyl group, a pyridyl group or a dicyclic heterocyclic group, wherein
   the phenyl group or pyridyl group has 1 to 5 substituents independently selected from substituent group a according to [1], and at least one of the substituents is independently selected from substituent group a2, and
   the dicyclic heterocyclic group is represented by any one of formulas (R¹-1A) to (R¹-1F) and optionally has 1 to 5 substituents independently selected from substituent group a according to [1],
   Substituent group a2: a C₁-C₆ alkyl group substituted with one hydroxy group, a hydroxy group, a C₁-C₆ alkoxy group, a C₁-C₆ alkoxy group substituted with one to three halogen atoms, a C₁-C₇ acyl group, a mono(C₁-C₆ alkyl)carbamoyl group, a di(C₁-C₆ alkyl)carbamoyl group, a mono (C₁-C₆ alkyl)amino group, a di(C₁-C₆ alkyl)amino group and a B(OH)₂ group.
[6] The compound or a pharmaceutically acceptable salt thereof according to [5], wherein, in the formula (I),
   R² is a phenyl group or a pyridyl group, wherein
   the phenyl group or pyridyl group optionally has one or two substituents independently selected from substituent group b2,
   Substituent group b2: a halogen atom and a C₁-C₃ alkyl group.
[7] The compound or a pharmaceutically acceptable salt thereof according to [5] or [6], wherein, in the formula (I), R³ is a group represented by formula (A), wherein
   n represents an integer of 0,
   R⁴ represents a hydrogen atom or a C₁-C₆ alkyl group,
   R^{4a} represents a hydrogen atom, and
   R⁵ represents a C₁-C₆ alkyl group, an amino group, a mono(C₁-C₆ alkyl)amino group, a di (C₁-C₆ alkyl)amino group or a morpholino group, wherein the C₁-C₆ alkyl group, amino group, mono(C₁-C₆ alkyl)amino group, di(C₁-C₆ alkyl)amino group or morpholino group optionally has one or two substituents independently selected from substituent group c,
   Substituent group c: a C₁-C₆ alkoxy group and a hydroxy group.
[8] The compound or a pharmaceutically acceptable salt thereof according to [5] or [6], wherein, in the formula (I), R³ is a group represented by formula (A), wherein
   n represents an integer of 0,
   R⁴ represents a C₃-C₆ alkyl group,
   R^{4a} represents a hydrogen atom, and
   R⁵ represents a mono(C₁-C₆ alkyl)amino group, a di (C₁-C₆ alkyl)amino group or a morpholino group, wherein the mono(C₁-C₆ alkyl)amino group, di(C₁-C₆ alkyl)amino group or morpholino group has no substituents.
[9] The compound or a pharmaceutically acceptable salt thereof according to [1], wherein, in the formula (I), R³ is a group represented by formula (A), wherein
   n represents an integer of 0,
   R⁴ represents a hydrogen atom or a C₁-C₆ alkyl group,
   R^{4a} represents a hydrogen atom or a C₁-C₆ alkyl group, and
   R⁵ represents a C₁-C₆ alkyl group, an amino group, a mono(C₁-C₆ alkyl)amino group, a di(C₁-C₆ alkyl)amino group or a morpholino group, wherein the C₁-C₆ alkyl group, amino group, mono(C₁-C₆ alkyl)amino group or di(C₁-C₆ alkyl)amino group has one or two substituents independently selected from substituent group c, and the morpholino group optionally has one or two substituents independently selected from substituent group c,
   Substituent group c: a C₁-C₆ alkoxy group and a hydroxy group.
[10] The compound or a pharmaceutically acceptable salt thereof according to [9], wherein, in the formula (I),
   R¹ is a phenyl group or a pyridyl group, wherein
   the phenyl group or pyridyl group optionally has 1 to 5 substituents independently selected from substituent group a3; and
   R² is a phenyl group or a pyridyl group, wherein
   the phenyl group or pyridyl group optionally has one or two substituents independently selected from substituent group b3,
   Substituent group a3: a halogen atom, a C₁-C₃ alkyl group, a cyano group, a carbamoyl group and a deuterium atom; and
   Substituent group b3: a halogen atom and a C₁-C₃ alkyl group.
[11] A medicament comprising the compound or a pharmaceutically acceptable salt thereof according to any one of [1] to [10].
[12] A suppressor of proliferation of activated T cells, comprising the compound or a pharmaceutically acceptable salt thereof according to any one of [1] to [10] .
[13] A suppressor of production of interferon α, comprising the compound or a pharmaceutically acceptable salt thereof according to any one of [1] to [10].
[14] A therapeutic or prophylactic agent for a disease a symptom of which is prevented, ameliorated or mitigated by suppressing proliferation of activated T cells or suppressing production of interferon α by activated pDC (plasmacytoid dendritic cells), comprising the compound or a pharmaceutically acceptable salt thereof according to any one of [1] to [10].
[15] A therapeutic or prevention method for a disease a symptom of which is prevented, ameliorated or mitigated by suppressing proliferation of activated T cells or suppressing production of interferon α by activated pDC (plasmacytoid dendritic cells), comprising administering the compound or a pharmaceutically acceptable salt thereof according to any one of [1] to [10], to a patient.
[16] The compound or a pharmaceutically acceptable salt thereof according to any one of [1] to [10] for use in treating or preventing a disease a symptom of which is prevented, ameliorated or mitigated by suppressing proliferation of activated T cells or suppressing production of interferon α by activated pDC (plasmacytoid dendritic cells).
[17] The therapeutic or prophylactic agent according to [14], wherein the disease is an autoimmune disease.
[18] The therapeutic or prevention method according to [15], wherein the disease is an autoimmune disease.
[19] The compound or a pharmaceutically acceptable salt thereof according to [16], wherein the disease is an autoimmune disease.
[20] A therapeutic or prophylactic agent for systemic lupus erythematosus; a renal disease associated with systemic lupus erythematosus, central nervous system disorder, lung disorder or vasculitis; polymyositis; ulcerative colitis; Sjogren's syndrome; graft-versus-host disease (GVHD) or psoriasis, comprising the compound or a pharmaceutically acceptable salt according to any one of [1] to [10].
[21] A method for treating or preventing systemic lupus erythematosus; a renal disease associated with systemic lupus erythematosus, central nervous system disorder, lung disorder or vasculitis; polymyositis; ulcerative colitis; Sjogren's syndrome; graft-versus-host disease (GVHD) or psoriasis, comprising administering the compound or a pharmaceutically acceptable salt according to any one of [1] to [10] to a patient.
[22] The compound or a pharmaceutically acceptable salt according to any one of [1] to [10] for use in treating or preventing systemic lupus erythematosus, a renal disease associated with systemic lupus erythematosus, central nervous system disorder, lung disorder or vasculitis; polymyositis; ulcerative colitis; Sjogren's syndrome; graft-versus-host disease (GVHD) or psoriasis.

In the present invention, the "halogen atom" refers to a fluorine atom, a chlorine atom, a bromine atom or an iodine atom, and preferably, a fluorine atom or a chlorine atom.

In the present invention, the "C₁-C₆ alkyl group" refers to a linear or branched alkyl group having 1 to 6 carbon atoms, for example, methyl, ethyl, propyl, isopropyl, butyl, isobutyl, s-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl or 1,2-dimethylbutyl group; preferably, a linear or branched alkyl group (C₁-C₄ alkyl group) having 1 to 4 carbon atoms; more preferably, a methyl group or an ethyl group (C₁ or C₂ alkyl group); and further preferably, a methyl group (C₁ alkyl group).

In the present invention, the "C₁-C₃ alkyl group" refers to a linear or branched alkyl group having 1 to 3 carbon atoms, for example, methyl, ethyl, propyl or isopropyl group; preferably, a methyl group or ethyl group (C₁ or C₂ alkyl group); and more preferably, a methyl group (C₁ alkyl group).

In the present invention, the "C₃-C₆ alkyl group" refers to a linear or branched alkyl group having 3 to 6 carbon atoms, for example, propyl, isopropyl, butyl, isobutyl, s-butyl, t-butyl, pentyl, isopentyl, 2-methylbutyl, neopentyl, 1-ethylpropyl, hexyl, 4-methylpentyl, 3-methylpentyl, 2-methylpentyl, 1-methylpentyl, 3,3-dimethylbutyl, 2,2-dimethylbutyl, 1,1-dimethylbutyl or 1,2-dimethylbutyl group; preferably, a linear or branched alkyl group (C₃ or C₄ alkyl group) having carbon atoms 3 or 4; more preferably, propyl, isopropyl, butyl, isobutyl, s-butyl or t-butyl group; and further preferably, an isopropyl group or a t-butyl group.

In the present invention, the "C₃-C₆ cycloalkyl group" refers to a cyclopropyl group, a cyclobutyl group, a cyclopentyl group or a cyclohexyl group; and preferably, a cyclopropyl group or a cyclohexyl group.

In the present invention, the "C₁-C₆ alkyl group substituted with one to three halogen atoms" refers to a "C₁-C₆ alkyl group" to which the same or different 1 to 3 "halogen atoms" are bound, for example, fluoromethyl, difluoromethyl, dichloromethyl, trifluoromethyl, trichloromethyl, 2,2,2-trifluoroethyl, 3,3,3-trifluoropropyl or 2,2,2-trichloroethyl group; preferably, a "C₁ or C₂ alkyl group" to which the same or different 2 or 3 "halogen atoms" are bound (dihalo C₁ or C₂ alkyl group or trihalo C₁ or C₂ alkyl group); more preferably, a dihalomethyl group (dihalo C₁ alkyl group); and further preferably, a difluoromethyl group.

In the present invention, a "C₁-C₆ alkyl group substituted with one carbamoyl group" refers to a "C₁-C₆ alkyl group" to which one carbamoyl group (-C(=O)-NH₂ group) is bound, for example, carbamoylmethyl, 1-carbamoylethyl, 2-carbamoylethyl, 1-carbamoylpropyl, 2-carbamoylpropyl or 3-carbamoylpropyl; preferably, 1-carbamoylethyl.

In the present invention, the "C₁-C₆ alkyl group substituted with one hydroxy group" refers to a "C₁-C₆ alkyl group" to which one hydroxy group is bound, for example, hydroxymethyl, 1-hydroxyethl, 2-hydroxyethyl, 1-hydroxypropyl, 2-hydroxypropyl or 3-hydroxypropyl; preferably, 1-hydroxyethyl.

In the present invention, the "C₁-C₆ alkyl group substituted with one pyrimidinyl group" refers to a group obtained by binding one pyrimidinyl group to a "C₁-C₆ alkyl group" as mentioned above, for example, 2-pyrimidinylmethyl or 2-pyrimidinyl ethyl.

In the present invention, the "C₁-C₆ alkoxy group" refers to a group obtained by binding a "C₁-C₆ alkyl group" (mentioned above) to an oxygen atom, for example, methoxy, ethoxy, propoxy, isopropoxy, butoxy, isobutoxy, s-butoxy, t-butoxy, pentoxy, 2-methylbutoxy, 3-ethylpropoxy, neopentoxy, hexyloxy or 2,3-didimethylbutoxy group; preferably, a linear or branched alkoxy group (C₁-C₄ alkoxy group) having 1 to 4 carbon atoms; more preferably, a methoxy group or an ethoxy group (C₁ or C₂ alkoxy group); and further preferably, a methoxy group (C₁ alkoxy group).

In the present invention, the "C₁-C₆ alkoxy group substituted with one to three halogen atoms" refers to a "C₁-C₆ alkoxy group" to which the same or different 1 to 3 "halogen atoms" are bound, for example, fluoromethyloxy, difluoromethyloxy, trifluoromethyloxy, trichloromethyloxy, 2,2,2-trifluoroethyloxy, 3,3,3-trifluoropropyloxy or 2,2,2-trichloroethyloxy group; preferably, a "C₁ or C₂ alkoxy group" to which the same or different three "halogen atoms" are bound (trihalo C₁ or C₂ alkoxy group); more preferably, a trihalomethyloxy group (trihalo C₁ alkoxy group); and further preferably, a trifluoromethyloxy group.

In the present invention, the "C₁-C₇ acyl group" refers to a group obtained by binding a hydrogen atom or the "C₁-C₆ alkyl group" to a carbonyl group, for example, formyl, acetyl, propionyl, butyryl, isobutyryl, valeryl, isovaleryl, pivaloyl, hexanoyl or heptanoyl group, preferably, an acetyl group or a propionyl group (C₂ or C₃ acyl group).

In the present invention, the "mono(C₁-C₆ alkyl)amino group" refers to an amino group to which one "C₁-C₆ alkyl group" (mentioned above) is bound; for example, methylamino, ethylamino, propylamino, isopropylamino or butylamino group; preferably, an amino group to which one "C₁-C₄ alkyl group" is bound (mono (C₁-C₄ alkyl) amino group); and more preferably, a methylamino group, an ethylamino group, a propylamino group or an isopropylamino group (mono (C₁-C₃ alkyl) amino group).

In the present invention, the "di(C₁-C₆ alkyl)amino group" refers to an amino group to which the same or different two "C₁-C₆ alkyl groups" (mentioned above) are bound, for example, dimethylamino, diethylamino, dipropylamino, N-ethyl-N-methylamino, N-methyl-N-propylamino or N-butyl-N-methylamino group; preferably, an amino group to which the same or different two "C₁-C₄ alkyl groups" are bound (di(C₁-C₄ alkyl)amino group); more preferably, a dimethylamino group, a diethylamino group or a N-ethyl-N-methylamino group (di(C₁ or C₂ alkyl)amino group); and further preferably, a dimethylamino group or a diethylamino group.

In the present invention, the "mono(C₁-C₆ alkyl)carbamoyl group" refers to a carbamoyl group to which one "C₁-C₆ alkyl group" (mentioned above) is bound, for example, methylcarbamoyl, ethylcarbamoyl, propylcarbamoyl, isopropylcarbamoyl or butylcarbamoyl group; preferably, a carbamoyl group to which one "C₁-C₄ alkyl group" is bound (mono(C₁-C₄ alkyl)carbamoyl group); and more preferably, a methylcarbamoyl group or an ethylcarbamoyl group (mono(C₁ or C₂ alkyl)carbamoyl group) .

In the present invention, the "di (C₁-C₆ alkyl)carbamoyl group" refers to a carbamoyl group to which the same or different two "C₁-C₆ alkyl groups" (mentioned above) are bound, for example, dimethylcarbamoyl, diethylcarbamoyl, dipropylcarbamoyl, N-ethyl-N-methylcarbamoyl, N-methyl-N-propylcarbamoyl or N-butyl-N-methylcarbamoyl group; preferably, a carbamoyl group to which the same or different two "C₁-C₄ alkyl groups" are bound (di(C₁-C₄ alkyl)carbamoyl group); more preferably, a dimethylcarbamoyl group, a diethylcarbamoyl group or a N-ethyl-N-methylcarbamoyl group (di(C₁ or C₂ alkyl)carbamoyl group); and further preferably, a dimethylcarbamoyl group.

In the present invention, the "(C₁-C₆ alkoxy)carbonyl group" refers to a group obtained by binding "C₁-C₆ alkoxy group" to a carbonyl group, for example, methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentoxycarbonyl, 2-methylbutoxycarbonyl, 3-ethylpropoxycarbonyl, neopentoxycarbonyl, hexyloxycarbonyl or 2,3-dimethylbutoxycarbonyl group; preferably, a linear or branched alkoxycarbonyl group ((C₁-C₄ alkoxy)carbonyl group) having 1 to 4 carbon atoms; more preferably, a methoxycarbonyl group or an ethoxycarbonyl group ((C₁ or C₂ alkoxy)carbonyl group); and further preferably, a methoxycarbonyl group ((C₁ alkoxy)carbonyl group).

In the present invention, the "5 to 6-membered aromatic heterocycle having 1 to 4 heteroatoms in the ring independently selected from oxygen atoms, sulfur atoms and nitrogen atoms " refers to a 5 or 6 membered monocyclic aromatic compound having 1 to 4 heteroatoms (nitrogen atom, oxygen atom or sulfur atom) as constituent atoms of the ring in addition to a carbon atom, for example, pyrrole, pyrazole, imidazole, furan, thiophene, oxazole, isoxazole, thiazole, isothiazole, pyridine, pyrimidine, pyridazine, pyrazine, triazine, pyran, 1,2-oxazine, 1,3-oxazine, 1,4-oxazine, 1,2-thiazine, 1,3-thiazine or 1,4-thiazine; preferably, a 5 or 6 membered monocyclic aromatic compound having 1 or 2 heteroatoms (nitrogen atom, oxygen atom or sulfur atom, preferably, nitrogen atom) in the ring; and more preferably, pyridine or pyrazole.

Now, three preferable embodiments of the compound of the present invention will be described.

### [First embodiment of the compound of the present invention]

In the present invention of the first embodiment, the group represented by R¹ is preferably a phenyl group, pyridyl group or pyrazolyl group which optionally has 1 to 5 substituents independently selected from substituent group a1.

Substituent group a1: a halogen atom, a C₁-C₃ alkyl group, a cyano group, a carbamoyl group and a deuterium atom.

Substituent group a1 is preferably the group consisting of a fluorine atom, a chlorine atom, a bromine atom, a methyl group, an ethyl group, a cyano group, a carbamoyl group and a deuterium atom, and more preferably, the group consisting of a fluorine atom, a chlorine atom and a deuterium atom.

The group represented by R¹ is, more preferably, a phenyl group or pyridyl group which optionally has 1 to 5 substituents independently selected from substituent group a1.

The group represented by R¹ is, further more preferably, a phenyl group substituted with 1 or 2 halogen atoms (preferably, fluorine atom or chlorine atom) or a phenyl group substituted with 5 deuterium atoms.

In the present invention of the first embodiment, the group represented by R² is preferably a phenyl group or pyridyl group which optionally has 1 or 2 substituents independently selected from substituent group b1.

Substituent group b1: a halogen atom and a C₁-C₃ alkyl group.

Substituent group b1 is preferably the group consisting of a fluorine atom, a chlorine atom and a methyl group.

The group represented by R² is more preferably a phenyl group or pyridyl group which is optionally substituted with one halogen atom, and further more preferably a phenyl group or pyridyl group substituted with one fluorine atom.

In the present invention of the first embodiment, the group represented by R³ is preferably a hydrogen atom or a group represented by formula (A):

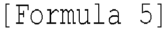

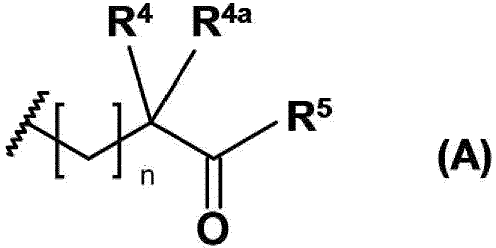

In formula (A),
n represents an integer of 0,
R⁴ represents a hydrogen atom or a C₁-C₆ alkyl group,
R^{4a} represents a hydrogen atom,
R⁵ represents a C₁-C₆ alkyl group, an amino group, a mono (C₁-C₆ alkyl)amino group or a di(C₁-C₆ alkyl)amino group, wherein the C₁-C₆ alkyl group, amino group, mono(C₁-C₆ alkyl)amino group or di(C₁-C₆ alkyl)amino group has no substituents.
The group represented by R³ is more preferably a group represented by formula (A).

In formula (A) herein, the group represented by R⁴ is preferably a C₃-C₆ alkyl group. Examples of the C₃-C₆ alkyl group are as described above. The group represented by R⁴ is more preferably a linear or branched alkyl group (C₃ or C₄ alkyl group) having 3 or 4 carbon atoms, further preferably, propyl, isopropyl, butyl, isobutyl, s-butyl or t-butyl group, and further more preferably, an isopropyl group or a t-butyl group.

In formula (A), the group represented by R⁵ is preferably a C₁-C₆ alkyl group (examples thereof are the same as described above), an amino group, a mono(C₁-C₆ alkyl)amino group (examples thereof are the same as described above) or a di(C₁-C₆ alkyl)amino group (examples thereof are the same as described above); further preferably, a mono(C₁-C₆ alkyl)amino group or a di(C₁-C₆ alkyl)amino group; and further more preferably, a mono-isopropylamino group, a mono-t-butylamino group, a dimethylamino group or a diethylamino group.

### [Second embodiment of the compound of the present invention]

In the present invention according to the second embodiment, the group represented by R¹ is preferably a phenyl group, a pyridyl group or a dicyclic heterocyclic group. The phenyl group or pyridyl group herein has 1 to 5 substituents independently selected from substituent group a. At least one of the substituents is independently selected from substituent group a2. The dicyclic heterocyclic group herein is represented by any one of formula (R¹-1A) to formula (R¹-1F):

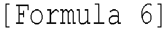

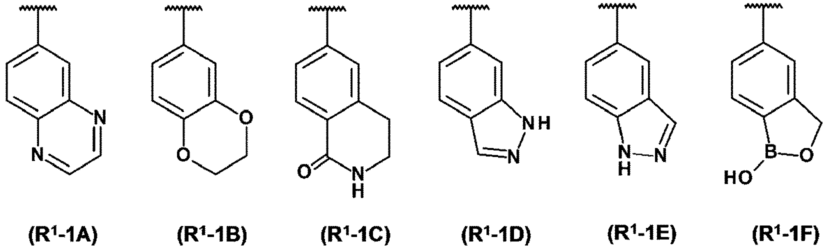

The dicyclic heterocyclic group optionally has 1 to 5 substituents independently selected from substituent group a.

Substituent group a: a halogen atom, a C₁-C₆ alkyl group, a C₁-C₆ alkyl group substituted with one to three halogen atoms, a C₁-C₆ alkyl group substituted with one carbamoyl group, a C₁-C₆ alkyl group substituted with one hydroxy group, a cyano group, a carbamoyl group, a deuterium atom, a phenyl group (wherein the phenyl group is optionally substituted with one to three substituents independently selected from C₁-C₆ alkyl groups and halogen atoms), a hydroxy group, a C₁-C₆ alkoxy group, a C₁-C₆ alkoxy group substituted with one to three halogen atoms, a C₁-C₇ acyl group, a mono(C₁-C₆ alkyl)carbamoyl group, a di (C₁-C₆ alkyl)carbamoyl group, a (C₁-C₆ alkoxy)carbonyl group, a carboxy group, a mono(C₁-C₆ alkyl)amino group, a di(C₁-C₆ alkyl)amino group and a B(OH)₂ group.

Substituent group a2: a C₁-C₆ alkyl group substituted with one hydroxy group, a hydroxy group, a C₁-C₆ alkoxy group, a C₁-C₆ alkoxy group substituted with one to three halogen atoms, a C₁-C₇ acyl group, a mono(C₁-C₆ alkyl)carbamoyl group, a di(C₁-C₆ alkyl)carbamoyl group, a mono (C₁-C₆ alkyl)amino group, a di(C₁-C₆ alkyl)amino group and a B(OH)₂ group.

Substituent group a2 preferably consists of a 1-hydroxyethyl group, a methoxy group, an ethoxy group, a difluoromethoxy group, an acetyl group, a propionyl group, a monoisopropylcarbamoyl group, a dimethylamino group and a B(OH)₂ group.

The dicyclic heterocyclic group is preferably represented by formula (R¹-1A), formula (R¹-1B) or formula (R¹-1C).

In the present invention according to the second embodiment, preferable groups represented by R² are the same as those mentioned in the present invention of the first embodiment.

In the present invention according to the second embodiment, the group represented by R³ is preferably a group represented by formula (A):

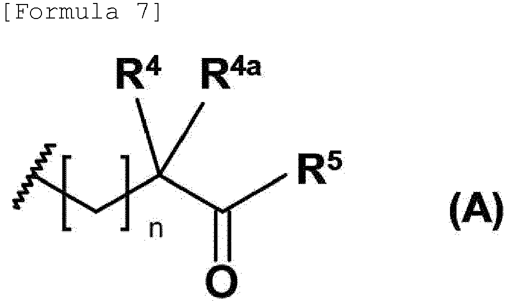

In formula (A),
n represents an integer of 0,
R⁴ represents a hydrogen atom or a C₁-C₆ alkyl group,
R^{4a} represents a hydrogen atom,
R⁵ represents a C₁-C₆ alkyl group, an amino group, a mono(C₁-C₆ alkyl)amino group, a di (C₁-C₆ alkyl)amino group or a morpholino group, wherein the C₁-C₆ alkyl group, amino group, mono(C₁-C₆ alkyl)amino group, di(C₁-C₆ alkyl)amino group or morpholino group optionally has one or two substituents independently selected from substituent group c,
Substituent group c: a C₁-C₆ alkoxy group and a hydroxy group.

In formula (A) herein, the group represented by R⁴ is preferably a C₃-C₆ alkyl group. Examples of the C₃-C₆ alkyl group are the same as described above. The group represented by R⁴ is more preferably a linear or branched alkyl group (C₃ or C₄ alkyl group) having 3 or 4 carbon atoms, further preferably is propyl, isopropyl, butyl, isobutyl, s-butyl or t-butyl group, and further more preferably, an isopropyl group or a t-butyl group.

In formula (A), the group represented by R⁵ is preferably a C₁-C₆ alkyl group (examples thereof are the same as described above), an amino group, a mono(C₁-C₆ alkyl)amino group (examples thereof are the same as described above), a di(C₁-C₆ alkyl)amino group (examples thereof are the same as described above) or a morpholino group. The group represented by R⁵ is, further preferably, a mono(C₁-C₆ alkyl)amino group, a di(C₁-C₆ alkyl)amino group or a morpholino group, and further more preferably, a mono-isopropylamino group, a mono t-butyl amino group, a dimethylamino group, a diethylamino group or a morpholino group.

Substituent group c preferably consists of a methoxy group, an ethoxy group and a hydroxy group.

### [Third embodiment of the compound of the present invention]

In the present invention according to the third embodiment, preferable groups represented by R¹ are the same as those mentioned in the present invention of the first embodiment and the present invention of the second embodiment.

In the present invention according to the third embodiment, preferable groups represented by R² are the same as those as mentioned in the present invention of the first embodiment and the present invention of the second embodiment.

In the present invention according to the third embodiment, a preferable group represented by R³ is a group represented by formula (A):

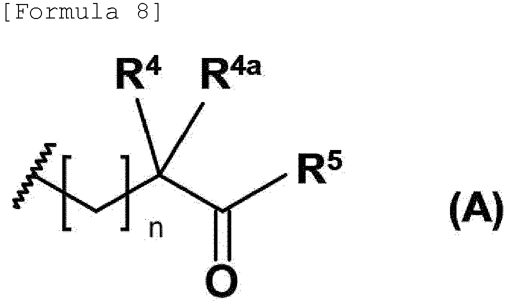

In formula (A),
n represents an integer of 0,
R⁴ represents a hydrogen atom or a C₁-C₆ alkyl group,
R^{4a} represents a hydrogen atom or a C₁-C₆ alkyl group, and
R⁵ represents a C₁-C₆ alkyl group, an amino group, a mono(C₁-C₆ alkyl)amino group, a di(C₁-C₆ alkyl)amino group or a morpholino group, wherein the C₁-C₆ alkyl group, amino group, mono (C₁-C₆ alkyl) amino group or di(C₁-C₆ alkyl)amino group has one or two substituents independently selected from substituent group c, and the morpholino group optionally has one or two substituents independently selected from substituent group c,
Substituent group c: a C₁-C₆ alkoxy group and a hydroxy group.

In formula (A), herein, the group represented by R⁴ is preferably a C₃-C₆ alkyl group. Examples of the C₃-C₆ alkyl group are the same as described above. The group represented by R⁴ is more preferably, a linear or branched alkyl group (C₃ or C₄ alkyl group) having 3 or 4 carbon atoms, further preferably, propyl, isopropyl, butyl, isobutyl, s-butyl or t-butyl group, and further more preferably, an isopropyl group or a t-butyl group.

In formula (A), the group represented by R⁵ is preferably a C₁-C₆ alkyl group (examples thereof are the same as described above), an amino group, a mono(C₁-C₆ alkyl)amino group (examples thereof are the same as described above), a di(C₁-C₆ alkyl)amino group (examples thereof are the same as described above) or a morpholino group, further preferably, a mono(C₁-C₆ alkyl)amino group, a di(C₁-C₆ alkyl)amino group or a morpholino group, and further more preferably a mono-isopropylamino group, a mono-t-butyl amino group, a dimethylamino group, a diethylamino group or a morpholino group.

Substituent group c is preferably a group consisting of a methyl group, an ethyl group and a hydroxy group.

A compound represented by the formula (I), in which R³ represents a group except a hydrogen atom is a prodrug (herein sometimes referred to as a prodrug form). The prodrug is metabolized *in vivo* and converted into a compound (herein sometimes referred to as an active form) in which R³ represents a hydrogen atom to produce a medicinal effect (see, Test Example 6).

A compound represented by formula (I) of the present invention or a pharmaceutically acceptable salt thereof may have all isomers (e.g., keto-enol isomer, diastereoisomer, optical isomer, rotational isomer).

A compound represented by formula (I) of the present invention or a pharmaceutically acceptable salt thereof may have various types of isomers if an asymmetric carbon atom exists within a molecule. The compound of the present invention including all of these isomers and a mixture thereof is represented by a single formula, that is, formula (I). Therefore, the present invention includes all of these isomers and mixtures containing these isomers in any ratio. However, in formula (I), a carbon atom to which R³-O-C(=O)- is bound, has a steric structure exactly represented by the formula.

Stereoisomers as described above can be obtained by synthesizing a compound according to the present invention from an optically active compound as the starting material or by use of asymmetric synthesis or asymmetric induction, or isolating the compound according to the present invention synthesized by means of an ordinary optical resolution method or separation method as desired.

A compound represented by formula (I) of the present invention or a pharmaceutically acceptable salt thereof may contain isotopes of one or more constituent atoms in a non-naturally occurring proportion. As atomic isotopes, for example, deuterium (²H), tritium (³H), iodine-125 (¹²⁵I) and carbon-14 (¹⁴C) are mentioned. The compound mentioned above may be labeled with a radioisotope such as tritium (³H), iodine-125 (¹²⁵I) or carbon-14 (¹⁴C). The radiolabeled compound is useful as a therapeutic or prophylactic agent, a research reagent including an assay reagent and a diagnostic agent such as an *in-vivo* diagnostic imaging agent. All isotopes (atomic variants) of the compound of the present invention, whether they are radioactive or not, are included in the scope of the present invention.

The "pharmaceutically acceptable salt thereof" refers to a salt having no significant toxicity and available as a medicament. A compound represented by formula (I) of the present invention having a basic group is allowed to react with an acid to form a salt. A compound represented by formula (I) of the present invention having an acidic group is allowed to react with a base to form a salt.

Examples of salts based on a basic group include, but are not limited to, inorganic acid salts including halide acid salts such as a hydrofluoric acid salt, a hydrochloride, a hydrobromide and a hydroiodide, a nitrate, a perchlorate, a sulfate and a phosphate; organic acid salts including C₁-C₆ alkyl sulfonates such as a methanesulfonate, a trifluoromethanesulfonate and an ethane sulfonate, aryl sulfonates such as a benzene sulfonate and a p-toluene sulfonate, an acetate, a phosphate, a fumarate, a succinate, a citrate, an ascorbate, a tartrate, an oxalate and a maleate; and amino acid salts such as a glycine salt, a lysine salt, an arginine salt, an ornithine salt, a glutamic acid salt and a aspartic acid salt.

Examples of salts based on an acid group include, but are not limited to, alkali metal salts such as a sodium salt, a potassium salt and a lithium salt; alkaline earth metal salts such as a calcium salt and a magnesium salt; metal salts such as an aluminum salt and an iron salt; inorganic salts such as a ammonium salt; organic amine salts such as a tert-butylamine salt, a tert-octylamine salt, a dibenzylamine salt, a morpholine salt, a glucosamine salt, a phenylglycine alkyl ester salt, an ethylenediamine salt, a N-methylglucamine salt, a guanidine salt, a diethylamine salt, a triethylamine salt, a dicyclohexylamine salt, N,N'-dibenzylethylenediamine salt, a chloroprocaine salt, a procaine salt, a diethanolamine salt, a N-benzylphenethylamine salt, a piperazine salt, a tetramethylammonium salt, a tris(hydroxymethyl)aminomethane salt; and amino acid salts such as a glycine salt, a lysine salt, an arginine salt, an ornithine salt, a glutamic acid salt and an aspartic acid salt.

A compound represented by formula (I) of the present invention or a pharmaceutically acceptable salt thereof, if it is allowed to remain in the air or recrystallized, may sometimes absorb water or incorporate water molecules to form a hydrate. Such hydrates are included in a pharmaceutically acceptable salt of the present invention.

A compound represented by formula (I) of the present invention or a pharmaceutically acceptable salt thereof, if it is allowed to remain in a solvent or recrystallized in a solvent, may sometimes absorb the solvent to form a solvate. Such solvates are included in a salt of the present invention.

The solvent that can form a solvate is not particularly limited as long as it has no significant toxicity and can be used as a medicament. Examples of the solvents include ethanol, 1-propanol, 2-propanol, 1-butanol, 2-butanol, acetone, methyl ethyl ketone, methyl isobutyl ketone, dimethyl sulfoxide, ethyl formate, ethyl acetate, propyl acetate, diethyl ether, tetrahydrofuran, formic acid, acetic acid, pentane, heptane, cumene and anisole.

### Advantageous Effects of Invention

A compound represented by formula (I) of the present invention or a pharmaceutically acceptable salt thereof suppresses proliferation of activated T cells and suppresses production of IFN-α by a pDC activator. Accordingly, the compound of the present invention or a pharmaceutically acceptable salt thereof is useful as a therapeutic or prophylactic agent for a disease the symptom of which can be prevented, ameliorated or mitigated by suppressing proliferation of activated T cells or production of IFN-α by activated pDC. As the disease, a disease involving the immune system, particularly an autoimmune disease, is mentioned. Examples of diseases involving the immune system include systemic lupus erythematosus; a renal disease associated with systemic lupus erythematosus, central nervous system disorder, lung disorder or vasculitis; polymyositis; ulcerative colitis; Sjogren's syndrome; rejection associated with organ transplantation, graft-versus-host disease (GVHD); dry eye; rheumatoid arthritis; Crohn's disease; psoriasis; interstitial cystitis; dermatomyositis; atopic dermatitis; systemic scleroderma; and type I diabetes. Preferably, as the disease, SLE; a renal disease associated with systemic lupus erythematosus, central nervous system disorder, lung disorder or vasculitis; polymyositis; ulcerative colitis; Sjogren's syndrome; and graft-versus-host disease (GVHD) or psoriasis, are mentioned. Particularly preferably, e.g., SLE and a renal disease associated with systemic lupus erythematosus (lupus nephritis) are mentioned. A compound represented by formula (I) of the present invention or a pharmaceutically acceptable salt thereof suppresses proliferation of activated T cells or suppresses production of IFN-α by activated pDC, thereby suppressing immune cell function, with the result that it produces a therapeutic and prophylactic effect on a disease involving the immune system.

### Brief Description of Drawings

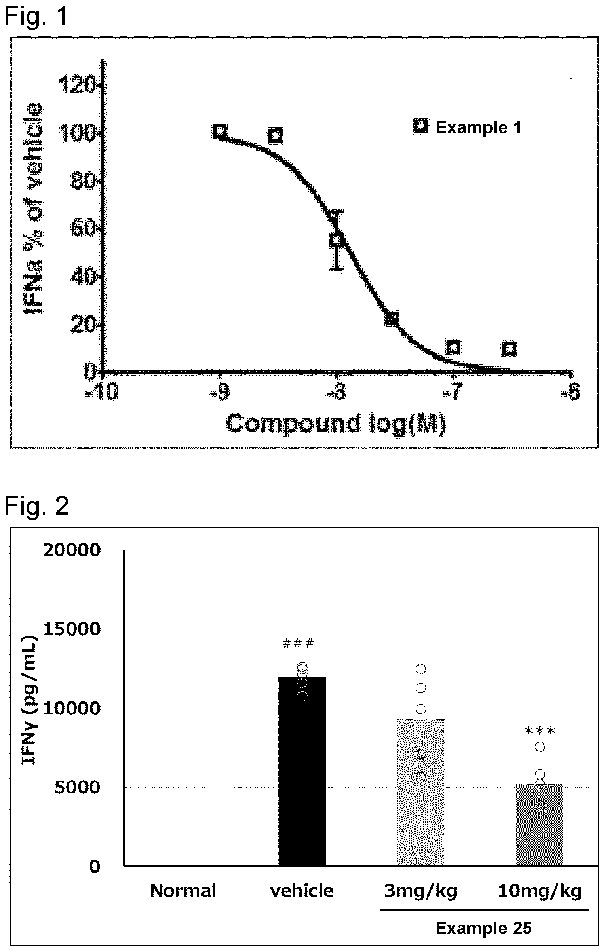

[Figure 1] Figure 1 shows the effect of a test substance (the compound of Example 1) on production of IFN-α (represented as IFNa in the figure) when mouse spleen cells were stimulated by CpG-ODN D35, a ligand of Toll-like receptor 9 (TLR9). The concentration of test substance added is 1 nM to 300 nM. The graph represents a curve showing response to concentration of a test substance, with the IFN-α production of a group not containing a test substance being specified as 100% and the IFN-α production of a group not stimulated being specified as 0%. Each plot shows an average value of samples (N = 3) with a standard deviation.
[Figure 2] Figure 2 is a graph showing the effect of the compound of Example 25 in a mouse acute GVHD model. The compound of Example 25 was orally administered in a dose of 3 or 10 mg/kg, twice a day (b.i.d.). The vertical axis represents the concentration of interferon gamma (sometimes abbreviated herein as IFN-γ or IFN-g) in the blood on day 7 after administration of the compound. A bar represents an average value of each group (N = 5), whereas plots represent individual values. As the result of the Dunnett multiple comparison test to Vehicle group, a group showing p value of 0.001 or less is indicated by ***. As the result of the Student's t test applied to a non-administration group (Normal group) and the vehicle group, a group showing p value of 0.001 or less is indicated by ###.

### Description of Embodiments

The compound of the present invention can be produced by various processes. The following process shown below is one of these and should not be construed as limiting the present invention. A compound represented by formula (I) and an intermediate thereof can be produced by use of various reactions described below. During the process, functional groups of a starting material or intermediates are sometimes protected by an appropriate protective group. Examples of the functional groups include a hydroxy group, a carboxy group and an amino group. With respect to the types of protective groups and conditions for introducing/removing protective groups, see, for example, T. W. Green, P. G. M. Wuts, Protective Groups in Organic Synthesis, Fourth Edition, 2007, John Wiley & Sons, Inc.

### [General process]

A typical process for producing a compound represented by formula (I) of the present invention will be described.

Note that, the solvents to be used for reactions in individual steps of the process shown below are not particularly limited as long as they do not inhibit the reactions and partially dissolve starting materials, and are, for example, selected from the following group of solvents. Examples of the solvents of the group include aliphatic hydrocarbons such as hexane, pentane, heptane, petroleum ether and cyclohexane; aromatic hydrocarbons such as toluene, benzene and xylene; halogenated hydrocarbons such as dichloromethane, chloroform, carbon tetrachloride, dichloroethane, chlorobenzene and dichlorobenzene; ethers such as diethyl ether, diisopropyl ether, cyclopentyl methyl ether, t-butyl methyl ether, tetrahydrofuran, 1,4-dioxane, dimethoxyethane and diethylene glycol dimethyl ether; ketones such as acetone, methyl ethyl ketone, methyl isobutyl ketone and cyclohexanone; esters such as methyl acetate, ethyl acetate, propyl acetate, butyl acetate and diethyl carbonate; nitriles such as acetonitrile, propionitrile, butyronitrile and isobutyronitrile; organic acids such as formic acid, acetic acid, propionic acid, trifluoro acetic acid, and pentafluoropropionic acid; alcohols such as methanol, ethanol, 1-propanol, 2-propanol, 1-butanol, 2-butanol, 2-methyl-1-propanol and 2-methyl-2-propanol; amides such as formamide, N,N-dimethylformamide, N,N-dimethyl acetamide, N-methylpyrrolidone, N,N'-dimethylpropylene urea and hexamethylphosphorotriamide; sulfoxides such as dimethyl sulfoxide and sulfolane; water; and mixtures of these.

The acids to be used for reactions in individual steps of the process shown below are not particularly limited as long as they do not inhibit the reactions, and may be selected from the following group of acids. Examples of the group of acids include inorganic acids such as hydrochloric acid, hydrobromic acid, hydroiodic acid, phosphoric acid, sulfuric acid and nitric acid; organic acids such as formic acid, acetic acid, propionic acid, trifluoro acetic acid and pentafluoropropionic acid; and organic sulfonic acids such as methanesulfonic acid, trifluoromethanesulfonic acid, p-toluenesulfonic acid and camphorsulfonic acid.

The bases to be used for reactions in individual steps of the process shown below are not particularly limited as long as they do not inhibit the reactions and may be selected from the following group of bases. Examples of the group of bases include alkali metal carbonates such as lithium carbonate, sodium carbonate, potassium carbonate and cesium carbonate; alkali metal hydrogen carbonates such as lithium hydrogen carbonate, sodium hydrogen carbonate and potassium hydrogen carbonate; alkali metal hydroxides such as lithium hydroxide, sodium hydroxide and potassium hydroxide; alkaline earth metal hydroxides such as calcium hydroxide and barium hydroxide; alkali metal phosphates such as sodium phosphate and potassium phosphate; alkali metal hydrides such as lithium hydride, sodium hydride and potassium hydride; alkali metal amides such as lithium amide, sodium amide and potassium amide; metal alkoxides such as lithium methoxide, sodium methoxide, sodium ethoxide, sodium tert-butoxide and potassium tert-butoxide; lithium amides such as lithium diisopropylamide (LDA), lithium cyclohexyl isopropyl amide and lithium tetramethylpiperidide; alkali metal silylamides such as lithium bistrimethylsilylamide, sodium bistrimethylsilylamide and potassium bistrimethylsilylamide; alkyllithiums such as methyllithium, n-butyllithium, sec-butyllithium and tert-butyllithium; alkyl magnesium halides such as methylmagnesium chloride, methylmagnesium bromide, methylmagnesium iodide, ethylmagnesium chloride, ethylmagnesium bromide, isopropylmagnesium chloride, isopropylmagnesium bromide and isobutylmagnesium chloride; and organic amines such as triethylamine, tributylamine, diisopropylethylamine, diethylamine, diisopropylamine, N-methylpiperidine, N-methylmorpholine, N-ethylmorpholine, pyridine, picoline, 2,6-lutidine, 4-(N,N-dimethylamino)pyridine, N,N-dimethylaniline, N,N-diethylaniline, 1,5-diazabicyclo[4,3,0]non-5-en, 1,4-diazabicyclo[2,2,2]octane (DABCO) and 1,8-diazabicyclo[5,4,0]-7-undecene (DBU).

In each of the steps of the following process, the reaction temperature varies depending on, e.g., the solvent, starting material and reagent, and the reaction time varies depending on the solvent, starting material and reagent.

In each of the steps of the following process, after completion of a reaction in the step, a target compound is isolated from a reaction mixture in accordance with a routine method. A target compound can be obtained, for example, by (i) removing an insoluble matter such as a catalyst as needed, by filtration, (ii) adding, to the reaction mixture, water and a solvent not miscible with water (for example, dichloromethane, chloroform, diethyl ether, ethyl acetate or toluene) to extract the target compound, (iii) washing the organic layer with water and drying it over a desiccant such as anhydrous sodium sulfate or anhydrous magnesium sulfate, and (iv) distilling the solvent. The obtained target compound may, if necessary, be further purified in accordance with a routine method such as recrystallization, reprecipitation or silica gel column chromatography. The target compound in each step can be directly used in the next reaction without purification.

### [Process 1] Synthesis for compound (1a) represented by formula (I) wherein R³ represents a hydrogen atom.

### [Process 1]

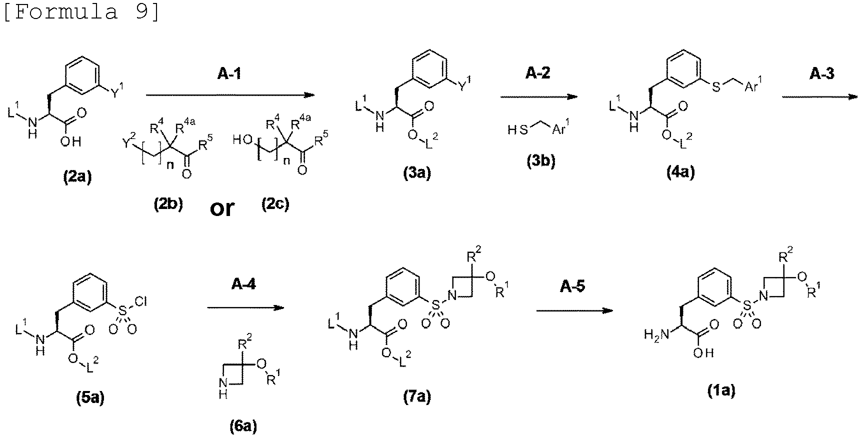

In the chemical formulas of the compounds in Process 1, R¹, R², R⁴, R^{4a} and R⁵ are the same as defined in formula (I). L¹ is a group commonly used as a protective group for an amino group and represents, for example, a tert-butylcarbonyl group. L² is a group commonly used as a protective group for a carboxyl group and represents, for example, a C₁-C₆ alkyl group. Other than this, a group represented by formula (A) can be used. Y¹ and Y² represent, e.g., a halogen atom and a triflate group. Ar¹ represents a substituted or unsubstituted aromatic ring, for example, benzene, 4-tert-butylbenzene or 4-trimethylsilylbenzene.

Step A-1 is a step of obtaining compound (3a) by protecting the carbonyl group of a compound (2a) with a protective group. Step A-1 can be carried out in accordance with a routine method as described, for example, in T. W. Green, P. G. M. Wuts, Protective Groups in Organic Synthesis. Fourth Edition, 2007, John Wiley & Sons, Inc. A compound (2a) may be a commercially available product or can be easily prepared from a commonly known compound.

If L² is a group represented by formula (A), step A-1 can be carried out by
(A-la) a process for obtaining a compound (3a) by a substitution reaction with a compound (2b) in a basic condition or,
(A-1b) a process for obtaining a compound (3a) by ester condensation (reaction) with a compound (2c) by a condensing agent.

A compound (2b) and a compound (2c) may be commercially available products or can be easily prepared from commonly known compounds.

The base to be used in step A-1a is an alkali metal carbonate or an alkali metal hydrogen carbonate, preferably an alkali metal carbonate, and further preferably, sodium carbonate. The reaction can be accelerated by adding, e.g., sodium iodide.

The solvent to be used is an aromatic hydrocarbon, a halogenated hydrocarbon, an ether, an amide or a sulfoxide, and preferably, N,N-dimethylformamide.

The reaction temperature is preferably room temperature to 100°C, and further preferably, 80°C.

The reaction time is one hour to 12 hours, preferably, 5 hours.

The ester condensation in step A-1b can be carried out by a method commonly known in the technical field of synthetic organic chemistry, for example, a method described in the fifth series of Experimental Chemistry, 2005, the Chemical Society of Japan.

The condensing agent to be used herein is preferably, a mixing agent of 1-(3-dimethylaminopropyl)-3-ethylcarbodiimide hydrochloride (EDCI) and N,N-dimethyl-4-aminopyridine (DMAP).

The reaction temperature is from 0°C to room temperature, preferably, room temperature.

The reaction time is 30 minutes to 5 hours, preferably, 2 hours.

Note that, an optically active substance in which different groups are used as R⁴ and R^{4a} can be synthesized by a method of synthesizing optical active compound (9c) by condensation reaction using optical active compound (2c) or a method of splitting the compound (9c) produced by an ordinary purification method.

Step A-2 is a step of obtaining a compound (4a) by a coupling reaction between a compound (3a) obtained in step A-1 and a compound (3b) in the presence of a metal catalyst. A compound (3b) may be a commercially available product or can be easily prepared from a commonly known compound.

This step is not particularly limited as long as the other parts of the compound are not affected and can be carried out by a method commonly known in the technical field of synthetic organic chemistry, for example, described in Palladium Reagents and Catalysts (2004, John Wiley & Sons Ltd.).

As the metal catalyst to be used, a palladium metal catalyst commonly used in a coupling reaction and an organic phosphorus compound are available; preferably, a tris (dibenzylideneacetone) dipalladium (0)(Pd₂(dba)₃) and [4,5-bis(diphenylphosphino)-9,9-dimethylxanthene]dichloropalladium (II) are used in combination.

The base to be used herein is preferably, an organic amine, and more preferably, triethylamine and diisopropylethylamine.

The solvent to be used herein is preferably an aliphatic hydrocarbon, an aromatic hydrocarbon or an ether, and more preferably, 1,4-dioxane.

The reaction temperature is preferably room temperature to 120°C, and more preferably, 80°C to 100°C.

The reaction time is 2 hours to 12 hours, and preferably, 5 hours.

Step A-3 is a step of obtaining the compound (5a) by reacting a chlorinating agent with the compound (4a) obtained in step A-2 in an acidic condition.

As the chlorinating agent to be used, a reactant commonly known in the technical field of synthetic organic chemistry, for example, described in TCI chemical (https://www.tcichemicals.com/ja/jp/support-download/brochure/R5105.pdf) can be used, and preferably, N-chlorosuccinimide or 1,3-dichloro-5,5-dimethylhydantoin is used.

The acid to be used is preferably an organic acid, and further preferably, acetic acid.

The solvent to be used is preferably an aliphatic hydrocarbon, an aromatic hydrocarbon, an ester, a nitrile, an ether, water or a mixture of these, and more preferably, a mixed solvent of acetonitrile and water.

The reaction temperature is preferably -20°C to room temperature, and more preferably, 0°C.

The reaction time is 30 minutes to 4 hours, preferably, 2 hours.

Step A-4 is a step of obtaining a compound (7a) by reacting an azetidine compound (6a) with the compound (5a) obtained in step A-3 in the presence of a base. The azetidine compound (6a) may be a commercially available product or can be easily prepared in accordance with Process 3 described later.

The base to be used is preferably an organic amine, and further preferably, triethylamine or diisopropylethylamine.

The solvent to be used is preferably an aromatic hydrocarbon, a halogenated hydrocarbon, an ether, an amide or a sulfoxide, and further preferably, tetrahydrofuran.

The reaction temperature is preferably 0°C to 80°C, and further preferably, room temperature.

The reaction time is from one hour to 12 hours, preferably, 4 hours.

Step A-5 is a step of obtaining a compound (1a) by removing protective groups (L¹, L²) from a compound (7a) obtained in step A-4.

The deprotection reaction of this step is not particularly limited as long as the other parts of the compound are not affected and varies depending on the protective group used. The deprotection reaction can be carried out in accordance with a routine method described, for example in, T. W. Green, P. G. M. Wuts, Protective Groups in Organic Synthesis, Fourth Edition, 2007, John Wiley & Sons, Inc. preferably, P¹ is a tert-butoxycarbonyl group and P² is a tert-butyl group or an ethyl group. The tert-butoxycarbonyl group and tert-butyl group can be removed in an acidic condition, whereas the ethyl group can be removed in a basic condition.

[Process 2] Synthesis for a compound (1b) represented by formula (I) wherein R³ is a group represented by formula (A).

### [Process 2]

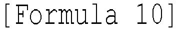

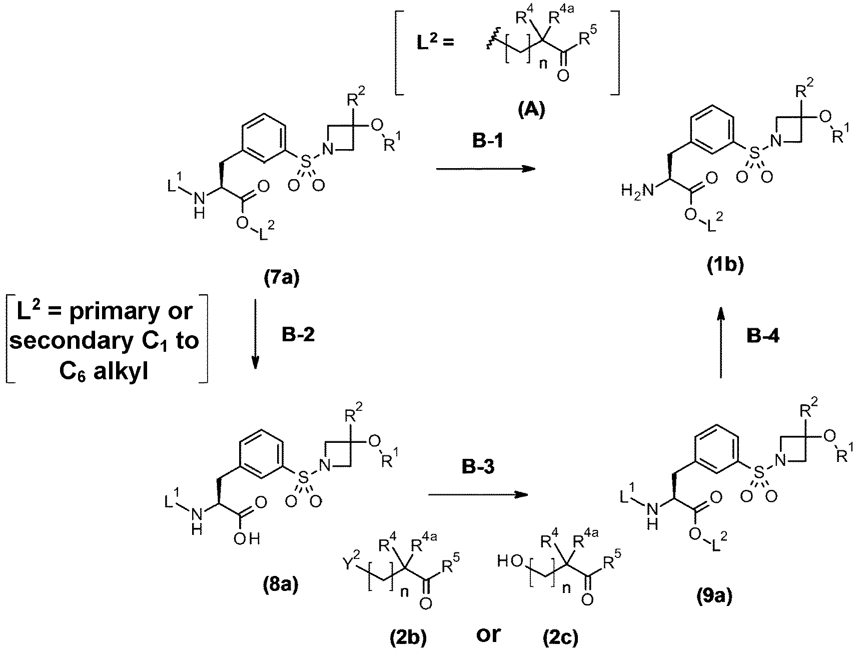

In the chemical formulas of compounds in Process 2, L¹, L², R¹, R², R⁴, R^{4a}, R⁵, Y² and n are the same as defined in formula (I) and Process 1. In Process 2, a route for obtaining a compound (1a) from a compound (7a) varies depending on the type of substituent L². More specifically, if substituent L² is a group represented by formula (A), a compound (1b) is synthesized in accordance with step B-1; whereas if substituent L² is a primary or secondary C₁-C₆ alkyl group, a compound (1b) is synthesized in accordance with steps B-2, B-3, and B-4.

Step B-1 is a step of obtaining a compound (1b) by removing a protective group (L¹) for an amino group. A compound (7a) can be prepared in accordance with Process 1.

The deprotection reaction of this step is not particularly limited as long as the other parts of the compound are not affected and varies depending on the protective group used. The deprotection reaction can be carried out in accordance with a routine method described, for example, in T. W. Green, P. G. M. Wuts, Protective Groups in Organic Synthesis, Fourth Edition,2007, John Wiley & Sons, Inc. Preferably, L¹ is a tert-butoxycarbonyl group. In the case, the reaction for removing the protective group can be carried out in an acidic condition.

The solvent to be used is an aliphatic hydrocarbon, an aromatic hydrocarbon, a halogenated hydrocarbon or an ether; preferably, a halogenated hydrocarbon, and further preferably, dichloromethane.

The acid catalyst to be used is an inorganic acid, an organic acid or an organic sulfonic acid, and preferably, hydrochloric acid or trifluoro acetic acid.

The reaction temperature is room temperature to 50°C, preferably, room temperature.

The reaction time is 30 minutes to 12 hours, preferably, 2 hours.

Step B-2 is a step of obtaining a compound (8a) by removing a protective group (L²) of a carbonyl group.

The deprotection reaction of this step is not particularly limited as long as the other parts of the compound are not affected, and varies depending on the protective group used. The deprotection reaction can be carried out in accordance with a routine method described, for example, in T. W. Green, P. G. M. Wuts, Protective Groups in Organic Synthesis, Fourth Edition,2007, John Wiley & Sons, Inc. Preferably, P¹ is a methyl group or an ethyl group. In the case, the deprotection reaction thereof can be carried out in a basic condition.

The base to be used is preferably an alkali metal hydroxide, and more preferably, sodium hydroxide or potassium hydroxide.

The solvent to be used is preferably an ether, an alcohol, water or a mixture of these, and more preferably, tetrahydrofuran, a mixed solvent of methanol and water.

The reaction temperature is preferably room temperature to 50°C.

The reaction time is 30 minutes to 12 hours, preferably, 2 hours.

Step B-3 is a step of obtaining a compound (9a) by introducing a group represented by formula (A) into a carbonyl group of a compound (8a) obtained in step B-2 in the same manner as in step A-1 of Process 1. The compounds (2b) and (2c) may be commercially available products or can be easily prepared from a commonly known compound. An optical active compound (9c) can be synthesized in the same manner as in step A-1 of Process 1.

### [Process 3] Synthesis of compound (6a) to be used in Process 1

### [Process 3]

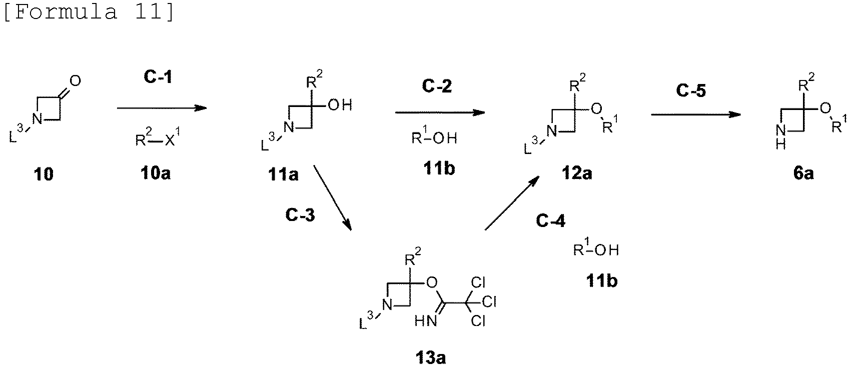

In the chemical formulas of compounds in Process 3, R¹ and R² are the same as defined in formula (I), Process 1 and Process 2. L³ is a group commonly used as a protective group for an amino group, and represents, for example, a tert-butoxycarbonyl group. X¹ is a group required for introducing a group represented by R² into a compound (10), and represents, for example, a magnesium halide (group) and a zinc halide (group).

Step C-1 is a step of obtaining compound (11a) by reacting a compound (10a) with a compound (10). A compound (10) and compound (10a) may be commercially available products or can be easily prepared from a commonly known compound.

The solvent to be used is preferably ether, and more preferably, tetrahydrofuran.

The reaction temperature is preferably -78°C to room temperature.

The reaction time is one hour to 12 hours, preferably, 5 hours.

Step C-2 is a step of obtaining a compound (12a) by reacting a phenol compound (11b) with the hydroxy group of a compound (11a) obtained in step C-1, in the presence of a condensing agent. A compound (11b) may be a commercially available product or can be easily prepared from a commonly known compound.

The condensation reaction of this step can be carried out by a method commonly known in the technical field of synthetic organic chemistry, for example, described, in the fifth series of Experimental Chemistry, 2005, the Chemical Society of Japan.

The condensing agent to be used is preferably a combination of triphenylphosphine and diisopropyl azodicarboxylate (DIAD) or a combination of tributyl phosphine and 1,1'-azobis (N,N-dimethylformamide)(TMAD).

The solvent to be used is preferably an aromatic hydrocarbon, a halogenated hydrocarbon or an ether, and more preferably, toluene.

The reaction temperature is preferably room temperature to 50°C and more preferably, room temperature.

The reaction time is one hour to 12 hours, preferably, 5 hours.

Step C-3 is a step of obtaining a compound (13a) by reacting trichloroacetonitrile with a compound (11a) in a basic condition.

The base to be used is preferably an alkali metal hydroxide, a metal alkoxide or an organic amine, and more preferably, 1,8-diazabicyclo[5,4,0]-7-undecene (DBU).

The solvent to be used is preferably an aromatic hydrocarbon, a halogenated hydrocarbon or an ether, and more preferably, dichloromethane.

The reaction temperature is preferably 0°C to 50°C, and more preferably, 0°C to room temperature.

The reaction time is one hour to 12 hours, preferably, 2 hours.

Step C-4 is a step of obtaining a compound (12a) by reacting a compound (11b) with a compound (13a) obtained in step C-3 in an acidic condition.

In the reaction, if L³ is a tert-butoxycarbonyl group, in some case, L³ is simultaneously removed to produce a compound (6a).

The acid to be used is preferably an organic acid or organic phosphoric acid, and more preferably, a trifluoroborane/diethyl ether complex (BF₃/Et₂O).

The solvent to be used is an aromatic hydrocarbon, a halogenated hydrocarbon or an ether, preferably, dichloromethane.

The reaction temperature is preferably room temperature to 50°C, and more preferably, room temperature.

The reaction time is 30 minutes to 5 hours, preferably, one hour.

Step C-5 is a step of obtaining a compound (6a) by removing a protective group (L³) from the amino group of a compound (12a) obtained in step C-2 or step C-4.

The deprotection reaction of this step is not particularly limited as long as the other parts of the compound are not affected and varies depending on the protective group used. The deprotection reaction can be carried out in accordance with a routine method described, for example, in T. W. Green, P. G. M. Wuts, Protective Groups in Organic Synthesis, Fourth Edition, 2007, John Wiley & Sons, Inc. Preferably, L² is a tert-butoxycarbonyl group. The deprotection reaction can be carried out in an acidic condition.

The solvent to be used is preferably an aliphatic hydrocarbon, an aromatic hydrocarbon, a halogenated hydrocarbon or an ether, and more preferably, dichloromethane.

The acid catalyst to be used is preferably an inorganic acid, an organic acid or an organic sulfonic acid, and more preferably, trifluoro acetic acid or hydrochloric acid.

The reaction temperature is preferably 0°C to room temperature.

The reaction time is preferably 30 minutes to 5 hours, and more preferably, 2 hours.

A compound represented by formula (I) of the present invention or a pharmaceutically acceptable salt thereof includes a hydrate thereof. When a compound represented by formula (I) or a pharmaceutically acceptable salt thereof is allowed to leave in the air or recrystallized, it absorbs water molecules and sometimes changes into a hydrate.

A compound represented by formula (I) of the present invention or a pharmaceutically acceptable salt thereof includes a solvate thereof. When a compound represented by formula (I) or a pharmaceutically acceptable salt thereof is allowed to remain in a solvent or recrystallized in a solvent, it absorbs the solvent and sometimes changes into a solvate.

A compound of the present invention or a pharmaceutically acceptable salt thereof can be administered in various dosage forms. Examples of the dosage forms include a tablet, a capsule, a granule, an emulsion, a pill, a powder and a syrup (liquid) for oral administration; and an injection (intravenous, intramuscular, subcutaneous or intraperitoneal administration), a drop and a suppository (rectal administration) for parenteral administration. These various formulations can be prepared in accordance with a routine method by adding, to a main ingredient, auxiliaries that can be commonly used in the technical field of pharmaceutical drugs, such as an excipient, a binder, a disintegrant, a lubricant, a flavoring agent, a dissolution aid, a suspension and a coating agent.

Examples of the carrier that can be used for tablets include excipients such as lactose, white sugar, sodium chloride, glucose, urea, starch, calcium carbonate, kaolin, crystalline cellulose and silicic acid; binders such as water, ethanol, propanol, simple syrup, a glucose solution, a starch solution, a gelatin solution, carboxymethyl cellulose, shellac, methyl cellulose, potassium phosphate and polyvinylpyrrolidone; disintegrants such as dried starch, sodium alginate, agar powder, laminarin powder, sodium hydrogen carbonate, calcium carbonate, polyoxyethylene sorbitan fatty acid ester, sodium lauryl sulfate, glycerol monostearate, starch and lactose; disintegration inhibitors such as white sugar, stearin, cocoa butter, and hydrogenated oil; absorption enhancers such as a quaternary ammonium salt and sodium lauryl sulfate; moisturizers such as glycerin and starch; adsorbents such as starch, lactose, kaolin, bentonite and colloidal silicic acid; and lubricants such as purified talc, stearate, boric acid powder and polyethylene glycol. If necessary, tablets may be coated with a film commonly used in the technical field to obtain, for example, sugar-coated tablets, gelatin capsules, enteric coated tablets, film coated tablets or double layer tablets, multi-layer tablets.

For pills, examples of the carrier include an excipient such as glucose, lactose, cocoa butter, starch, hardened vegetable oil, kaolin and talc; a binder such as gum Arabic powder, tragacanth powder, gelatin, and ethanol; and a disintegrant such as laminarin and agar.

For suppositories, a wide variety of carriers commonly known in the field can be used. Examples of carriers include polyethylene glycol, cocoa butter, a higher alcohol, an ester of a higher alcohol, gelatin and semi-synthetic glyceride.

For injections, a liquid, an emulsion or a suspension can be used. The liquid, emulsion or suspension is preferably sterilized and controlled to be isotonic to blood. The solvent for the liquid, emulsion or suspension is not particularly limited as long as it can be used as a diluent for medical use. Examples of the solvent include water, ethanol, propylene glycol, ethoxylated isostearyl alcohol, polyoxylated isostearyl alcohol and polyoxyethylene sorbitan fatty acid ester. Note that, in this case, a preparation may contain a sufficient amount of salt, glucose or glycerin to prepare an isotonic solution, and additionally, a dissolution aid, a buffer and/or a soothing agent (commonly used).

The above preparation may contain e.g., a colorant, a preservative, a fragrance, a flavoring agent and a sweetener, as needed and further another pharmaceutical preparation.

The content of a compound serving as an active ingredient in the above preparation is not particularly limited and appropriately selected from a wide range. The content is usually 0.5 to 70 wt%, and preferably, 1 to 30 wt% of the whole composition.

The dosage of the preparation varies depending on, e.g., the symptoms and age of the patient (a warm-blooded animal, particularly a human). For oral administration, the lower limit of a dose per time is 0.01 mg/kg body weight (preferably, 0.1 mg/kg body weight), whereas the upper limit thereof is 500 mg/kg body weight (preferably, 100 mg/kg body weight). For intravenous administration, the lower limit of a dose per time is 0.002 mg/kg body weight (preferably, 0.02 mg/kg body weight), whereas the upper limit is 100 mg/kg body weight (preferably, 20 mg/kg body weight). The dose is desirably administered within one to several times per day depending on the symptoms.

### Examples

Now, the present invention will be more specifically described by way of, e.g., Examples and Test Examples, however, the scope of the present invention is not limited to these.

In the Examples and Reference Examples, elution of column chromatography was carried out based on observation by TLC (thin layer chromatography). In observation by TLC, silica gel 60F₂₅₄ (Merck) was used as TLC plate; and the solvent used as elution solvent in the column chromatography was used as the developing solvent. Detection was made by UV detector or by chromogenic method with a coloring agent (for example, a solution containing ninhydrin, anisaldehyde, ammonium phosphomolybdate, cerium ammonium nitrate (CAM) or alkaline permanganate).

Silica gel used in the column was silica gel SK-85 (230-400 mesh) (Merck), silica gel 60 N (40-50 µm) manufactured by Kanto Chemical Co., Inc., or Chromatorex NH (200-350 mesh) manufactured by FUJI SILYSIA CHEMICAL LTD. Other than the column chromatography commonly used, an automatic chromatography device such as Purif-**α**2 or Purif-espoir2 (Shoko Science Co., Ltd.), W-Prep 2XY (Yamazen Corporation), Isolera One (Biotage) and CombiFlash Rf (Isco) was appropriately used. The type of elution solvent was determined based on observation by TLC. As high performance liquid chromatography, a preparatory purification HPLC system manufactured by Gilson was used in combination with a column (Develosil Combi-RP-5 28x100) manufactured by NOMURA CHEMICAL. Note that, abbreviations used in the Reference Examples and Examples have the following meanings.

mg: milligram, g: gram, **µ**L: micro liter, mL: milliliter, L: liter, N: normality, M: mol concentration, MHz: megahertz, Boc: tert-butoxycarbonyl, DMAP: N,N-dimethyl-4-aminopyridine, EDCI: 1-(3-dimethylaminopropyl)-3-ethylcarbodiimide monohydrochloride, DMF: N,N-dimethylformamide, HATU: O-(7-azabenzotriazol-1-yl)-N,N,N',N'-tetramethyluronium hexafluorophosphate.

In the Examples and Reference Examples, nuclear magnetic resonance (¹H NMR) spectra were obtained using tetramethylsilane as standard substance and a chemical shift values were described as **δ** values (ppm). In split patterns, a single line is represented by s, a double line by d, a triple line by t, a quadruple line by q, a multiple line by m, and a broad line by br.

Mass spectrometry (hereinafter referred to as MS) was carried out in accordance with EI (electron ionization), ESI (electron spray ionization), APCI (atmospheric pressure chemical ionization), ES/APCI (electron spray atmospheric pressure chemical ionization) or FAB (fast atom bombardment).

In individual steps of the Examples and Reference Examples, unless otherwise specified, preparation of reaction solutions and reactions were carried out at room temperature.

The structural formulas of compounds produced in the Reference Examples and data obtained by devices are shown in Table 1 (Table 1-1 to Table 1-7).

The structural formulas of compounds produced in the Examples and data obtained by devices are shown in Table 2 (Table 2-1 to Table 2-27).

### Reference Example 1

### 3-(4-Fluorophenyl)-3-[(1-hydroxy-1,3-dihydro-2,1-benzoxaborol-5-yl)oxy]azetidine-1-carboxylic acid tert-butyl ester

### (1a) 3-(4-Bromo-3-formylphenoxy)-3-(4-fluorophenyl)azetidine-1-carboxylic acid tert-butyl ester

To a toluene (9.35 mL) suspension of 3-(4-fluorophenyl)-3-hydroxyazetidine-1-carboxylic acid tert-butyl ester (750 mg, 2.81 mmol), 2-bromo-5-hydroxybenzaldehyde (1.13 g, 5.61 mmol) and triphenylphosphine (810 mg, 3.09 mmol), diisopropyl azodicarboxylate (0.608 mL, 3.09 mmol) was added at 0°C. The reaction mixture was stirred at room temperature, overnight. The solvent was distilled away under reduced pressure and the obtained residue was purified by silica gel column chromatography [elution solvent: n-hexane/ethyl acetate = 9/1-3/1 (V/V)] to obtain the title compound as a solid substance (467 mg, 37%).

### (1b) 3-(4-Fluorophenyl)-3-[3-formyl-4-(4,4,5,5-tetramethyl-1,3,2-dioxaborolan-2-yl)phenoxy]azetidine-1-carboxylic acid tert-butyl ester

To a 1,4-dioxane (10 mL) solution of 3-(4-bromo-3-formylphenoxy)-3-(4-fluorophenyl)azetidine-1-carboxylic acid tert-butyl ester (467 mg, 1.04 mmol) obtained in Reference Example 1a, potassium acetate (305 mg, 3.11 mmol), [1,1'-bis(diphenylphosphino)ferrocene]palladium(II) dichloride dichloromethane adduct (85 mg, 0.104 mmol) and bis(pinacolato)diboron (395 mg, 1.56 mmol) were added. The reaction mixture was heated at 80°C for 9 hours. After cooling to room temperature, the reaction mixture was filtered through Celite and washed with ethyl acetate. The solvent of the filtrate was distilled away under reduced pressure and the obtained residue was purified by silica gel column chromatography [elution solvent: n-hexane/ethyl acetate = 95/5-5/1 (V/V)] to obtain the title compound (383 mg, 74%) as a solid substance.

### (1c) 3-(4-Fluorophenyl)-3-[(1-hydroxy-1,3-dihydro-2,1-benzoxaborol-5-yl)oxy]azetidine-1-carboxylic acid tert-butyl ester

To a methanol (3.08 mL) solution of 3-(4-fluorophenyl)-3-[3-formyl-4-(4,4,5,5-tetramethyl-1,3,2-dioxaborolan-2-yl)phenoxy]azetidine-1-carboxylic acid tert-butyl ester (383 mg, 0.770 mmol) obtained in Reference Example 1b, sodium borohydride (14 mg, 0.385 mmol) was added at 0°C. The reaction mixture was stirred at room temperature for 80 minutes. Further, sodium borohydride (7 mg, 0.193 mmol) was added thereto and the reaction mixture was stirred at room temperature for 30 minutes. Thereafter, 6N hydrochloric acid and water were added to the reaction mixture, which was extracted with ethyl acetate. The extract was washed with saturated saline solution and dried over anhydrous sodium sulfate. After filtration, the solvent was distilled away under reduced pressure and the obtained residue was purified by silica gel column chromatography [elution solvent: n-hexane/ethyl acetate = 3/1-1/1 (V/V)] to obtain the title compound as a solid substance.

### Reference Example 2

### (4-{[1-(tert-Butoxycarbonyl)-3-phenylazetidin-3-yl]oxy}phenyl)boronic acid

To an acetone (9 mL)-water (4.5 mL) solution of 3-phenyl-3-[4-(4,4,5,5-tetramethyl-1,3,2-dioxaborolan-2-yl)phenoxy]azetidine-1-carboxylic acid tert-butyl ester (332 mg, 0.736 mmol) obtained in the same manner as in Reference Example 1b, sodium periodate (787 mg, 3.68 mmol) and ammonium acetate (283 mg, 3.68 mmol) were added. The reaction mixture was stirred at room temperature, overnight. To the reaction mixture, water and ethyl acetate were added. After the reaction mixture was filtered through Celite, the filtrate was extracted with ethyl acetate. The extract was washed with saturated saline solution and dried over anhydrous sodium sulfate. After filtration, the solvent was distilled away under reduced pressure and the obtained residue was purified by silica gel column chromatography [elution solvent: n-hexane/ethyl acetate = 5/1-1/1 (V/V)] to obtain the title compound as a solid substance.

### Reference Example 3

### 3-(3-Fluorophenoxy)-3-phenylazetidine hydrochloride

### (3a) tert-Butyl 3-hydroxy-3-phenylazetidine-1-carboxylate

To a tetrahydrofuran (75 mL) solution of 1-(tert-butoxycarbonyl)-3-azetidinone (5.2 g), phenylmagnesium bromide (1.0 M/tetrahydrofuran solution, 45.6 mL) was added while cooling on ice. The reaction mixture was stirred at room temperature, overnight, neutralized by adding 1 N hydrochloric acid while cooling on ice and extracted with ethyl acetate. The organic layers were combined, washed with saturated saline solution and dried over sodium sulfate. After the sodium sulfate was filtered off, the filtrate was concentrated under reduced pressure. The obtained residue was purified by silica gel column chromatography (n-hexane/ethyl acetate) to obtain the title compound (7.0 g).

### (3b) tert-Butyl 3-(3-fluorophenoxy)-3-phenylazetidine-1-carboxylate

To the compound (6.55 g) obtained in Reference Example 3a, triphenylphosphine (7.63 g), 3-fluorophenol (5.89 g) and toluene (58 mL) were added. The reaction mixture was stirred at room temperature to obtain a homogenous solution. Diisopropyl azodicarboxylate (5.84 g) was added to the solution while stirring under room temperature. After the solution was further stirred for 40 minutes, the solvent was distilled away under reduced pressure. The obtained residue was purified by silica gel column chromatography (n-hexane/ethyl acetate) to obtain the title compound (3.10 g).

### (3c) 3-(3-Fluorophenoxy)-3-phenylazetidine hydrochloride

To the compound (3.00 g) obtained in Reference Example 3b, a 4 N hydrochloric acid/ethyl acetate solution (20 mL) was added. The reaction solution was stirred at room temperature for one hour. To the reaction solution, ethyl acetate (20 mL) was added. The reaction solution was stirred at room temperature and filtered to obtain a solid substance. In this manner, the title compound (2.22 g) was obtained.

### Reference Example 4

### 3-[([1,1'-Biphenyl]-4-yl)oxy]-3-phenylazetidine

### (4a) tert-Butyl 3-phenyl-3-[(2,2,2-trichloroethanimidoyl)oxy]azetidine-2-carboxylate

A dichloromethane solution (10 mL) of the compound (1.0 g, 4.0 mmol) obtained in Reference Example 3a and DBU (0.73 g, 4.8 mmol) was cooled to 0°C. To this, 2,2,2-trichloroacetonitrile (2.5 g, 17 mmol) was added. The reaction solution was stirred at room temperature for 2 hours. To the reaction solution, water (50 mL) was added and the reaction solution was extracted with dichloromethane (50 mL × 2). The organic layer was concentrated, and the obtained residue was purified by silica gel column chromatography (petroleum ether/ethyl acetate) to obtain the title compound (1.6 g) as an oily substance.

### (4b) 3-[([1,1'-Biphenyl]-4-yl)oxy]-3-phenylazetidine

To a dichloromethane solution (8 mL) of the compound (0.60 g, 1.5 mmol) obtained in Reference Example 4a, 4-phenylphenol (0.31 g, 1.8 mmol) and a trifluoroborane/diethyl ether complex (0.022 mg, 0.15 mmol) were added. The reaction solution was stirred at room temperature for one hour in a nitrogen atmosphere. To the reaction solution, saturated aqueous sodium hydrogen carbonate solution (20 mL) was added and the solution was extracted with ethyl acetate (50 mL × 3). The organic layer was washed with saturated saline solution and dried over sodium sulfate. After the sodium sulfate was filtered off, the filtrate was concentrated under reduced pressure. The obtained residue was purified by silica gel column chromatography (dichloromethane/methanol) to obtain the title compound (0.35 g, 1.0 mmol) as a solid substance.

### Reference Example 5

### Ethyl (2S)-((tert-butoxycarbonyl)amino)-3-(3-(chlorosulfonyl)phenyl)propanoate

### (5a) Ethyl (2S)-((tert-butoxycarbonyl)amino)-3-(3-iodophenyl)propanoate

To a dichloromethane (2 1) solution of N-tert-butoxycarbonyl-L-3-iodinephenylalanine (200 g), ethanol (149 mL), 1-ethyl-3-(3-dimethylaminopropyl)carbodiimide hydrochloride (147 g) and N,N-dimethylaminopyridine (6.25 g) were added. The reaction solution was stirred at room temperature for 3 hours. To the reaction solution, 0.5 N hydrochloric acid (1 L) was added to separate the solution into layers. The water layer was extracted with dichloromethane (200 mL). The organic layers were combined, washed with saturated saline solution (1 L) and dried over sodium sulfate (250 g). After the sodium sulfate was filtered off, the filtrate was concentrated under reduced pressure. To the residue, n-hexane (800 mL) was added. The solid substance precipitated was obtained by filtration. In this manner, the title compound (195 g) was obtained.

### (5b) Ethyl (2S)-((tert-butoxycarbonyl)amino)-3-(3-((4-(tert-butyl)benzyl)thio)phenyl)propanoate

To a N-methylpyrrolidone (1.25 L) solution of the compound (125 g) obtained in Reference Example 5a and 4-tert-butyl benzyl mercaptan (100 mL), copper (I) iodide (28.4 g) and potassium carbonate (82 g) were added. The reaction solution was stirred at 100°C in an argon atmosphere, overnight. The reaction solution was allowed to cool to room temperature and diluted with n-hexane/ethyl acetate and water was added thereto. An insoluble matter was removed by Celite filtration and the filtrate was separated into layers. The filtrate was extracted with n-hexane/ethyl acetate. The organic layers were combined, washed with water and saturated saline solution, and dried over sodium sulfate. The residue was concentrated under reduced pressure, roughly purified by silica gel column chromatography (n-hexane/ethyl acetate) to obtain a crude product of the title compound (144 g).

### (5c) Ethyl (2S)-((tert-butoxycarbonyl)amino)-3-(3-(chlorosulfonyl)phenyl)propanoate

To an acetonitrile (1 L) solution of the compound (144 g) obtained in Reference Example 5b, acetic acid (68.5 mL) and 1,3-dichloro-5,5-dimethylhydantoin (124 g) were added while cooling on ice. The reaction solution was stirred for 30 minutes. To the reaction solution, water was added. After concentratration, the reaction solution was diluted with n-hexane/ethyl acetate, washed sequentially with water and saturated saline solution in this order and dried over sodium sulfate. After the sodium sulfate was filtered off, the filtrate was concentrated. The obtained residue was purified by silica gel column chromatography (n-hexane/ethyl acetate) to obtain the title compound (63 g).

### Reference Example 6

### 1-(Morpholin-4-yl)-1-oxopropan-2-yl (2S)-2-[(tert-butoxycarbonyl)amino]-3-[3-(chlorosulfonyl)phenyl]propanoate

### (6a) (1-Methyl-2-morpholino-2-oxoethyl) (2S)-3-(3-Bromophenyl)-2-(tert-butoxycarbonylamino)propanoate

To (2S)-3-(3-bromophenyl)-2-(tert-butoxycarbonylamino)propanoic acid (20 g, 58 mmol), 2-chloro-1-morpholino-propan-1-on (11 g, 64 mmol), potassium carbonate (24 g, 174 mmol) and sodium iodide (8.7 g, 58 mmol), DMF (200 mL) was added. The reaction solution was stirred at 30°C for 60 hours. To the reaction solution, water (1.5 L) was added and the reaction solution was extracted with ethyl acetate (1.5 L). The organic layer was washed with saturated saline solution and dried over sodium sulfate. After the sodium sulfate was filtered off, the filtrate was concentrated. The obtained residue was purified by silica gel column chromatography (petroleum ether/ethyl acetate) to obtain the title compound (28 g).

### (6b) 1-(Morpholin-4-yl)-1-oxopropan-2-yl (2S)-2-[(tert-Butoxycarbonyl)amino]-3-(3-{ [(4-tert-butylphenyl)methyl]sulfanyl}phenyl)propanoate

To the compound (27.8 g, 57.3 mmol) obtained in Reference Example 6a, 4-(tert-butyl)benzyl mercaptan (12.4 g, 68.7 mmol), 4,5-bis(diphenylphosphino)-9,9-dimethylxanthene (Xantphos) (3.31 g, 5.73 mmol) and N,N-diisopropylethylamine (DIPEA) (22.2 g, 172 mmol), 1,4-dioxane (280 mL) was added and tris(dibenzylideneacetone)dipalladium (0) (Pd₂(dba)₃) was added thereto. The reaction solution was stirred at 105°C and for 15 hours in a nitrogen atmosphere. After the reaction solution was concentrated, water (1.5 L) was added thereto. The reaction solution was extracted with ethyl acetate (1.5 L). The organic layer was washed with saturated saline solution (1.5 L × 2) and dried over sodium sulfate. After the sodium sulfate was filtered off, the filtrate was concentrated. The obtained residue was purified by silica gel column chromatography (petroleum ether/ethyl acetate) to obtain the title compound (33.5 g).

### (6c) 1-(Morpholin-4-yl)-1-oxopropan-2-yl (2S)-2-[(tert-butoxycarbonyl)amino]-3-[3-(chlorosulfonyl)phenyl]propanoate

To the compound (2.00 g, 3.42 mmol) obtained in Reference Example 6b, acetonitrile (34 mL), acetic acid (1.2 mL) and water (0.8 mL) were added. The reaction solution was cooled to 0°C and 1,3-dichloro-5,5-dimethyl-imidazoline-2,4-dione (1.35 g, 6.84 mmol) was added thereto. The reaction solution was stirred for 2 hours. To the reaction solution, water (100 mL) was added. The reaction solution was neutralized by adding saturated aqueous sodium hydrogen carbonate solution, and then, extracted with dichloromethane (100 mL). The organic layer was washed with saturated saline solution (100 mL × 2) and dried over sodium sulfate. After the sodium sulfate was filtered off, the filtrate was concentrated. The obtained residue was purified by silica gel column chromatography (petroleum ether/ethyl acetate) to obtain the title compound (0.87 g).

### Reference Example 7

### 1-(Dimethylamino)-3-methyl-1-oxobutan-2-yl (2S)-2-[(tert-butoxycarbonyl)amino]-3-[3-(chlorosulfonyl)phenyl]propanoate

### (7a) 1-(Dimethylamino)-3-methyl-1-oxobutan-2-yl (2S)-2-[(tert-butoxycarbonyl)amino]-3-(3-iodophenyl)propanoate

To (2S)-3-(3-iodophenyl)-2-(tert-butoxycarbonylamino)propanoic acid (14.7 g, 36.5 mmol), 2-hydroxy-N,N,3-trimethyl-butanamide (7.43 g, 40.1 mmol) and 4-dimethylaminopyridine (DMAP) (0.89 g, 7.29 mmol), dichloromethane (91 mL) was added. The reaction solution was cooled to 0°C. To the reaction solution, EDCI (8.39 g, 43.7 mmol) was added. The reaction solution was stirred at room temperature for one hour. After the reaction solution was concentrated, water (100 mL) was added thereto. The reaction solution was extracted with ethyl acetate (100 mL). The organic layer was washed with saturated saline solution (100 mL × 2) and dried over sodium sulfate. After the sodium sulfate was filtered off, the filtrate was concentrated. The obtained residue was purified by silica gel column chromatography (hexane/ethyl acetate) to obtain the title compound (17.2 g).

### (7b) 1-(Dimethylamino)-3-methyl-1-oxobutan-2-yl (2S)-2-[(tert-butoxycarbonyl)amino]-3-(3-{[(4-tert-butylphenyl)methyl]sulfanyl}phenyl)propanoate

The title compound (680 mg, 1.19 mmol) was obtained by production according to the method described in Examples 5b using the compound (700 mg, 1.35 mmol) obtained in Reference Example 7a.

### (7c) 1-(Dimethylamino)-3-methyl-1-oxobutan-2-yl (2S)-2-[(tert-butoxycarbonyl)amino]-3-[3-(chlorosulfonyl)phenyl]propanoate

The title compound (580 mg, 1.18 mmol) was obtained in the same manner as in Reference Example 5c except that the compound (680 mg, 1.19 mmol) obtained in Reference Example 7b was used.

### Reference Example 8

### 1-(Dimethylamino)-1-oxopropan-2-yl(2S)-2-[(tert-butoxycarbonyl)amino]-3-[3-(chlorosulfonyl)phenyl]propanoate

The title compound was synthesized in the same manner as in Reference Example 6 except that (2S)-3-(3-bromophenyl)-2-(tert-butoxycarbonylamino)propionic acid was used.

### Reference Example 9

### 1-Oxo-1-[(propan-2-yl)amino]propan-2-yl (2S)-2-[(tert-butoxycarbonyl)amino]-3-[3-(chlorosulfonyl)phenyl]propanoate

The title compound was synthesized in the same manner as in Reference Example 6 except that (2S)-3-(3-iodinephenyl)-2-(tert-butoxycarbonylamino)propionic acid was used.

### Reference Example 10

### tert-Butyl (2S)-2-((tert-butoxycarbonyl)amino)-3-(3-(chlorosulfonyl)phenyl)propanoate

### (10a) tert-Butyl (2S)-3-(3-bromophenyl)-2-((tert-butoxycarbonyl)amino)propanoate

To a toluene (120 mL) solution of N-tert-butoxycarbonyl-L-3-bromophenylalanine (9.5 g), tert-butanol (60 mL) was added. The reaction solution was heat/refluxed for 15 minutes. While heating and refluxing, N,N-dimethylformamide dineopentylacetal (35 mL) was slowly added dropwise in three portions over 3 hours. The reaction solution was further stirred for one hour. After cooled to room temperature, the reaction solution was diluted with ethyl acetate, sequentially washed with saturated aqueous sodium hydrogen carbonate solution, water and saturated saline solution in this order and dried over sodium sulfate. After the sodium sulfate was filtered off, the filtrate was concentrated under reduced pressure. The residue was purified by silica gel column chromatography (n-hexane/ethyl acetate) to obtain the title compound (10.2 g).

### (10b) tert-Butyl (2S)-2-((tert-butoxycarbonyl)amino)-3-(3-(chlorosulfonyl)phenyl)propanoate

The title compound was synthesized in the same manner as in Reference Example 6 except that the compound obtained in Reference Example 10a was used.

### Reference Example 11

### tert-Butyl 3-phenoxy-3-phenylazetidine-1-carboxylate

A toluene solution (5.7 mL) of the compound (500 mg, 2.01 mmol) obtained in Reference Example 3a, phenol (380 mg) and tri-n-butylphosphine (0.79 mL) was cooled to 0°C, and 1,1'-azobis(N,N-dimethylformamide) (530 mg) was added thereto. The reaction solution was stirred at room temperature for 3 hours and concentrated. The obtained residue was purified by silica gel column chromatography (hexane/ethyl acetate) to obtain the title compound (354 mg) as a solid substance.

### Reference Example 12

### tert-Butyl 3-(carbamoylphenoxy)-3-phenylazetidine-1-carboxylate

### (12a) tert-Butyl 3-[4-(methoxycarbonyl)phenoxy]-3-phenylazetidine-1-carboxylate

The compound (1.55 g) obtained in Reference Example 3a was dissolved in toluene (50 mL) and methyl 4-hydroxybenzoate (1.42 g) and triphenylphosphine (2.4 g) were added thereto. The reaction solution was cooled to 0°C. To the reaction solution, diisopropyl azodicarboxylate (DIAD) (1.84 mL) was added. The reaction solution was stirred at room temperature for 5 hours and concentrated. The obtained residue was purified by silica gel column chromatography (hexane/ethyl acetate) to obtain the title compound (1.2 g) as a solid substance.

### (12b) tert-Butyl 3-(carbamoylphenoxy)-3-phenylazetidine-1-carboxylate

The compound (1.2 g) obtained in Reference Example 12a was dissolved in tetrahydrofuran (10 mL) and methanol (10 mL), and a 5 N aqueous sodium hydroxide solution (2.0 mL) was added thereto. The reaction solution was stirred at room temperature for 3 hours. To the reaction solution, 5 N hydrochloric acid (2.0 mL) was added to neutralize. Thereafter, the reaction solution was concentrated under reduced pressure to obtain a crude product of 4-{[1-(tert-butoxycarbonyl)-3-phenylazetidin-3-yl]oxy}benzoate. To the obtained compound, DMF (16 mL) was added, and then, N,N-diisopropylethylamine (1.1 mL) and HATU (1.8 g) were added thereto. The reaction solution was cooled to 0°C. Thereafter, a 2 N ammonia/isopropyl alcohol solution (6.2 mL) was added and the reaction solution was stirred at room temperature 12 hours. To the reaction solution, water (30 mL) was added. The reaction solution was extracted with ethyl acetate (30 mL × 2). The organic layer was washed with saturated saline solution and dried over anhydrous sodium sulfate. After filtration, the solvent was distilled away under reduced pressure. The residue was purified by silica gel column chromatography (n-hexane/ethyl acetate) to obtain the title compound (0.98 g) as a solid substance.

### Reference Example 13

### tert-Butyl 3-(5-fluoropyridin-2-yl)-3-(4-methylphenoxy)azetidine-1-carboxylate

### (13a) tert-Butyl 3-(5-fluoropyridin-2-yl)-3-hydroxyazetidine-1-carboxylate

A toluene (320 mL) solution of 2-bromo-5-fluoropyridine (8.0 g) was cooled to -78°C. To this, n-butyllithium (1.6 M hexane solution, 32 mL) was added dropwise. The reaction solution was stirred for 30 minutes. A toluene (40 mL) solution of tert-butyl 3-oxoazetidine-1-carboxylate (7.0 g) was added. The reaction solution was stirred for one hour, raised in temperature to 0°C, and then, stirred for 2 hours. To the reaction solution, saturated aqueous ammonium chloride solution (200 mL) and water (200 mL) were added. The resultant reaction solution was extracted with ethyl acetate (300 mL × 2). The organic layer was washed sequentially with water and saturated saline solution in this order and dried over sodium sulfate. After the sodium sulfate was filtered off, the filtrate was concentrated under reduced pressure. The residue was purified by silica gel column chromatography (n-hexane/ethyl acetate) to obtain the title compound (6.6 g) •

(13b) The title compound was obtained in the same manner as in Reference Example 3b except that the compound obtained in Reference Example 13a and 4-methylphenol were used.

### Reference Example 14

### tert-Butyl 3-(4-fluorophenyl)-3-phenoxyazetidine-1-carboxylate

The title compound was obtained in the same manner as in Reference Example 3 except that 4-fluorophenylmagnesium bromide and phenol were used.

### Reference Example 15

### tert-Butyl 3-phenyl-3-[(²H₅)phenyloxy]azetidine-1-carboxylate

The title compound was obtained in the same manner as in Reference Example 3 except that (²H₅) phenol was used.

### Reference Example 16

### tert-Butyl 3-(4-fluorophenyl)-3-[(²H₅)phenyloxy]azetidine-1-carboxylate

The title compound was obtained in the same manner as in Reference Example 3 except that 4-fluorophenylmagnesium bromide and (²H₅) phenol were used.

### Reference Example 17

### tert-Butyl 3-(4-fluorophenoxy)-3-(5-fluoropyridin-2-yl)azetidine-1-carboxylate

The title compound was obtained in the same manner as in Reference Example 13 except that 4-fluorophenol was used.

### Example 1

### (2S)-2-Amino-3-(3-((3-(3-fluorophenoxy)-3-phenylazetidin-1-yl)sulfonyl)phenyl)propanoic acid

### (Example 1-1)

### Ethyl (2S)-((tert-butoxycarbonyl)amino)-3-(3-((3-(3-fluorophenoxy)-3-phenylazetidin-1-yl)sulfonyl)phenyl)propanoate

To a dichloromethane (100 mL) solution of the compound (12.9 g) obtained in Reference Example 5, N,N-diisopropylethylamine (10.7 mL) and the compound (5.71 g) obtained in Reference Example 3c were added. The reaction solution was stirred at room temperature for 1.5 hours. To the reaction solution, saturated aqueous potassium hydrogen sulfate solution was added. The solution was separated into layers. The water layer was (re)extracted with dichloromethane. The dichloromethane layers were combined, washed sequentially with water and saturated saline solution, in this order, and dried over sodium sulfate. After the sodium sulfate was filtered off, the filtrate was concentrated under reduced pressure. The residue was purified by silica gel column chromatography (n-hexane/ethyl acetate) to obtain the title compound (8.66 g).

### (Example 1-2)

### (2S)-((tert-Butoxycarbonyl)amino)-3-(3-((3-(3-fluorophenoxy)-3-phenylazetidin-1-yl)sulfonyl)phenyl)propanoic acid

To the compound (8.66 g) obtained in Example 1-1, tetrahydrofuran (60 mL) and methanol (30 mL) were added. After dissolving the compound, a 2 N aqueous sodium hydroxide solution (14.5 mL) was added dropwise while cooling it on ice. Subsequently, the reaction solution was stirred on ice for 4.5 hours. After 2N hydrochloric acid (13.5 mL) was added dropwise, the organic solvent was distilled off under reduced pressure. Ethyl acetate was added to the residue. The resultant solution was sequentially washed with water and saturated saline solution in this order and dried over sodium sulfate. Sodium sulfate was filtered off and the filtrate was concentrated under reduced pressure to obtain a crude product (8.55 g) of the title compound. To the crude product roughly purified, tetrahydrofuran (140 mL), n-hexane (70 mL) and (R)-(+)-1-phenethylamine (1.72 mL) were added. The reaction solution was stirred while heating at 70°C. After stirring at room temperature for 4 hours, the solid substance precipitated was obtained by filtration. In this manner, an (R)-(+)-1-phenethylamine salt (5.93 g) of the title compound was obtained. To a part of the obtained solid substance (5.90 g), ethyl acetate (70 mL) was added. The reaction solution was suspended and stirred, and then, 0.1 N hydrochloric acid was added to the solution to dissolve it. After the solution was allowed to separate into layers, the obtained organic layer was dried over sodium sulfate. Sodium sulfate was filtered off and the filtrate was concentrated under reduced pressure to obtain the title compound (5.11 g).

### (Example 1-3)

### (2S)-Amino-3-(3-((3-(3-fluorophenoxy)-3-phenylazetidin-1-yl)sulfonyl)phenyl)propanoic acid

To the compound (5.11 g) obtained in Example 1-2, 1,4-dioxane (10 mL) and a 4N hydrochloric acid/1,4-dioxane solution (17 mL) were added. The reaction solution was stirred at room temperature for 2 hours. The solvent was distilled off under reduced pressure, ethyl acetate (25 mL) was added. The resultant solution was neutralized with saturated aqueous sodium hydrogen carbonate solution and stirred at room temperature. The solid substance generated was obtained by filtration. In this manner, the title compound (3.97 g) was obtained.

### Example 2

### (2S)-2-Amino-3-(3-{3-[([1,1'-biphenyl]-4-yl)oxy]-3-phenylazetidin-1-sulfonyl}phenyl)propanoic acid

The title compound was synthesized in the same manner as in Example 1 except that the compound obtained in Reference Example 4 was used.

### Example 3

### (2S)-2-Amino-3-(3-{3-[([1,1'-biphenyl]-3-yl)oxy]-3-phenylazetidin-1-sulfonyl}phenyl)propanoic acid

The title compound was synthesized in the same manner as in Example 1 except that a compound, which was obtained in the same manner as in Reference Example 4 except that 3-phenylphenol was used, was employed.

### Example 4

### (2S)-3-{3-[3-(4-Acetyl-3-fluorophenoxy)-3-(4-fluorophenyl)azetidine-l-sulfonyl]phenyl}-2-aminopropanoic acid monohydrochloride

The title compound was synthesized in the same manner as in Example 1 except that a compound, which was obtained in the same manner as in Reference Example 1a except that 1-(2-fluoro-4-hydroxyphenyl)ethan-1-one was used, was employed.

### Example 5

### (2S)-3-{3-[3-(3-Acetyl-4-fluorophenoxy)-3-(4-fluorophenyl)azetidine-1-sulfonyl]phenyl}-2-aminopropanoic acid monohydrochloride

The title compound was synthesized in the same manner as in Example 1 except that a compound, which was obtained in the same manner as in Reference Example 1a except that 1-(2-fluoro-5-hydroxyphenyl)ethan-1-one was used, was employed.

### Example 6

### (2S)-2-Amino-3-(3-{3-phenyl-3-[(quinoxalin-6-yl)oxy]azetidin-1-sulfonyl}phenyl)propanoic acid monohydrochloride

### (Example 6-1)

### tert-Butyl (2S)-2-[(tert-butoxycarbonyl)amino]-3-(3-{3-phenyl-3-[(quinoxalin-6-yl)oxy]azetidin-1-sulfonyl}phenyl)propanoate

A tetrahydrofuran (2 mL)/DMF (1 mL) solution of the compound (149 mg, 0.310 mmol) obtained in Reference Example 10, the compound obtained in Reference Example 10 and 6-(3-phenylazetidin-3-yl)oxyquinoxaline (1 00 mg, 0.31 mmol) obtained from quinoxalin-6-ol in the same manner as Reference Example 3, was cooled to 0°C and diisopropylethylamine (120 mg, 0.93 mmol) was added thereto. After the reaction solution was stirred at room temperature for 16 hours, water (2 mL) was added to the reaction solution, which was extracted with ethyl acetate (2 mL × 2). The organic layer was washed with saturated saline solution and dried over sodium sulfate. After the sodium sulfate was filtered off, the filtrate was concentrated under reduced pressure. The residue was purified by silica gel column chromatography (petroleum ether/ethyl acetate) to obtain the title compound (65 mg) .

### (Example 6-2)

### (2S)-2-Amino-3-(3-{3-phenyl-3-[(quinoxalin-6-yl)oxy]azetidin-1-sulfonyl}phenyl)propanoic acid monohydrochloride

To a dichloromethane solution (2 mL) of the compound (65 mg, 0.098 mmol) obtained in Example 6-1, trifluoromethanesulfonic acid (TFA) (0.2 mL) was added. The reaction solution was stirred at 30°C for 16 hours. The reaction solution was concentrated and the obtained residue was diluted with methanol (2 mL), neutralized with ammonia water, and then, purified by high performance liquid chromatography (0.05 %hydrochloric acid/acetonitrile) to obtain the title compound (28 mg) as a solid substance.

### Example 7

### (2S)-2-Amino-3-(3-{3-[(2,3-dihydro-1,4-benzodioxin-6-yl)oxy]-3-phenylazetidin-1-sulfonyl}phenyl)propanoic acid monohydrochloride

The title compound was synthesized in the same manner as in Example 6 except that 2,3-dihydro-1,4-benzodioxin-6-ol was used.

### Example 8

### (2S)-2-Amino-3-(3-{3-(4-fluorophenyl)-3-[(1-oxo-1,2,3,4-tetrahydroisoquinolin-6-yl)oxy]azetidin-1-sulfonyl}phenyl)propanoic acid monohydrochloride

The title compound was synthesized in the same manner as in Example 6 except that a compound, which was synthesized in the same manner as in Reference Example 1a except that 6-hydroxy-3,4-dihydroisoquinolin-1(2H)-one was used, was employed.

### Example 9

### (2S)-3-{3-[3-(4-Acetylphenoxy)-3-(4-chlorophenyl)azetidin-lyl]sulfonylphenyl}-2-aminopropanoic acid monohydrochloride

The title compound was synthesized in the same manner as in Example 1 except that a compound, which was synthesized in the same manner as in Reference Example 3 except that 4-chlorophenylmagnesium bromide and 1-(4-hydroxyphenyl)ethan-1-one were used, was employed.

### Example 10

### (2S)-2-Amino-3-(3-{3-[(1H-indazol-6-yl)oxy]-3-phenylazetidine-1-sulfonyl}phenyl)propanoic acid monohydrochloride

The title compound was synthesized in the same manner as in Example 6 except that a compound, which was synthesized in the same manner as in Reference Example 3 except that 1H-indazol-6-ol was used, was employed.

### Example 11

### (2S)-2-Amino-3-[3-(3-phenyl-3-{4-[(propan-2-yl)carbamoyl]phenoxy}azetidin-1-sulfonyl)phenyl]propanoic acid monoformate

The title compound was synthesized in the same manner as in Example 1 except that a compound, which was synthesized in the same manner as in Reference Example 12 except that isopropylamine was used, was employed.

### Example 12

### (2S)-2-Amino-3-(3-{3-[4-(difluoromethoxy)phenoxy]-3-phenylazetidin-1-sulfonyl}phenyl)propanoic acid monoformate

The title compound was synthesized in the same manner as in Example 1 except that a compound, which was synthesized in the same manner as in Reference Example 3 except that 4-(difluoromethoxy)phenol was used, was employed.

### Example 13

### (2S)-2-Amino-3-{3-[3-(4-cyanophenoxy)-3-(4-methylphenyl)azetidine-1-sulfonyl]phenyl}propanoic acid monoformate

The title compound was synthesized in the same manner as in Example 1 except that a compound, which was synthesized in the same manner as in Reference Example 3 except that 4-methylphenylmagnesium bromide and 4-cyanophenol were used, was employed.

### Example 14

### (2S)-3-{3-[3-(4-Acetylphenoxy)-3-(4-methylphenyl)azetidine-1-sulfonyl]phenyl}-2-aminopropanoic acid monoformate

The title compound was synthesized in the same manner as in Example 1 except that a compound, which was synthesized in the same manner as in Reference Example 3 except that 4-methylphenylmagnesium bromide and 1-(4-hydroxyphenyl)ethan-1-one were used, was employed.

### Example 15

### (2S)-Amino-3-(3-((3-(3-fluorophenoxy)-3-(4-fluorophenyl)azetidin-1-yl)sulfonyl)phenyl)propanoic acid

### (Example 15-1)

### tert-Butyl (2S)-((tert-butoxycarbonyl)amino)-3-(3-((3-(3-fluorophenoxy)-3-(4-fluorophenyl)azetidin-1-yl)sulfonyl)phenyl)propanoate

The title compound was synthesized in the same manner as in Example 1-1 except that 3-(3-fluorophenoxy)-3-(4-fluorophenyl)azetidine (which was synthesized in the same manner as in Reference Example 3 except that 4-fluorophenylmagnesium bromide was used) and the compound obtained in Reference Example 10 were employed.

### (Example 15-2)

### (2S)-Amino-3-(3-((3-(3-fluorophenoxy)-3-(4-fluorophenyl)azetidin-1-yl)sulfonyl)phenyl)propanoic acid

The compound (1.21 g) obtained in Example 15-1 was dissolved in a 1N hydrochloric acid/acetic acid solution (13 mL). The reaction solution was stirred at 45°C for 2.5 hours and concentrated under reduced pressure. To the residue, n-hexane/dichloromethane (2/1) was added to obtain a suspension. The precipitate was obtained by filtration. In this manner, the title compound (935 mg) containing a small amount of impurities was obtained. A part (100 mg) of the solid substance was dissolved in a small amount of acetic acid while heating and cooled to room temperature. Water (1.6 mL) was added to the solution and a precipitate was obtained by filtration. The solid substance was again dissolved in a small amount of acetic acid while heating and cooled to room temperature. The precipitate was obtained by filtration. In this manner, the title compound (69 mg) was obtained.

### Example 16

### (2S)-Amino-3-(3-((3-phenyl-3-(p-tolyloxy)azetidin-1-yl)sulfonyl)phenyl)propanoic acid

The title compound was synthesized in the same manner as in Example 15 except that the compound, which was synthesized in the same manner as in Reference Example 3 except that 4-methylphenol was used, was employed.

### Example 17

### (2S)-Amino-3-(3-((3-(3-chloro-4-fluorophenoxy)-3-phenylazetidin-1-yl)sulfonyl)phenyl)propanoic acid

The title compound was synthesized in the same manner as in Example 15 except that the compound, which was synthesized in the same manner as in Reference Example 3 except that 3-chloro-4-fluorophenol was used, was employed.

### Example 18

### (2S)-Amino-3-(3-((3-(4-cyanophenoxy)-3-phenylazetidin-1-yl)sulfonyl)phenyl)propanoic acid

The title compound was synthesized in the same manner as in Example 15 except that the compound, which was synthesized in the same manner as in Reference Example 3 except that 4-cyanophenol was used, was employed.

### Example 19

### (2S)-2-Amino-3-[3-[3-(4-fluorophenyl)-3-(4-propanoylphenoxy)azetidin-1-yl]sulfonylphenyl]propanoic acid monotrifluoroacetate

### (Example 19-1)

### tert-Butyl (2S)-2-(tert-butoxycarbonylamino)-3-[3-[3-(4-fluorophenyl)-3-(4-propanoylphenoxy)azetidin-1-yl]sulfonylphenyl]propanoate

The title compound was synthesized in the same manner as in Example 15-1 except that 1-(4-{[3-(4-fluorophenyl)azetidin-3-yl]oxy}phenyl)propan-1-one, which was synthesized in the same manner as in Reference Example 3 except that 4-fluorophenylmagnesium bromide and 1-(4-hydroxyphenyl)propan-1-one were used, was employed.

### (Example 19-2)

### (2S)-2-Amino-3-[3-[3-(4-fluorophenyl)-3-(4-propanoylphenoxy)azetidin-1-yl]sulfonylphenyl]propanoic acid monotrifluoroacetate

The compound (100 mg, 0.126 mmol) obtained in Example 19-1 was dissolved in dichloromethane (3 mL). Then, 4N hydrochloric acid/dioxane (0.5 mL) was added thereto and the reaction solution was stirred at room temperature for 2 hours and concentrated. The obtained residue was diluted with methanol (3 mL) and neutralized by adding saturated sodium hydrogen carbonate. The solid substance precipitated was obtained by filtration and purified by high performance liquid chromatography (aqueous solution containing 0.1 % trifluoro acetic acid /acetonitrile) to obtain the title compound as a solid substance.

### Example 20

### (2S)-2-Amino-3-{3-[3-(4-carbamoylphenoxy)-3-phenylazetidine-1-sulfonyl]phenyl}propanoic acid monoformate

The title compound was synthesized in the same manner as in Example 19 except that the compound obtained in Reference Example 12 was used.

### Example 21

### (2S)-2-Amino-3-[3-[4-(dimethylamino)phenoxy]-3-(4-fluorophenyl)azetidin-1-yl]sulfonylphenyl]propanoic acid monohydrochloride

The title compound was synthesized in the same manner as in Example 6 except that the compound, which was synthesized in the same manner as in Reference Example 1a except that 4-(dimethylamino)phenol was used, was employed.

### Example 22

### (2S)-2-Amino-3-{3-[3-(4-carbamoylphenoxy)-3-(4-fluorophenyl)azetidine-1-sulfonyl]phenyl}propanoic acid monotrifluoroacetate

The title compound was synthesized in the same manner as in Example 19 except that the compound, which was synthesized in the same manner as in Reference Example 12 except that 4-fluorophenylmagnesium bromide was used, was employed.

### Example 23

### 1-Oxo-1-[(propan-2-yl)amino]propan-2-yl (2S)-2-amino-3-(3-{3-phenyl-3-[(²H₅)phenyloxy]azetidin-1-sulfonyl}phenyl)propanoate monohydrochloride

### (Example 23-1)

### 1-Oxo-1-[(propan-2-yl)amino]propan-2-yl (2S)-2-[(tert-butoxycarbonyl)amino]-3-(3-{3-phenyl-3-[(²H₅)phenyloxy]azetidin-1-sulfonyl}phenyl)propanoate

The title compound was synthesized in the same manner as in Example 1-1 except that the compounds, where were obtained in Reference Example 15 and Reference Example 9, were used.

### (Example 23-2)

### 1-Oxo-1-[(propan-2-yl)amino]propan-2-yl (2S)-2-amino-3-(3-{3-phenyl-3-[(²H₅)phenyloxy]azetidin-1-sulfonyl}phenyl)propanoate monohydrochloride

The title compound was synthesized in the same manner as in Example 1-3 except that the compound, which was obtained in Example 23-1 was used.

### Example 24

### (2S)-2-Amino-3-(3-{3-phenyl-3-[(²H₅)phenyloxy]azetidin-1-sulfonyl}phenyl)propanoic acid

The title compound was synthesized in the same manner as in Example 1-2 except that the compound, which was obtained in Example 23, was used.

### Example 25

### 1-(Dimethylamino)-3-methyl-1-oxobutan-2-yl (2S)-2-amino-3-(3-{3-phenyl-3-[(²H₅)phenyloxy]azetidin-1-sulfonyl}phenyl)propanoate monohydrochloride

The title compound was synthesized in the same manner as in Example 1-1 and Example 1-3 except that the compounds obtained in Reference Example 15 and Reference Example 7 were used.

### Example 26

### 1-Oxo-1-[(propan-2-yl)amino]propan-2-yl (2S)-2-amino-3-(3-{3-(4-fluorophenyl)-3-[(²H₅)phenyloxy]azetidin-1-sulfonyl}phenyl)propanoate monohydrochloride

### (Example 26-1)

### 1-Oxo-1-[(propan-2-yl)amino]propan-2-yl (2S)-2-[(tert-butoxycarbonyl)amino]-3-(3-{3-(4-fluorophenyl)-3-[(²H₅)phenyloxy]azetidin-1-sulfonyl}phenyl)propanate

To (2S)-2-[(tert-butoxycarbonyl)amino]-3-(3-{3-(4-fluorophenyl)-3-[(²H₅)phenyloxy]azetidine)-1-sulfonyl}phenyl)propanoic acid (260 mg, 0.45 mmol) obtained in the same manner as in Example 1-1 and Example 1-2 except that the compound obtained in Reference Example 16 was used, 2-chloro-N-isopropyl-propanamide (120 mg, 0.90 mmol), sodium iodide (120 mg 0.90 mmol) and potassium carbonate (240 mg, 1.8 mmol), DMF (6 mL) was added. The reaction solution was stirred at 80°C for 5 hours. To the reaction solution, water (20 mL) was added. The reaction solution was extracted with ethyl acetate and the organic layer was concentrated. The obtained residue was purified by silica gel column chromatography (n-hexane/ethyl acetate) to obtain the title compound (200 mg) as an oily substance.

### (Example 26-2)

### 1-Oxo-1-[(propan-2-yl)amino]propan-2-yl (2S)-2-amino-3-(3-{3-(4-fluorophenyl)-3-[(²H₅)phenyloxy]azetidin-1-sulfonyl}phenyl)propanoate monohydrochloride

The title compound was synthesized in the same manner as in Example 1-3 except that the compound obtained Example 26-1 was used.

### Example 27

### 1-(Dimethylamino)-3-methyl-1-oxobutan-2-yl (2S)-2-amino-3-(3-{3-(4-fluorophenyl)-3-[(²H₅)phenyloxy]azetidin-1-sulfonyl}phenyl)propanoate monohydrochloride

The title compound was synthesized in the same manner as in Example 1-1 and Example 1-3 except that 3-(4-fluorophenyl)-3-[(²H₅)phenyloxy]azetidine, (which was obtained in the same manner as in Reference Example 3 except that (²H₅)phenol and 4-fluorophenylmagnesium bromide were used) and the compound obtained in Reference Example 7 were employed.

### Example 28

### 1-(Dimethylamino)-3-methyl-1-oxobutan-2-yl (2S)-2-amino-3-{3-[3-(4-carbamoylphenoxy)-3-phenylazetidin-1-sulfonyl]phenyl}propanoate monohydrochloride

The title compound was synthesized in the same manner as in Example 1-1 and Example 1-3 except that the compound, which was obtained Example 12 was used.

### Example 29

### 1-Oxo-1-[(propan-2-yl)amino]propan-2-yl (2S)-2-amino-3-{3-[3-(4-fluorophenyl)-3-(4-propanoylphenoxy)azetidin-1-sulfonyl]phenyl}propanoate monohydrochloride

The title compound was synthesized in the same manner as in Example 23 except that 1-(4-{[3-(4-fluorophenyl)azetidin-3-yl]oxy}phenyl)propan-1-one (which was synthesized in the same manner as in Reference Example 3 except 4-fluorophenylmagnesium bromide and 1-(4-hydroxyphenyl)propan-1-one were used) and the compound obtained in Reference Example 9 were employed.

### Example 30

### 1-Oxo-1-[(propan-2-yl)amino]propan-2-yl (2S)-2-amino-3-(3-{3-[4-(dimethylamino)phenoxy]-3-(4-fluorophenyl)azetidin-1-sulfonyl}phenyl)propanoate monohydrochloride

The title compound was synthesized in the same manner as in Example 23 except that the compound, which was synthesized in the same manner as in Reference Example 1a except that 4-(dimethylamino)phenol was used, and the compound obtained in Reference Example 9 were employed.

### Example 31

### (2S)-1-(Dimethylamino)-1-oxopropan-2-yl (2S)-2-amino-3-{3-[3-(3-fluorophenoxy)-3-phenylazetidin-1-sulfonyl]phenyl}propanoate monotrifluoroacetate

### (Example 31-1)

### (2S)-1-(Dimethylamino)-1-oxopropan-2-yl (2S)-2-[(tert-butoxycarbonyl)amino]-3-{3-[3-(3-fluorophenoxy)-3-phenylazetidin-1-sulfonyl]phenyl}propanoate

To a dichloromethane solution (25 mL) of the compound (2.44 g, 4.27 mmol) obtained in Example 1-2 and (2S)-2-hydroxy-N,N-dimethyl-propanamide (0.50 g, 4.27 mmol), EDCI (2.45 g, 12.8 mmol) and DMAP (521 mg, 4.27 mmol) were added. The reaction solution was stirred at 40°C for 12 hours. To the reaction solution, water (50 mL) was added. The reaction solution was extracted with ethyl acetate (50 mL × 3). The organic layer was washed with saturated saline solution and dried over sodium sulfate. After the sodium sulfate was filtered off, the filtrate was concentrated under reduced pressure. The residue was purified by silica gel column chromatography (n-hexane/ethyl acetate) to obtain the title compound (1.0 g) as a solid substance.

### (Example 31-2)

### (2S)-1-(Dimethylamino)-1-oxopropan-2-yl (2S)-2-amino-3-{3-[3-(3-fluorophenoxy)-3-phenylazetidin-1-sulfonyl]phenyl}propanoate monotrifluoroacetate

The title compound was synthesized in the same manner as in Example 1-3 except that the compound obtained Example 31-1 was used.

### Example 32

### (2R)-1-(Dimethylamino)-1-oxopropan-2-yl (2S)-2-amino-3-{3-[3-(3-fluorophenoxy)-3-phenylazetidin-1-sulfonyl]phenyl}propanoate monotrifluoroacetate

The title compound was synthesized in the same manner as in Example 31 except that (2R)-2-hydroxy-N,N-dimethyl-propanamide was used.

### Example 33

### 1-Oxo-1-[(propan-2-yl)amino]propan-2-yl (2S)-2-amino-3-{3-[3-(4-cyanophenoxy)-3-phenylazetidin-1-sulfonyl]phenyl}propanoate monohydrochloride

The title compound was synthesized in the same manner as in Example 23 except that the compound synthesized in the same manner as in Reference Example 3 except that 4-cyanophenol was used, was employed.

### Example 34

### (2S)-2-Amino-3-{3-[3-(4-cyanophenoxy)-3-(4-fluorophenyl)azetidine-1-sulfonyl]phenyl}propanoic acid monohydrochloride

The title compound was synthesized in the same manner as in Example 15 except that the compound, which was synthesized in the same manner as in Reference Example 1a except that 4-cyanophenol was used, was employed.

### Example 35

### (2S)-2-Amino-3-{3-[3-(4-chlorophenoxy)-3-(4-fluorophenyl)azetidine-1-sulfonyl]phenyl}propanoic acid monohydrochloride

The title compound was synthesized in the same manner as in Example 15 except that the compound, which was synthesized in the same manner as in Reference Example 1a except that 4-chlorophenol was used, was employed.

### Example 36

### (2S)-2-Amino-3-{3-[3-(4-chlorophenoxy)-3-(3,4-difluorophenyl)azetidin-1-sulfonyl]phenyl}propanoic acid monohydrochloride

The title compound was synthesized in the same manner as in Example 15 except that a compound, which was obtained in the same manner as in Reference Example 3 except that 3,4-difluorophenylmagnesium bromide and 4-chlorophenol were used, was employed.

### Example 37

### (2S)-2-Amino-3-{3-[3-(3,4-difluorophenyl)-3-(4-fluorophenoxy)azetidin-1-sulfonyl]phenyl}propanoic acid monohydrochloride

The title compound was synthesized in the same manner as in Example 15 except that a compound, which was obtained in the same manner as in Reference Example 3 except that 3,4-difluorophenylmagnesium bromide and 4-fluorophenol were used, was employed.

### Example 38

### (2S)-2-Amino-3-{3-[3-(4-cyanophenoxy)-3-(3,4-difluorophenyl)azetidin-1-sulfonyl]phenyl}propanoic acid monohydrochloride

The title compound was synthesized in the same manner as in Example 15 except that a compound, which was obtained in the same manner as in Reference Example 3 except that 3,4-difluorophenylmagnesium bromide and 4-cyanophenol were used, was employed.

### Example 39

### (2S)-2-Amino-3-{3-[3-(4-carbamoylphenoxy)-3-(3,4-difluorophenyl)azetidin-1-sulfonyl]phenyl}propanoic acid monohydrochloride

The title compound was synthesized in the same manner as in Example 15 except that the compound, which was synthesized in the same manner as in Reference Example 12 except that 3,4-difluorophenylmagnesium bromide was used, was employed.

### Example 40

### (2S)-2-Amino-3-(3-{3-[(1-hydroxy-1,3-dihydro-2,1-benzoxaborol-5-yl)oxy]-3-phenylazetidin-1-sulfonyl}phenyl)propanoic acid monohydrochloride

The title compound was synthesized in the same manner as in Example 1 except that the compound, which was synthesized in the same manner as in Reference Example 1 except that the compound obtained in Reference Example 3a was used, was employed.

### Example 41

### 1-(Dimethylamino)-3-methyl-1-oxobutan-2-yl (2S)-2-amino-3-{3-[3-(4-chlorophenyl)-3-(4-fluorophenoxy)azetidin-1-sulfonyl]phenyl}propanoate monohydrochloride

The title compound was synthesized in the same manner as in Example 1-1 and Example 1-3 except that the compound, which was synthesized in the same manner as in Reference Example 3 except that 4-chlorophenylmagnesium bromide and 4-fluorophenol were used, and the compound obtained in Reference Example 7 were employed.

### Example 42

### 1-(Dimethylamino)-3-methyl-1-oxobutan-2-yl (2S)-2-amino-3-{3-[3-(4-cyanophenoxy)-3-phenylazetidin-1-sulfonyl]phenyl}propanoate monohydrochloride

The title compound was synthesized in the same manner as in Example 1-1 and Example 1-3 except that a compound, which was obtained in the same manner as in Reference Example 3 except that 4-cyanophenol was used, and the compound obtained in Reference Example 7 were employed.

### Example 43

### (2S)-2-Amino-3-(3-{[3-(3-methylphenoxy)-3-phenylazetidin-1-yl]sulfonyl}phenyl)propanoic acid monohydrochloride

The title compound was synthesized in the same manner as in Example 1 except that the compound, which was synthesized in the same manner as in Reference Example 3 except that 3-methylphenol was used, was employed.

### Example 44

### 1-(Dimethylamino)-1-oxopropan-2-yl (2S)-2-amino-3-(3-{[3-(3-fluoro-4-methylphenoxy)-3-phenylazetidin-1-yl]sulfonyl}phenyl)propanoate monohydrochloride

The title compound was synthesized in the same manner as in Example 23 except that a compound, which was obtained in the same manner as in Reference Example 3 except that 3-fluoro-4-methylphenol was used, and the compound obtained in Reference Example 8 were employed.

### Example 45

### (2S)-2-Amino-3-(3-{[3-(3-fluoro-4-methylphenoxy)-3-phenylazetidin-1-yl]sulfonyl}phenyl)propanoic acid monoformate

The title compound was synthesized in the same manner as in Example 1-2 except that the compound obtained in Example 44 was used.

### Example 46

### (2S)-2-Amino-3-[3-({3-(4-fluorophenyl)-3-[(6-methylpyridin-3-yl)oxy]azetidin-1-yl}sulfonyl)phenyl]propanoic acid dihydrochloride

The title compound was synthesized in the same manner as in Example 1 except that a compound, which was obtained in the same manner as in Reference Example 1a except that 6-methylpyridin-3-ol was used, was employed.

### Example 47

### (2S)-2-Amino-3-(3-{[3-(4-fluorophenoxy)-3-(5-fluoropyridin-2-yl)azetidin-1-yl]sulfonyl}phenyl)propanoic acid dihydrochloride

The title compound was synthesized in the same manner as in Example 1 except that the compound obtained in Reference Example 17 was used.

### Example 48

### (2S)-2-Amino-3-[3-({3-[(6-ethylpyridin-3-yl)oxy]-3-(4-fluorophenyl)azetidin-l-yl}-sulfonyl)phenyl]propanoic acid dihydrochloride

The title compound was synthesized in the same manner as in Example 1 except that a compound, which was obtained in the same manner as in Reference Example 1a except that 6-ethylpyridin-3-ol was used, was employed.

### Example 49

### (2S)-2-Amino-3-[3-(({3-[(5-fluoro-6-methylpyridin-3-yl)oxy]-3-(4-fluorophenyl)azetidin-1-yl)sulfonyl)phenyl]propanoic acid dihydrochloride

The title compound was synthesized in the same manner as in Example 1 except that a compound, which was obtained in the same manner as in Reference Example 1a except that 5-fluoro-6-methylpyridin-3-ol was used, was employed.

### Example 50

### (2S)-2-Amino-3-(3-{[3-(5-fluoropyridin-2-yl)-3-(4-methylphenoxy)azetidin-1-yl]sulfonyl}phenyl)propanoic acid dihydrochloride

The title compound was synthesized in the same manner as in Example 1 except that the compound obtained in Reference Example 13 was used.

### Example 51

### (4-1[1-(13-[(2S)-2-Amino-3-oxo-3-1[1-oxo-1-(propan-2-ylamino)propan-2-yl]oxy}propyl]phenyl}sulfonyl)-3-phenylazetidin-3-yl]oxy}phenyl)boronic acid, monohydrochloride

The title compound was synthesized in the same manner as in Example 1-1 and Example 1-3 except that the compound obtained in Reference Example 2 and the compound obtained in Reference Example 9 were employed.

### Example 52

### (2S)-2-Amino-3-(3-{[3-(4-boronophenoxy)-3-phenylazetidin-1-yl]sulfonyl}phenyl)propanoic acid monoformate

The title compound was synthesized in the same manner as in Example 1-2 except that the compound obtained in Example 51 was used.

### Example 53

### (2S)-2-Amino-3-(3-{[3-(4-chlorophenoxy)-3-(4-chlorophenyl)azetidin-1-yl]sulfonyl}phenyl)propanoic acid monoformate

The title compound was synthesized in the same manner as in Example 15 except that the compound, which was synthesized in the same manner as in Reference Example 3 except that 4-chlorophenylmagnesium bromide and 4-chlorophenol were used, was employed.

### Example 54

### (2S)-2-Amino-3-(3-{[3-(4-chlorophenyl)-3-(4-fluorophenoxy)azetidin-1-yl]sulfonyl}phenyl)propanoic acid monoformate

The title compound was synthesized in the same manner as in Example 15 except that the compound, which was synthesized in the same manner as in Reference Example 3 except that 4-chlorophenylmagnesium bromide and 4-fluorophenol were used, was employed.

### Example 55

### (2S)-2-Amino-3-(3-{[3-(4-chlorophenyl)-3-(3-fluorophenoxy)azetidin-1-yl]sulfonyl}phenyl)propanoic acid monoformate

The title compound was synthesized in the same manner as in Example 15 except that the compound, which was synthesized in the same manner as in Reference Example 3 except that 4-chlorophenylmagnesium bromide and 3-fluorophenol were used, was employed.

### Example 56

### (2S)-2-Amino-3-[3-({3-[(1-ethyl-1H-pyrazol-4-yl)oxy]-3-(4-fluorophenyl)azetidin-1-yl)sulfonyl)phenyl]propanoic acid dihydrochloride

The title compound was synthesized in the same manner as in Example 1 except that the compound, which was synthesized in the same manner as in Reference Example 1a except that 1-ethyl-1H-pyrazol-4-ol was used, was employed.

### Example 57

### (2S)-2-Amino-3-[3-({3-(4-fluorophenyl)-3-[(6-methoxypyridin-3-yl)oxy]azetidin-1-yl}sulfonyl)phenyl]propanoic acid monoformate

The title compound was synthesized in the same manner as in Example 1 except that the compound, which was synthesized in the same manner as in Reference Example 1a except that 6-methoxypyridin-3-ol was used, was employed.

### Example 58

### (2S)-2-Amino-3-[3-({3-(4-fluorophenyl)-3-[(1-hydroxy-1,3-dihydro-2,1-benzoxaborol-5-yl)oxy]azetidin-1-yl)sulfonyl)phenyl]propanoic acid monoformate

The title compound was synthesized in the same manner as in Example 23 and Example 1-2 except that the compound obtained in Reference Example 1 was used.

### Example 59

### (2S)-2-Amino-3-(3-{[3-(3-fluorophenoxy)-3-(4-methylphenyl)azetidin-1-yl]sulfonyl}phenyl)propanoic acid monoformate

The title compound was synthesized in the same manner as in Example 15 except that the compound, which was synthesized in the same manner as in Reference Example 3 except that 4-methylphenylmagnesium bromide and 3-fluorophenol were used, was employed.

### Example 60

### (2S)-3-(3-{[3-(4-acetylphenoxy)-3-(5-fluoropyridin-2-yl)azetidin-1-yl]sulfonyl}phenyl)-2-aminopropanoic acid dihydrochloride

The title compound was synthesized in the same manner as in Example 1 except that the compound, which was synthesized in the same manner as in Reference Example 13 except that 1-(4-hydroxyphenyl)ethan-1-one was used, was employed.

### Example 61

### (2S)-2-Amino-3-(3-{[3-(4-carbamoylphenoxy)-3-(4-chlorophenyl)azetidin-1-yl]sulfonyl}phenyl)propanoic acid monohydrochloride

The title compound was synthesized in the same manner as in Example 1 except that the compound, which was synthesized in the same manner as in Reference Example 12 except that 4-chlorophenylmagnesium bromide was used, was employed.

### Example 62

### 1-(Dimethylamino)-1-oxopurpan-2-yl (2S)-2-amino-3-(3-{[3-(4-fluorophenoxy)-3-(5-fluoropyridin-2-yl)azetidin-1-yl]sulfonyl}phenyl)propanoate dihydrochloride

The title compound was synthesized in the same manner as in Example 26 except that the compound obtained in Reference Example 17 and 2-chloro-N,N-dimethylpropanamide were used.

### Example 63

### 1-(Dimethylamino)-1-oxopropan-2-yl (2S)-2-amino-3-[3-({3-(4-fluorophenyl)-3-[(6-methylpyridin-3-yl)oxy]azetidin-1-yl}sulfonyl)phenyl]propanoate dihydrochloride

The title compound was synthesized in the same manner as in Example 26 except that a compound, which was obtained in the same manner as in Reference Example 1a except 6-methylpyridin-3-ol was used, and 2-chloro-N,N-dimethylpropanamide were used, was employed.

### Example 64

### 1-(Dimethylamino)-3-methyl-1-oxobutan-2-yl (2S)-2-amino-3-(3-{[3-(4-methylphenoxy)-3-phenylazetidin-1-yl]sulfonyl}phenyl)propanoate monohydrochloride

The title compound was synthesized in the same manner as in Example 1-1 and Example 1-3 except that a compound, which was obtained in the same manner as in Reference Example 3 except 4-methylphenol was used, and the compound obtained in Reference Example 7 were employed.

### Example 65

### 1-(Dimethylamino)-3-methyl-1-oxobutan-2-yl (2S)-2-amino-3-(3-{ [3-(4-chlorophenoxy)-3-phenylazetidin-1-yl]sulfonyl}phenyl)propanoate monohydrochloride

The title compound was synthesized in the same manner as in Example 31 except that the compound, which was synthesized in the same manner as in Reference Example 3 except 4-chlorophenol was used, and 2-hydroxy-N,N,3-trimethylbutanamide were used, was employed.

### Example 66

### 1-(Dimethylamino)-3-methyl-1-oxobutan-2-yl (2S)-2-amino-3-(3-{ [3-(3-chloro-4-fluorophenoxy)-3-phenylazetidin-1-yl]sulfonyl}phenyl)propanoate monohydrochloride

The title compound was synthesized in the same manner as in Example 1-1 and Example 1-3 except that a compound, which was obtained in the same manner as in Reference Example 3 except 3-chloro-4-fluorophenol was used, and the compound obtained in Reference Example 7 were employed.

### Example 67

### 1-(Morpholin-4-yl)-1-oxopropan-2-yl (2S)-2-amino-3-[3-({3-(4-fluorophenyl)-3-[(6-methylpyridin-3-yl)oxy]azetidin-1-yl}sulfonyl)phenyl]propanoate dihydrochloride

The title compound was synthesized in the same manner as in Example 1-1 and Example 1-3 except that the compound obtained in Reference Example 6 and the compound obtained in Reference Example 13 were employed.

### Example 68

### (1-Oxo-1-(propan-2-ylamino)propan-2-yl (2S)-2-amino-3-(3-{[3-(4-chlorophenyl)-3-(4-fluorophenoxy)azetidin-1-yl]sulfonyl}phenyl)propanoate monohydrochloride

The title compound was synthesized in the same manner as in Example 1-1 and Example 1-3 except that a compound, which was obtained in the same manner as in Reference Example 3 except that 4-chlorophenylmagnesium bromide and 4-fluorophenol were used, and the compound obtained in Reference Example 7 were employed.

### Example 69

### 1-(Dimethylamino)-3-methyl-1-oxobutan-2-yl (2S)-2-amino-3-(3-{ [3-(4-fluorophenoxy)-3-(5-fluoropyridin-2-yl)azetidin-1-yl]sulfonyl}phenyl)propanoate dihydrochloride

### (Example 69-1)

### 1-(Dimethylamino)-3-methyl-1-oxobutan-2-yl (2S)-2-[(tert-butoxycarbonyl)amino]-3-(3-{[3-(4-fluorophenoxy)-3-(5-fluoropyridin-2-yl)azetidin-1-yl]sulfonyl}phenyl)propanoate

To a dichloromethane (2 mL) solution of tert-butyl 3-(4-fluorophenoxy)-3-(5-(fluoropyridin-2-yl)azetidine-1-carboxylate (237 mg, 0.654 mmol) obtained in Reference Example 17, trifluoroacetic acid (1 mL) was added. The reaction solution was stirred at room temperature for 30 minutes. To this, toluene (3 mL) was added and the reaction solution was concentrated under reduced pressure to obtain 5-fluoro-2-[3-(4-fluorophenoxy)azetidin-3-yl]pyridine trifluoroacetate as a light yellow oil. To a dichloromethane (7 mL) solution of 5-fluoro-2-[3-(4-fluorophenoxy)azetidin-3-yl]pyridine trifluoroacetate obtained, 1-(dimethylamino)-3-methyl-1-oxobutan-2-yl (2S)-2-[(tert-butoxycarbonyl)amino]-3-[3-(chlorosulfonyl)phenyl]propanoate (290 mg, 0.591 mmol) obtained in Reference Example 7 and N,N-diisopropylethylamine (0.449 mL, 2.62 mmol) were added. The reaction solution was stirred at room temperature overnight. The residue, which was obtained by distilling away the solvent under reduced pressure, was purified by silica gel column chromatography [elution solvent: hexane/ethyl acetate = 3/1-1/2 (V/V)] to obtain the title compound (363 mg, 77%, two steps) as a colorless solid substance.

### (Example 69-2)

The title compound was synthesized in the same manner as in Example 23-2 except that the compound, which was obtained in Example 69-1, was used.

### Example 70

### 1-(Dimethylamino)-3-methyl-1-oxobutan-2-yl (2S)-2-amino-3-[3-({3-(4-fluorophenyl)-3-[ (6-methylpyridin-3-yl)oxy]azetidin-1-yl}sulfonyl)phenyl]propanoate dihydrochloride

The title compound was synthesized in the same manner as in Example 69 except that a compound, which was obtained in the same manner as in Reference Example 1a except of 6-methylpyridin-3-ol was used, was employed.

### Example 71

### 2-(Dimethylamino)-2-oxoethyl (2S)-2-amino-3-{3-[3-(3-fluorophenoxy)-3-phenylazetidin-1-sulfonyl]phenyl}propanoate monohydrochloride

The title compound was synthesized in the same manner as in Example 26 except that the compound obtained in Example 1-2 and 2-chloro-N,N-dimethylacetamide were used.

### Example 72

### 3,3-Dimethyl-2-oxobutyl (2S)-2-amino-3-{3-[3-(3-fluorophenoxy)-3-phenylazetidin-1-sulfonyl]phenyl}propanoate monohydrochloride

The title compound was synthesized in the same manner as in Example 26 except that the compound obtained in Example 1-2 and 1-chloro-3,3-dimethylbutan-2-one were used.

### Example 73

### 2-Oxo-2-[(propan-2-yl)amino]ethyl (2S)-2-amino-3-{3-[3-(3-fluorophenoxy)-3-phenylazetidin-1-sulfonyl]phenyl}propanoate monochloride

The title compound was synthesized in the same manner as in Example 26 except that the compound obtained in Example 1-2 and 2-chloro-N-isopropylacetamide were used.

### Example 74

### 2-(tert-Butylamino)-2-oxoethyl (2S)-2-amino-3-{3-[3-(3-fluorophenoxy)-3-phenylazetidin-1-sulfonyl]phenyl}propanoate monohydrochloride

The title compound was synthesized in the same manner as in Example 26 except that the compound obtained in Example 1-2 and N-tert-butyl-2-chloroacetamide were used.

### Example 75

### 2-(Morpholin-4-yl)-2-oxoethyl (2S)-2-amino-3-{3-[3-(3-fluorophenoxy)-3-phenylazetidin-1-sulfonyl]phenyl}propanoate monohydrochloride

The title compound was synthesized in the same manner as in Example 26 except that the compound obtained in Example 1-2 and 2-chloro-1-(morpholin-4-yl)ethan-1-one were used.

### Example 76

### 1-(Diethylamino)-1-oxopropan-2-yl (2S)-2-amino-3-(3-{[3-(3-fluorophenoxy)-3-phenylazetidin-1-yl]sulfonyl}phenyl)propanoate monohydrochloride

The title compound was synthesized in the same manner as in Example 26 except that the compound obtained in Example 1-2 and 2-chloro-N,N-diethylpropanamide were used.

### Example 77

### 1-(tert-Butylamino)-1-oxopropan-2-yl (2S)-2-amino-3-(3-{[3-(3-fluorophenoxy)-3-phenylazetidin-1-yl]sulfonyl}phenyl)propanoate monohydrochloride

The title compound was synthesized in the same manner as in Example 26 except that the compound obtained in Example 1-2 and N-tert-butyl-2-chloropropanamide were used.

### Example 78

### 1-(Morpholin-4-yl)-1-oxopropan-2-yl (2S)-2-amino-3-(3-{[3-(3-fluorophenoxy)-3-phenylazetidin-1-yl]sulfonyl}phenyl)propanoate monohydrochloride

The title compound was synthesized in the same manner as in Example 26 except that the compound obtained in Example 1-2 and 2-chloro-1-(morpholin-4-yl)propan-1-one were used.

### Example 79

### 2-Oxopiperidin-3-yl (2S)-2-amino-3-(3-{[3-(3-fluorophenoxy)-3-phenylazetidin-1-yl]sulfonyl}phenyl)propanoate monohydrochloride

The title compound was synthesized in the same manner as in Example 31 except that the compound obtained in Example 1-2 and 3-hydroxypiperidin-2-one were used.

### Example 80

### 1-(Dimethylamino)-3-methyl-1-oxobutan-2-yl (2S)-2-amino-3-(3-{ [3-(3-fluorophenoxy)-3-phenylazetidin-1-yl]sulfonyl}phenyl)propanoate monohydrochloride

The title compound was synthesized in the same manner as in Example 31 except that the compound obtained in Example 1-2 and 2-hydroxy-N,N,3-trimethylbutanamide were used.

### Example 81

### (2R)-1-(Dimethylamino)-3,3-dimethyl-1-oxobutan-2-yl (2S)-2-amino-3-(3-{[3-(3-fluorophenoxy)-3-phenylazetidin-1-yl]sulfonyl}phenyl)propanoate monohydrochloride

The title compound was synthesized in the same manner as in Example 31 except that the compound obtained in Example 1-2 and (2R)-2-hydroxy-N,N,3,3-tetramethylbutanamide were used.

### Example 82

### 1-(Diethylamino)-3-methyl-1-oxobutan-2-yl (2S)-2-amino-3-(3-{ [3-(3-fluorophenoxy)-3-phenylazetidin-1-yl]sulfonyl}phenyl)propanoate monohydrochloride

The title compound was synthesized in the same manner as in Example 31 except that the compound obtained in Example 1-2 and N,N-diethyl-2-hydroxy-3-methylbutanamide were used.

### Example 83

### (Pyrimidin-2-yl)methyl (2S)-2-amino-3-(3-{[3-(3-fluorophenoxy)-3-phenylazetidin-1-yl]sulfonyl}phenyl)propanoate monoformate

The title compound was synthesized in the same manner as in Example 26 except that the compound obtained in Example 1-2 and 2-(chloromethyl)pyrimidine hydrochloride were used.

### Example 84

### 3-Amino-3-oxopropyl (2S)-2-amino-3-(3-{[3-(3-fluorophenoxy)-3-phenylazetidin-1-yl]sulfonyl}phenyl))propanoate monohydrochloride

The title compound was synthesized in the same manner as in Example 26 except that the compound obtained in Example 1-2 and 3-chloropropanamide were used.

### Example 85

### 3-(Dimethylamino)-2,2-dimethyl-3-oxopropyl (2S)-2-amino-3-(3-{ [3-(3-fluorophenoxy)-3-phenylazetidin-1-yl]sulfonyl}phenyl))propanoate monoformate

The title compound was synthesized in the same manner as in Example 26 except that the compound obtained in Example 1-2 and 3-chloro-N,N,2,2-tetramethyl-propanamide were used.

### Example 86

### (86a) (2S)-2-Amino-3-(3-{3-[(6-cyanopyridin-3-yl)oxy]-3-(4-fluorophenyl)azetidin-1-sulfonyl}phenyl)propanoic acid dihydrochloride

### (86b) (2S)-2-Amino-3-[3-({3-[(6-carbamoylpyridin-3-yl)oxy]-3-(4-fluorophenyl)azetidin-1-yl)sulfonyl)phenyl]propanoic acid monoformate

1-(Dimethylamino)-1-oxopurpan-2-yl (2S)-2-amino-3-(3-{3-[(6-cyanopyridin-3-yl)oxy]-3-(4-fluorophenyl)azetidin-1-sulfonyl}phenyl)propanoate and 1-(dimethylamino)-1-oxopurpan-2-yl (2S)-2-amino-3-[3-({3-[(6-carbamoylpyridin-3-yl)oxy]-3-(4-fluorophenyl)azetidin-1-yl)sulfonyl)phenyl]propanoate were obtained in the same manner as in Example 26 except that a compound, which was obtained in the same manner as in Reference Example 3 except that 4-fluorophenylmagnesium bromide and 5-hydroxypyridine-2-carbonitrile were used, was employed

The compounds obtained were each subjected to the same reaction as in Example 1-2 and appropriately purified. In this manner, the title compounds were obtained.

### Example 87

### (2S)-2-Amino-3-(3-{[3-(4-chlorophenoxy)-3-phenylazetidine-1-sulfonyl]phenyl}propanoic acid

The title compound was synthesized in the same manner as in Example 15 except that a compound, which was obtained in the same manner as in Reference Example 3 except that 4-chlorophenol was used, was employed.

### Example 88

### (2S)-2-Amino-3-(3-((3-(3-phenoxy-3-phenylazetidin-1-yl)sulfonyl)phenyl)propanoic acid

The title compound was synthesized in the same manner as in Example 15 except that the compound obtained in Reference Example 11 was used.

### Example 89

### (2S)-2-Amino-3-{3-[3-(4-fluorophenyl)-3-phenoxyazetidine-1-sulfonyl]phenyl}propanoic acid

The title compound was synthesized in the same manner as in Example 15 except that the compound obtained in Reference Example 14 was used.

### Example 90

### (2S)-2-Amino-3-(3-{3-[(1H-indazol-5-yl)oxy]-3-phenylazetidin-1-sulfonyl}phenyl)propanoic acid

The title compound was synthesized in the same manner as in Example 6 except that a compound, which was obtained in the same manner as in Reference Example 3 except that 1H-indol-5-ol was used, was employed.

### Example 91

### 1-(Dimethylamino)-3-methyl-1-oxobutan-2-yl (2S)-2-amino-3-[3-(3-phenoxy-3-phenylazetidin-1-sulfonyl)phenyl]propanoate monohydrochloride

The title compound was synthesized in the same manner as in Example 69 except that the compound obtained in Reference Example 11 was used.

### Example 92

### 1-(Dimethylamino)-3-methyl-1-oxobutan-2-yl (2S)-2-amino-3-(3-{3-[(2,3-dihydro-1,4-benzodioxin-6-yl)oxy]-3-phenylazetidin-1-sulfonyl}phenyl)propionoate monohydrochloride

The title compound was synthesized in the same manner as in Example 69 except that the compound, which was obtained in the same manner as in Reference Example 3 except that 2,3-dihydro-1,4-benzodioxin-6-ol was used, was employed.

### Example 93

### 1-(Dimethylamino)-3-methyl-1-oxobutan-2-yl (2S)-2-amino-3-(3-{ [3-(4-fluorophenyl)-3-phenoxyazetidin-1-yl]sulfonyl}phenyl)propanoate monohydrochloride

### (Example 93-1)

### 1-(Dimethylamino)-3-methyl-1-oxobutan-2-yl (2S)-2-[(tert-butoxycarbonyl)amino]-3-(3-{ [3-(4-fluorophenyl)-3-phenoxyazetidin-l-yl]sulfonyl}phenyl)propanoate

To a dichloromethane (3 mL) solution of tert-butyl 3-(4-fluorophenyl)-3-phenoxyazetidine-1-carboxylate (285 mg, 0.83 mmol) obtained in Reference Example 14, trifluoroacetic acid (1.3 mL) was added. The reaction solution was stirred at room temperature for 30 minutes. To the reaction solution, toluene (3 mL) was added and the resultant reaction solution was concentrated under reduced pressure to obtain 3-(4-fluorophenyl)-3-phenoxyazetidine trifluoroacetate as a light yellow oil. To a dichloromethane (8 mL) solution of 3-(4-fluorophenyl)-3-phenoxyazetidine trifluoroacetate obtained, 1-(dimethylamino)-3-methyl-1-oxobutan-2-yl (2S)-2-[(tert-butoxycarbonyl)amino]-3-[3-(chlorosulfonyl)phenyl]propanoate (367 mg, 0.747 mmol) obtained in Reference Example 7 and N,N-diisopropylethylamine (0.568 mL, 3.32 mmol) were added. The reaction solution was stirred at room temperature, overnight. The residue obtained by distilling away the solvent under reduced pressure was purified by silica gel column chromatography [elution solvent: hexane/ethyl acetate = 3/1-1/1 (V/V)] to obtain the title compound (425 mg, 73%, two steps) as a solid substance.

### (Example 93-2)

The title compound was obtained in the same manner as in Example 23-2 except that the compound obtained in Example 93-1 was used.

### Example 94

### 1-(Dimethylamino)-3-methyl-1-oxobutan-2-yl (2S)-2-amino-3-(3-{3-phenyl-3-[(quinoxalin-6-yl)oxy]azetidin-1-sulfonyl}phenyl)propanoate monohydrochloride

The title compound was synthesized in the same manner as in Example 69 except that a compound, which was obtained in the same manner as in Reference Example 3 except that quinoxalin-6-ol was used, was employed.

### Example 95

### (2S)-1-(Dimethylamino)-3-methyl-1-oxobutan-2-yl (2S)-2-amino-3-(3-{3-phenyl-3-[(²H₅)phenyloxy]azetidin-1-sulfonyl}phenyl)propanoate monohydrochloride

The title compound was synthesized in the same manner as in Example 31 except that the compound obtained in Reference Example 15 and (2S)-2-hydroxy-N,N,3-trimethylbutanamide were used.

### Example 96

### (2R)-1-(Dimethylamino)-3-methyl-1-oxobutan-2-yl (2S)-2-amino-3-(3-{3-phenyl-3-[(2H5)phenyloxy]azetidin-1-sulfonyl}phenyl)propanoate monohydrochloride

The title compound was synthesized in the same manner as in Example 31 except that the compound obtained in Reference Example 15 and (2R)-2-hydroxy-N,N,3-trimethylbutanamide were used.

### Example 97

### 1-(Dimethylamino)-3-methyl-1-oxobutan-2-yl (2S)-2-amino-3-{3-[3-(5-fluoropyridin-2-yl)-3-(4-methylphenoxy)azetidin-1-sulfonyl]phenyl]propanoate monohydrochloride

The title compound was synthesized in the same manner as in Example 69 except that the compound obtained in Reference Example 13 was used.

### Example 98

### (2S)-1-(Dimethylamino)-3-methyl-1-oxobutan-2-yl (2S)-2-[(tert-butoxycarbonyl)amino]-3-(3-{[3-(4-fluorophenoxy)-3-(5-fluoropyridin-2-yl)azetidin-1-yl]sulfonyl}phenyl)propanoate

The title compound was synthesized in the same manner as in Example 31 except that the compound obtained in Reference Example 17 and (2S)-2-hydroxy-N,N,3-trimethylbutanamide were used.

### Example 99

### (2R)-1-(Dimethylamino)-3-methyl-1-oxobutan-2-yl (2S)-2-[(tert-butoxycarbonyl)amino]-3-(3-{[3-(4-fluorophenoxy)-3-(5-fluoropyridin-2-yl)azetidin-1-yl]sulfonyl}phenyl)propanoate

The title compound was synthesized in the same manner as in Example 31 except that the compound obtained in Reference Example 17 and (2R)-2-hydroxy-N,N,3-trimethylbutanamide were used.

The chemical structures of the compounds produced in the Reference Examples and data obtained by devices are as shown in Table 1 (Table 1-1 to Table 1-7). The chemical structures of the compounds produced in the Examples and data obtained by devices are as shown in Table 2 (Table 2-1 to Table 2-27). The compounds listed in Table 3 can be produced in the same manner as in the Examples.

**[Table 1-1]**

| Reference Example No. | Structure | Data |
|---|---|---|
| 1 a | 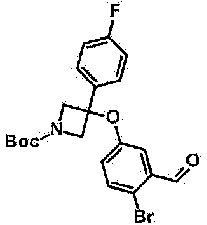 | ¹H-NMR (CDCl₃) δ: 10.21 (1H, s), 7.46 (1H, d, *J* = 9.2 Hz), 7.43-7.38 (2H, m), 7.10-7.03 (2H, m), 7.01 (1H, d, *J* = 3.1 Hz), 6.77-6.73 (1H, m), 4.35 (2H, d, *J* = 9.8 Hz), 4.30 (2H, d, *J* = 9.2 Hz), 1.47 (9H, s). |
| 1 b | 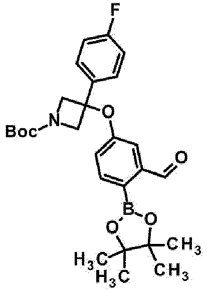 | ¹H-NMR (CDCl₃) δ: 10.55 (1H, s),7.78 (1H, d, *J* = 8.6Hz), 7.43-7.37 (2H, m), 7.10 (1H, d,*J* = 2.5 Hz), 7.07-7.00 (2H, m), 6.82-6.79 (1H, m), 4.37 (2H, d, *J* = 9.2 Hz), 4.31 (2H, d, *J* = 9.2 Hz), 1.46 (9H, s), 1.33 (12H, s). |
| 1 c | 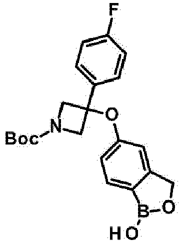 | ¹H-NMR (CDCl₃) δ: 7.55 (1H, d, *J* = 8.6 Hz), 7.46-7.40 (2H, m), 7.09-7.02 (2H, m), 6.62-6.57 (1H, m), 6.44 (1H, d, *J* = 1.8 Hz), 4.94 (2H, s), 4.38 (2H, d, *J* = 9.8 Hz), 4.27 (2H, d, *J* = 9.8 Hz), 1.47 (9H, s). |
| 2 | 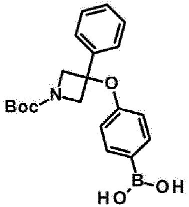 | No data |

**[Table 1-2]**

| | | |
|---|---|---|
| 3 a | 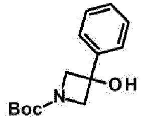 | ¹H-NMR (CDCl₃) δ: 1.47 (9H, s), 2.38 (1H, s), 4.17 (2H, d, J = 9.3 Hz), 4.29 (2H, d, J = 9.3 Hz), 7.30-7.36 (1H, m), 7.38-7.44 (2H, m), 7.48-7.52 (2H, m). MS (m/z): 272 (M+Na)⁺. |
| 3 b | 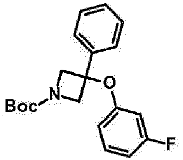 | ¹H-NMR (CDCl₃) δ: 1.47 (9H, s), 4.29 (2H, d, J = 10.0 Hz), 4.36 (2H, d, J = 9.8 Hz), 6.29-6.34 (2H, m), 6.58-6.64 (1H, m), 7.06-7.13 (1H, m), 7.28-7.33 (1H, m), 7.34-7.40 (2H, m), 7.42-7.47 (2H, m). MS (m/z): 366 (M+Na)⁺. |
| 3 c | 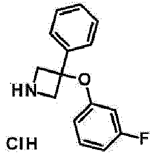 | ¹H-NMR (DMSO-d₆) δ: 4.40 (2H, d, J = 12.5 Hz), 4.61 (2H, d, J = 12.5 Hz), 6.44-6.48 (1H, m), 6.50-6.54 (1H, m), 6.75-6.81 (1H, m), 7.20-7.27 (1H, m), 7.35-7.40 (1H, m), 7.42-7.48 (2H, m), 7.56-7.61 (2H, m), 9.79 (1H, br s). MS (m/z): 244 (M+H)⁺. |
| 4 a | 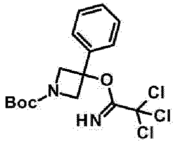 | ¹H-NMR (CDCl₃) δ: 8.39 (s, 1H), 7.51 (d, J = 7.6 Hz, 2H), 7.41 (t, J = 7.6 Hz, 2H), 7.35 (d, J = 7.2 Hz, 1H), 4.50 - 4.44 (m, 2H), 4.42 - 4.36 (m, 2H), 1.49 (s, 9H). |
| 4 b | 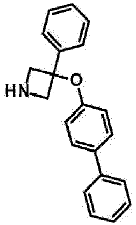 | ¹H-NMR (CDCl₃) δ: 7.62 (d, J = 8.0 Hz, 2H), 7.48 (d, J = 8.0 Hz, 2H), 7.41-7.36 (m, 7H), 7.32 (d, J = 7.2 Hz, 1H), 6.63 (d, J = 8.4 Hz, 2H), 4.29 (d, J = 9.2 Hz, 2H), 4.15-4.06 (m, 2H), 3.91-3.75 (m, 1H). |
| 5 a | 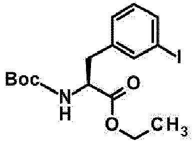 | ¹H-NMR (CDCl₃) δ: 1.25 (3H, t, J = 7.2 Hz), 1.44 (9H, s), 3.07 (1H, dd, J = 13.7, 6.1 Hz), 4.13-4.22 (2H, m), 4.48-4.57 (1H, m), 5.01 (1H, d, J = 6.3 Hz), 7.03 (1H, t, J = 7.8 Hz), 7.12 (1H, br d, J = 7.8 Hz), 7.50 (1H, br s), 7.55-7.60 (1H, m). MS (m/z): 364 (M-55)⁺. |

**[Table 1-3]**

| | | |
|---|---|---|
| 5 b | 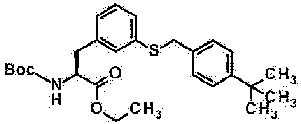 | ¹H-NMR (CDCl₃) δ: 1.23 (3H, t, J = 7.2 Hz), 1.32 (9H, s), 1.44 (9H, s), 2.95-3.11 (2H, m), 4.10 (2H, s), 4.15 (2H, q, J = 7.2 Hz), 4.50-4.57 (1H, m), 4.95-5.02 (1H, m), 6.94-7.00 (1H, m), 7.10 (1H, s), 7.17-7.25 (4H, m), 7.31-7.36 (2H, m). MS (m/z): 372 (M-BOC+H)⁺. |
| 5 c | 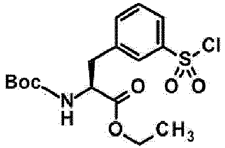 | ¹H-NMR (CDCl₃) δ: 1.27 (3H, t, J = 7.2 Hz), 1.43 (9H, s), 3.16 (1H, dd, J = 13.7,5.9 Hz), 3.30 (1H, dd, J = 13.8,5.8 Hz), 4.20 (2H, q, J = 7.2 Hz), 4.56-4.65 (1H, m), 5.06-5.14 (1H, m), 7.53-7.57 (2H, m), 7.80 (1H, br s), 7.90-7.96 (1H, m). MS (m/z): 336 (M-55)⁺. |
| 6 a | 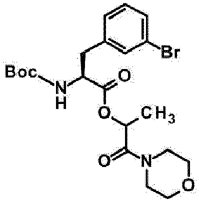 | ¹H-NMR (CDCl₃) δ: 1.33-1.50 (12H, m), 2.86-3.31 (2H, m), 3.37-3.61 (3H, m), 3.63-3.80 (5H, m), 4.50-4.62 (1H, m), 4.85-5.10 (1H, m), 5.33-5.43 (1H, m), 7.10-7.21 (2H, m), 7.30-7.43 (2H, m). MS (m/z): 487.2 [M+H]⁺. |
| 6 b | 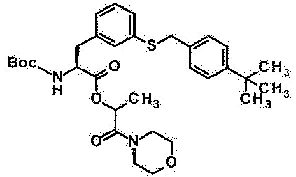 | ¹H-NMR (CDCl₃) δ: 1.31 (9H, s), 1.36-1.49 (12H, m), 2.96-3.30 (2H, m), 3.35-3.59 (3H, m), 3.62-3.77 (5H, m), 4.09-4.13 (2H, m), 4.52-4.62 (1H, m), 4.80-5.01 (1H, m), 5.34-5.44 (1H, m), 6.97-7.07 (1H, m), 7.10-7.26 (5H, m), 7.30-7.36 (2H, m). MS (m/z): 585.4 [M+H]⁺. |
| 6 | 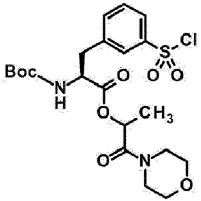 | ¹H-NMR (CDCl₃) δ: 1.39-1.48 (12H, m), 3.20-3.60 (5H, m), 3.65-3.80 (5H, m), 4.63-4.72 (1H, m), 4.95-5.21 (1H, m), 5.32-5.41 (1H, m), 7.52-7.71 (2H, m), 7.81-8.00 (2H, m). MS (m/z): 505.1 [M+H]⁺. |

**[Table 1-4]**

| | | |
|---|---|---|
| 7 a | 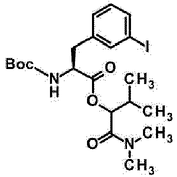 | ¹H-NMR (CDCl₃) δ: 7.58-7.47 (2H, m), 7.21-7.11 (1H, m), 7.04-6.94 (1H, m), 5.07-4.54 (2H, m), 3.24-2.91 (8H, m), 2.24-2.11 (1H, m), 1.39 (9H, s), 1.01-0.90 (6H, m). MS (m/z): 519 [M+H]⁺. |
| 7 b | 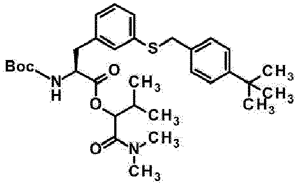 | ¹H-NMR (CDCl₃) δ: 7.32-6.94 (8H, m), 5.07-4.53 (2H, m), 4.12-4.05 (2H, m), 3.25-2.89 (8H, m), 2.22-2.11 (1H, m), 2.22-2.11 (1H, m), 1.38 (9H, s), 1.27 (9H, s), 1.00 0.90 (6H, m). MS (m/z): 571 [M+H]⁺. |
| 7 | 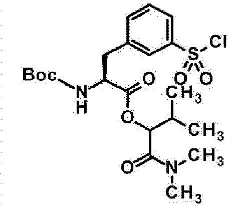 | ¹H-NMR (CDCl₃) δ: 7.97-7.17 (4H, m), 5.17-4.63 (2H, m), 3.50-2.94 (8H, m), 2.29-2.10 (1H, m), 1.41 (9H, s), 1.08-0.92 (6H, m). MS (m/z): 491 [M+H]⁺. |
| 8 | 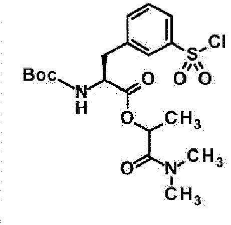 | ¹H-NMR (CDCl₃) δ: 1.37-1.49 (12H, m), 2.95-3.08 (6H, m), 3.17-3.50 (2H, m), 4.62-4.72 (1H, m), 5.02-5.27 (1H, m), 5.34-5.44 (1H, m), 7.20-7.25 (0.5H, m), 7.34-7.39 (0.5H, m), 7.51-7.63 (1H, m), 7.82-7.97 (2H, m). MS (m/z): 463.2 [M+H]⁺. |
| 9 | 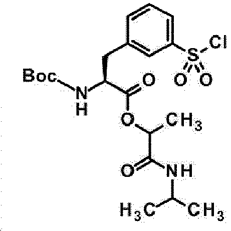 | ¹H-NMR (CDCl³) δ: 1.14-1.20 (6H, m), 1.30-1.48 (12H, m), 3.15-3.43 (2H, m), 4.01-4.10 (1H, m), 4.35-4.60 (1H, m), 4.89-5.20 (2H, m), 6.25-6.53 (1H, m), 7.56-7.65 (2H, m), 7.86-7.91 (1H, m), 7.94-8.00 (1H, m). |

**[Table 1-5]**

| | | |
|---|---|---|
| 10a | 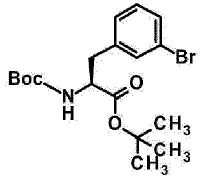 | ¹H-NMR (CDCl₃) δ: 1.41 (9H, s), 1.43 (9H, s), 2.96-3.09 (2H, m), 4.38-4.47 (1H, m), 5.03 (1H, d, J = 6.6 Hz), 7.09-7.13 (1H, m), 7.16 (1H, t, J = 7.8 Hz), 7.31-7.34 (1H, m), 7.34-7.39 (1H, m). MS (m/z): 422 (M+Na)⁺. |
| 10 | 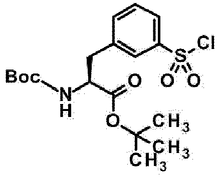 | ¹H-NMR (CDCl₃) δ: 1.43 (9H, s), 1.43 (9H, s), 3.15 (1H, dd, J = 13.8,5.3 Hz), 3.26 (1H, dd, J = 13.9,6.1 Hz), 4.44-4.54 (1H, m), 5.05-5.16 (1H, m), 7.51-7.60 (2H, m), 7.82-7.85 (1H, m), 7.90-7.95 (1H, m). MS (m/z): 442 (M+Na)⁺. |
| 11 | 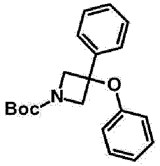 | ¹H-NMR (CDCl₃) δ: 1.47 (9H, s), 4.28 (2H, d, J = 8.5 Hz), 4.37 (2H, d, J = 10.0 Hz), 6.56 (2H, dt, J = 7.4, 1.3 Hz), 6.88-6.92 (1H, m), 7.15-7.19 (2H, m), 7.30 (1H, dt, J = 7.4, 1.8 Hz), 7.34-7.38 (2H, m), 7.44-7.49 (2H, m). MS (m/:z) : 226 (M-99)⁺. |
| 12a | 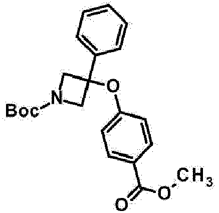 | MS (m/z): 284 (M-BOC+H)⁺. |
| 12b | 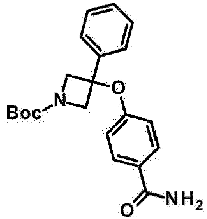 | ¹H-NMR (CDCl₃) δ: 7.62 (2H, dd, J= 6.8,2.0 Hz), 7.43-7.39 (2H, m), 7.37-7.32 (2H, m), 7.30-7.26 (1H, m), 6.58 (2H, dd, *J* = 6.8, 2.0 Hz), 4.35 (2H, d, *J* = 9.3 Hz), 4.30 (2H, d, *J* = 9.8 Hz), 1.44 (9H, s). |

**[Table 1-6]**

| | | |
|---|---|---|
| 13a | 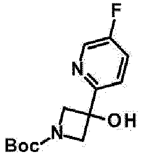 | ¹H-NMR (CDCl₃) δ: 8.37 (1H, d, *J* = 3.1 Hz), 7.72-7.67 (1H, m), 7.59-7.53 (1H, m), 5.49 (1H, s), 4.30 (2H, d, *J* = 9.8 Hz), 4.09 (2H, d, J = 9.2 Hz), 1.48 (9H, s). |
| 13 | 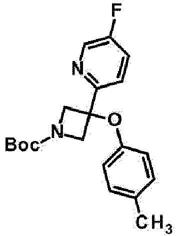 | ¹H-NMR (CDCl₃) δ: 8.53 (1H, d, *J* = 2.5 Hz), 7.37-7.25 (2H, m), 6.97 (2H, d, *J* = 8.0 Hz), 6.43-6.38 (2H, m), 4.54 (2H, br), 4.29 (2H, d, *J* = 8.6 Hz), 2.23 (3H, s), 1.45 (9H, s). |
| 14 | 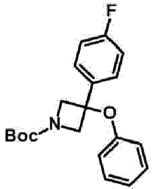 | ¹H-NMR (CDCl₃) δ: 7.47-7.41 (2H, m), 7.21-7.15 (2H, m), 7.08-7.02 (2H, m), 6.94-6.89 (1H, m), 6.56-6.52 (2H, m), 4.37 (2H, d, *J* = 9.8 Hz), 4.25 (2H, d, *J* = 9.8 Hz), 1.47 (9H, s). |
| 15 | 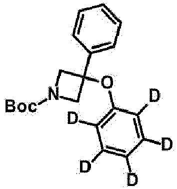 | ¹H-NMR (CDCl₃) δ: 7.45-7.43 (1H, m), 7.35-7.32 (1H, m), 7.29-7.25 (1H, m), 4.34 (6H, d, *J* = 9.8 Hz), 4.26 (6H, d, *J* = 9.3 Hz), 1.44 (9H, s). |
| 16 | 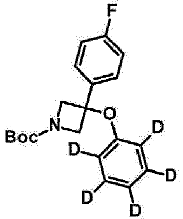 | ¹H-NMR (CDCl₃) δ: 7.45-7.38 (2H, m), 7.06-6.98 (2H, m), 4.34 (2H, d, *J* = 9.8 Hz), 4.22 (2H, d, J = 9.3 Hz), 1.44 (9H, s). |

**[Table 1-7]**

| | | |
|---|---|---|
| 17 | 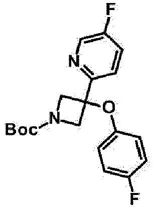 | ¹H-NMR (CDCl₃) δ: 8.55-8.51 (1H, m), 7.36-7.31 (2H, m), 6.90-6.82 (2H, m), 6.50-6.43 (2H, m), 4.56 (2H, br), 4.28 (2H, d, *J* = 9.2 Hz), 1.46 (9H, s). |

**[Table 2-1]**

| Example No. | Structure | Data |
|---|---|---|
| 1-1 | 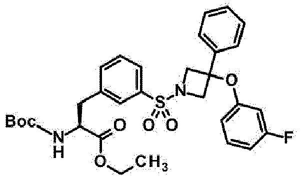 | ¹H-NMR (CDCl₃) δ: 1.26 (3H, t, J = 7.2 Hz), 1.42 (9H, s), 3.14 (1H, dd, J = 13.7, 5.4 Hz), 3.26 (1H, dd, J = 13.6, 5.5 Hz), 4.13-4.25 (6H, m), 4.54-4.62 (1H, m), 5.02 (1H, d, J = 6.5 Hz), 6.12-6.17 (2H, m), 6.56-6.62 (1H, m), 7.01-7.08 (1H, m), 7.26-7.36 (5H, m), 7.46-7.54 (2H, m), 7.64 (1H, br s), 7.73-7.77 (1H, m). MS (m/z): 621 (M+Na)⁺. |
| 1-2 | 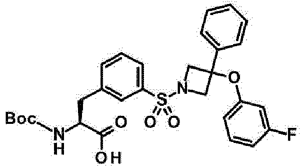 | ¹H-NMR (CDCl₃) δ: 1.41 (9H, s), 3.17 (1H, dd, J = 13.7, 5.4 Hz), 3.31 (1H, dd, J = 13.6, 5.3 Hz), 4.15 (1H, d, J = 9.0 Hz), 4.16 (1H, d, J = 9.3 Hz), 4.23 (1H, d, J = 9.0 Hz), 4.23 (1H, d, J = 9.3 Hz), 4.56-4.66 (1H, m), 5.00 (1H, d, J = 7.0 Hz), 6.10-6.15 (2H, m), 6.56-6.62 (1H, m), 7.00-7.07 (1H, m), 7.27-7.35 (5H, m), 7.51-7.55 (2H, m), 7.70-7.77 (2H, m). MS (m/z): 593 (M+Na)⁺. |
| 1 | 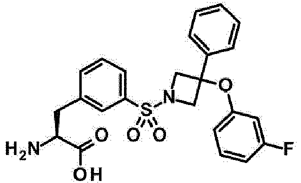 | ¹H-NMR (DMSO-d₆) δ: 2.86 (1H, dd, J = 13.6,7.3 Hz), 3.16 (1H, dd, J = 13.7,4.9 Hz), 4.09 (1H, d, J = 9.5 Hz), 4.11 (1H, d, J = 9.8 Hz), 4.33 (1H, d, J = 9.5 Hz), 4.34 (1H, d, J = 9.8 Hz), 6.24-6.34 (2H, m), 6.67-6.73 (1H, m), 7.11-7.19 (1H, m), 7.20-7.35 (5H, m), 7.55 (1H, t, J = 7.7 Hz), 7.64-7.70 (2H, m), 7.77 (1H, br s), signal of one proton was overlapped with H₂O signal. MS (m/z) : 471 (M+H)⁺. |
| 2 | 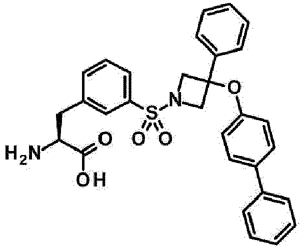 | ¹H-NMR (DMSO-D₆) δ: 7.82 (s, 1H), 7.79 - 7.69 (m, 2H), 7.64 (d, J = 7.6 Hz, 1H), 7.52 (d, J = 7.2 Hz, 2H), 7.44 - 7.26 (m, 10H), 6.53 (d, J = 8.8 Hz, 2H), 4.35 (dd, J = 6.4, 9.2 Hz, 2H), 4.13 (dd, J = 7.2, 9.2 Hz, 2H), 4.01 - 3.95 (m, 1H), 3.24 (d, J = 6.0 Hz, 1H), 3.18 - 3.14 (m, 1H) MS (m/z): 529 (M+H)⁺. |

**[Table 2-2]**

| | | |
|---|---|---|
| 3 | 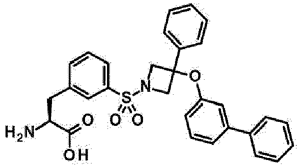 | ¹H-NMR (DMSO-D₆) δ: 7.87 - 7.71 (m, 2H), 7.70 - 7.60 (m, 2H), 7.51 - 7.38 (m, 4H), 7.34 - 7.29 (m, 5H), 7.23 - 7.10 (m, 3H), 6.70 (s, 1H), 6.39 (d, J = 8.0 Hz, 1H), 4.42 - 4.31 (m, 2H), 4.19 - 4.09 (m, 3H), 3.20 - 3.13 (m, 2H). MS (m/z): 529 (M+H)⁺. |
| 4 | 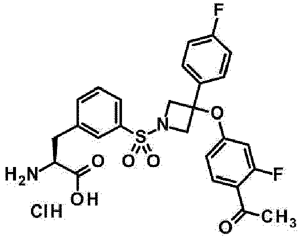 | ¹H-NMR: (DMSO-d₆) δ 2.45 (3H, d, J = 4.4 Hz), 3.20-3.24 (2H, m), 4.09-4.17 (2H, m), 4.21-4.28 (1H, m), 4.38-4.45 (2H, m), 6.36-6.43 (2H, m), 7.15-7.22 (2H, m), 7.27-7.35 (2H, m), 7.60-7.67 (2H, m), 7.70-7.74 (1H, m), 7.75-7.82 (2H, m), 8.25-8.55 (3H, m). MS (m/z): 531.1 [M+H]⁺ |
| 5 | 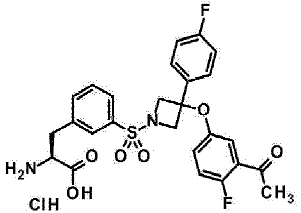 | ¹H-NMR (DMSO-d₆) δ: 2.48 (3H, d, J = 4.4 Hz), 3.13-3.20 (2H, m), 4.01-4.06 (1H, m), 4.10 (2H, t, J = 9.2 Hz), 4.34 (2H, t, J = 8.8 Hz), 6.64-6.71 (1H, m), 6.75-6.79 (1H, m), 7.10-7.20 (3H, m), 7.31-7.37 (2H, m), 7.58-7.64 (1H, m), 7.66-7.71 (1H, m), 7.73-7.80 (2H, m). MS (m/z): 531.1 [M+H]⁺ |
| 6-1 | 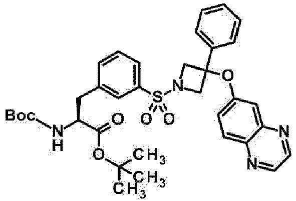 | ¹H-NMR (CDCl₃): 1.36 (s, 9H), 1.37 (s, 9H), 3.04 - 3.08 (m, 1H), 3.15 - 3.20 (m, 1H), 4.22 - 4.32 (m, 4H), 4.39 -4.41 (m, 1H), 5.01 - 5.03(m, 1H), 6.55 (d, J = 2.8 Hz, 1H), 7.19 - 7.30 (m, 6H), 7.43 - 7.47 (m, 2H), 7.63 (s, 1H),7.68 - 7.70 (m, 1H), 7.91 (d, J=9.2 Hz, 1H), 8.59 - 8.60 (m, 1H). |
| 6 | 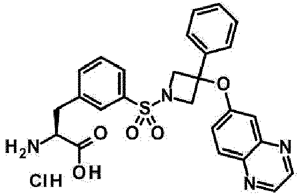 | ¹H-NMR (DMSO-d₆) δ: 3.18-3.21 (m, 2H), 4.21-4.26 (m, 3H),449 (t, J=9.2 Hz, 2H), 6.63 (d, J=2.8 Hz, 1H), 7.31-7.36 (m, 6H), 7.62-7.66 (m, 1H),7.71-7.73 (m, 1H), 7.80-7.84 (m, 2H), 7.98 (d, J=9.2 Hz, 1H), 8.38-8.40 (m, 3H), 8.76-8.77 (m, 2H). MS (m/z): 505 (M+H)⁺. |

**[Table 2-3]**

| | | |
|---|---|---|
| 7 | 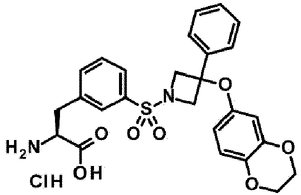 | ¹H-NMR (DMSO-d₆) δ: 3.19-3.25 (m, 2H), 4.03 (t, J=8.8 Hz, 2H), 4.09-4.13 (m, 4H), 4.23-4.26 (m, 3H), 5.88-5.90 (m, 2H), 6.59 (d, J=9.2 Hz, 1H), 7.26-7.34 (m, 5H), 7.67-7.79 (m, 4H), 8.30-8.36 (m, 3H). MS (m/z): 511 (M+H)⁺. |
| 8 | 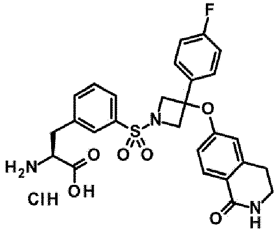 | ¹H-NMR (DMSO-d₆) δ: 2.74 (2H, t, J = 6.4 Hz), 3.15-3.25 (4H, m), 4.10 (2H, t, J = 10.0 Hz), 4.23 (1H, t, J = 6.4 Hz), 4.32-4.41 (2H, m), 6.29-6.34 (1H, m), 6.38-6.41 (1H, m), 7.14-7.21 (2H, m), 7.29-7.35 (2H, m), 7.58 (1H, d, J = 8.8 Hz), 7.61-7.67 (1H, m), 7.69-7.81 (4H, m), 8.15-8.55 (3H, m). MS (m/z) : 540.2 [M+H]⁺. |
| 9 | 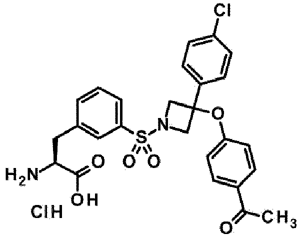 | ¹H-NMR (DMSO-d₆) δ: 2.45 (3H, s), 3.19-3.25 (2H, m), 4.08-4.22 (3H, m), 4.38 (2H, t, J = 9.2 Hz), 6.54 (2H, d, J = 8.8 Hz), 7.27 (2H, d, J = 8.8 Hz), 7.40 (2H, d, J = 8.8 Hz), 7.59-7.67 (1H, m), 7.70-7.82 (5H, m), 7.97-8.98 (2H, m). MS (m/z) : 529.1 [M+H]⁺; |
| 10 | 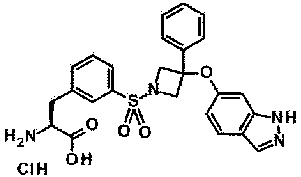 | ¹H-NMR (DMSO-d₆) δ: 3.22 (d, J = 6.8 Hz, 2H), 4.14 (t, J = 9.6 Hz, 2H), 4.22-4.26 (m, 1H), 4.34 (t, J = 9.6 Hz, 2H), 6.19 (s, 1H), 6.45 (dd, J = 2.0, 8.8 Hz, 1H), 7.28-7.37 (m, 5H), 7.56 (d, J = 8.8 Hz, 1H), 7.64 (t, J = 7.6 Hz, 1H), 7.73 (d, J = 7.6 Hz, 1H), 7.78-7.81 (m, 2H), 7.89 (d, J = 0.8 Hz, 1H), 8.47-8.48 (m, 3H). MS (m/z): 493 (M+H)⁺. |
| 11 | 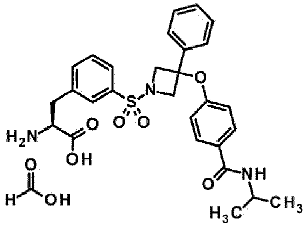 | ¹H-NMR (CD₃OD) δ: 7.85 (1H, s), 7.80 (1H, d, J = 7.8 Hz), 7.69 (1H, d, J = 7.8 Hz), 7.61 (1H, t, J = 7.8 Hz), 7.56 (2H, d, J = 8.8 Hz), 7.32-7.22 (5H, m), 6.46 (2H, d, J = 8.8 Hz), 4.32 (2H, d, J = 9.8 Hz), 4.17 (2H, d, J = 9.3 Hz), 4.15-4.06 (1H, m), 3.83-3.75 (1H, m), 3.44-3.05 (2H, m), 1.17 (6H, d, J = 6.8 Hz). MS (m/z): 538 (M+H)⁺. |

**[Table 2-4]**

| | | |
|---|---|---|
| 12 | 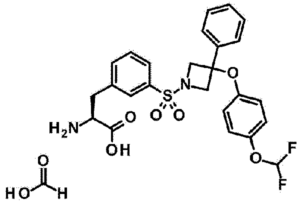 | ¹H-NMR (DMSO-d₆) δ: 7.75 (1H, s), 7.67 (2H, t, J = 6.8 Hz), 7.60-7.53 (1H, m), 7.34-7.21 (5H, m), 7.02 (1H, t, J = 74.4 Hz), 6.92 (2H, d, J = 9.3 Hz), 6.45 (2H, d, J = 9.3 Hz), 4.33-4.26 (2H, m), 4.09-4.03 (2H, m), 3.44-3.13 (2H, m), 2.97-2.88 (1H, m). MS (m/z): 519 (M+H)⁺. |
| 13 | 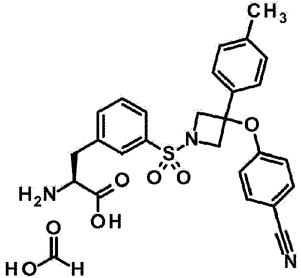 | ¹H-NMR (CD₃OD) δ: 7.84 (1H, s), 7.79 (1H, d, J = 7.8 Hz), 7.68 (1H, d, J = 7.3 Hz), 7.61 (1H, t, J = 7.6 Hz), 7.47-7.45 (2H, m), 7.16-7.09 (4H, m), 6.59-6.53 (2H, m), 4.30 (2H, d, J = 9.8 Hz), 4.15 (2H, dd, J = 9.8, 2.4 Hz), 3.87-3.82 (1H, m), 3.37-3.08 (2H, m), 2.26 (3H, s). MS (m/z): 490 (M-H)⁻. |
| 14 | 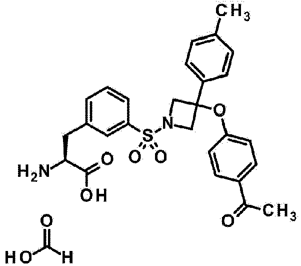 | ¹H-NMR (CD₃OD) δ: 7.84 (1H, s), 7.79 (1H, d, J = 7.8 Hz), 7.76 (2H, d, J = 9.3 Hz), 7.68 (1H, d, J = 7.3 Hz), 7.61 (1H, t, J = 7.8 Hz), 7.16 (2H, d, J = 8.3 Hz), 7.10 (2H, d, J = 8.3 Hz), 6.49 (2H, d, J = 8.8 Hz), 4.30 (2H, d, J = 9.8 Hz), 4.16 (2H, d, J = 9.8 Hz), 3.82-3.77 (1H, m), 3.43-3.05 (2H, m), 2.45 (3H, s), 2.25 (3H, s). MS (m/z): 509 (M+H)⁺. |
| 15-1 | 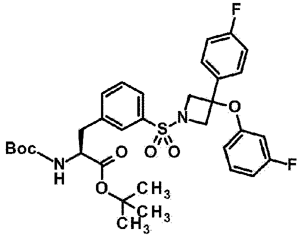 | ¹H-NMR (CDCl₃) δ: 1.43 (9H, s), 1.44 (9H, s), 3.12 (1H, dd, J = 13.7, 5.4 Hz), 3.25 (1H, dd, J = 13.4, 6.4 Hz), 4.13-4.21 (4H, m), 4.42-4.52 (1H, m), 5.00 (1H, d, J = 6.3 Hz), 6.11-6.17 (2H, m), 6.58-6.64 (1H, m), 6.99-7.09 (3H, m), 7.25-7.32 (2H, m), 7.50 (2H, d, J = 5.0 Hz), 7.67 (1H, s), 7.70-7.76 (1H, m). MS (m/z): 667 (M+Na)⁺. |

**[Table 2-5]**

| | | |
|---|---|---|
| 15 | 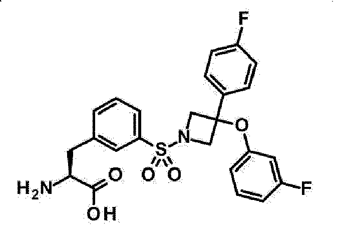 | ¹H-NMR (DMSO-d₆) δ: 2.97 (1H, dd, J = 14.2,7.4 Hz), 3.20 (1H, dd, J = 14.2, 5.1 Hz), 3.46 (1H, dd, J = 7.3, 5.3 Hz), 4.11 (1H, d, J = 9.8 Hz), 4.12 (1H, d, J = 9.5 Hz), 4.35 (1H, d, J = 9.5 Hz), 4.36 (1H, d, J = 9.7 Hz), 6.25-6.30 (1H, m), 6.32 (1H, dt, J = 10.8, 2.3 Hz), 6.69-6.76 (1H, m), 7.12-7.20 (3H, m), 7.28-7.35 (2H, m), 7.57 (1H, t, J = 7.8 Hz), 7.66-7.72 (2H, m), 7.77-7.80 (1H, m). MS (m/z) : 489 (M+H)⁺. |
| 16 | 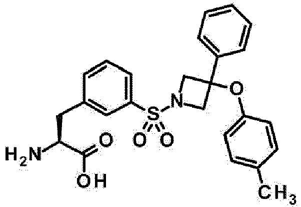 | ¹H-NMR (DMSO-d₆) δ: 2.14 (3H, s), 2.95 (1H, dd, J = 14.3, 7.5 Hz), 3.21 (1H, dd, J = 14.3, 5.0 Hz), 3.41 (1H, dd, J = 7.5, 5.0 Hz), 4.06 (1H, d, J = 9.8 Hz), 4.08 (1H, d, J = 9.8 Hz), 4.28 (2H, d, J = 9.8 Hz), 6.30-6.35 (2H, m), 6.88-6.94 (2H, m), 7.22-7.34 (5H, m), 7.58 (1H, t, J = 7.7 Hz), 7.66-7.71 (2H, m), 7.76-7.79 (1H, m). MS (m/z) : 467 (M+H)⁺. |
| 17 | 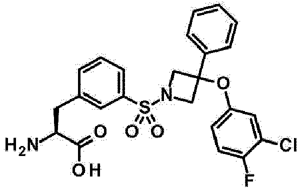 | ¹H-NMR (DMSO-d₆) δ: 2.96 (1H, dd, J = 14.2, 7.4 Hz), 3.21 (1H, dd, J = 13.9, 5.1 Hz), 3.44 (1H, dd, J = 7.2, 5.1 Hz), 4.10 (1H, d, J = 9.5 Hz), 4.11 (1H, d, J = 9.3 Hz), 4.34 (2H, d, J = 10.5 Hz), 6.41 (1H, dt, J = 8.9, 3.4 Hz), 6.66 (1H, dd, J = 6.3, 3.0 Hz), 7.17 (1H, t, J = 9.2 Hz), 7.23-7.39 (5H, m), 7.58 (1H, t, J = 7.7 Hz), 7.69 (2H, t, J = 6.8 Hz), 7.78 (1H, br s). MS (m/z) : 528 (M+Na)⁺. |
| 18 | 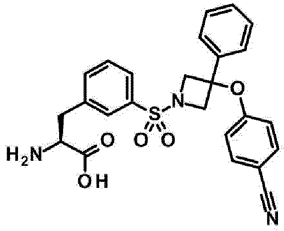 | ¹H-NMR (DMSO-d₆) δ: 2.89-3.00 (1H, m), 3.16-3.23 (1H, m), 3.38-3.45 (1H, m), 4.13 (1H, d, J = 9.8 Hz), 4.15 (1H, d, J = 9.8 Hz), 4.36 (1H, d, J = 9.8 Hz), 4.38 (1H, d, J = 9.8 Hz), 6.59-6.65 (2H, m), 7.21-7.36 (5H, m), 7.54-7.65 (3H, m), 7.66-7.72 (2H, m), 7.79 (1H, s). MS (m/z) : 478 (M+H)⁺. |

**[Table 2-6]**

| | | |
|---|---|---|
| 19-1 | 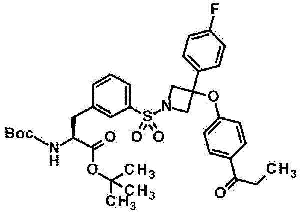 | MS (m/z): 583 (M-BOC+H)⁺. |
| 19 | 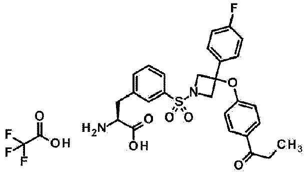 | ¹H-NMR (DMSO-d₆) δ: 1.00 (3H, t, J - 7.2 Hz), 2.85-2.92 (2H, m), 3.13-3.23 (2H, m), 4.07-4.15 (3H, m), 4.34-4.41 (2H, m), 6.54 (2H, d, J - 8.8 Hz), 7.16 (2H, t, J = 8.8 Hz), 7.26-7.34 (2H, m), 7.59 - 7.81 (6H, m). MS (m/z) : 527.2 [M+H]⁺. |
| 20 | 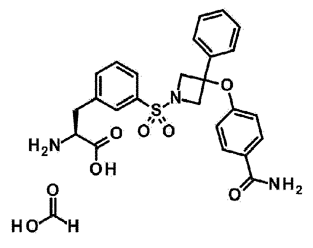 | ¹H-NMR (CD₃OD) δ: 7.84 (1H, s), 7.80 (1H, d, J = 7.3 Hz), 7.69 (1H, d, J = 7.8 Hz), 7.66-7.57 (3H, m), 7.35-7.22 (5H, m), 6.46 (2H, d, J = 8.3 Hz), 4.32 (2H, d, J - 9.3 Hz), 4.16 (2H, d, J - 9.3 Hz), 3.79 (1H, dd, J = 7.8, 4.9 Hz), 3.38-3.02 (2H, m). MS (m/z): 496 (M+H)⁺. |
| 21 | 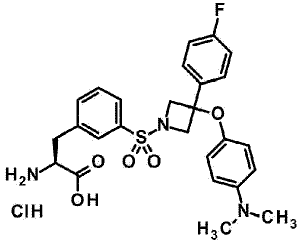 | ¹H-NMR (CD₃OD) δ: 3.13 (6H, s), 3.23-3.30 (1H, m), 3.34-3.42 (1H, m), 4.19 (2H, dd, J = 9.2, 3.6 Hz), 4.27-4.36 (3H, m), 6.58-6.63 (2H, m), 7.02-7.11 (2H, m), 7.24-7.40 (4H, m), 7.64-7.75 (2H, m), 7.81-7.89 (2H, m). MS (m/z) : 514.1 [M+H]⁺. |

**[Table 2-7]**

| | | |
|---|---|---|
| 22 | 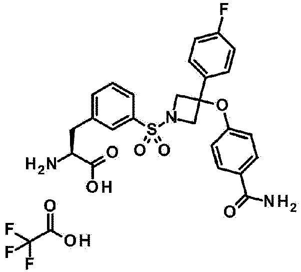 | ¹H-NMR (CD₃OD) δ: 7.86-7.80 (2H, m), 7.70-7.61 (4H, m), 7.39-7.32 (2H, m), 7.05 (2H, t, J = 8.5 Hz), 6.45 (2H, d, J - 8.8 Hz), 4.36-4.11 (5H, m), 3.35-3.12 (2H, m). MS (m/z): 514 (M+H)⁺. |
| 23-1 | 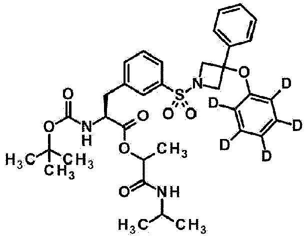 | ¹H-NMR (CDCl₃) δ: 7.79-7.73 (1H, m), 7.67-7.64 (1H, m), 7.56-7.45 (2H, m), 7.36-7.25 (5H, m), 6.57-6.42 (NH, 1H, m). 5.16-5.08 (1H, m), 4.84-4.71 (1H, m), 4.29-3.99 (5H, m), 3.32-3.06 (2H, m). 1.45-1.19 (12H, m), 1.17-1.09 (6H, m). MS (m/z): 571 (M+H-BOC)⁺. |
| 23 | 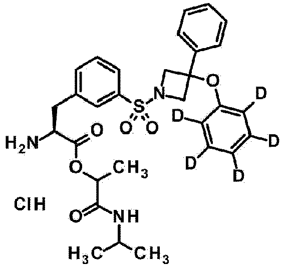 | ¹H-NMR (DMSO-d₆) δ: 8.62-8.42 (NH, 2H, m), 8.01-7.57 (4H, m), 7.35-7.24 (5H, m), 4.91 (0.5H, q, J = 7.0 Hz), 4.86 (0.5H, q, J = 6.8 Hz), 4.48-4.26 (3H, m), 4.09-4.01 (2H, m), 3.86-3.76 (1H, m), 3.31-3.10 (2H, m), 1.28-0.99 (9H, m). MS (m/z): 571 (M+H)⁺. |
| 24 | 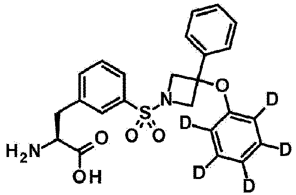 | ¹H-NMR (DMSO-d₆) δ: 7.76 (1H, s), 7.67 (2H, t, J = 6.6 Hz), 7.56 (1H, t, J = 7.8 Hz), 7.33-7.21 (5H, m), 4.29 (2H, d, J = 9.3 Hz), 4.07 (2H, dd, J = 9.5, 4.1 Hz), 3.42 (1H, t, J=6.3 Hz), 3.20-3.14 (1H, m), 2.97-2.89 (1H, m). MS (m/z): 458 (M+H)⁺. |

**[Table 2-8]**

| | | |
|---|---|---|
| 25 | 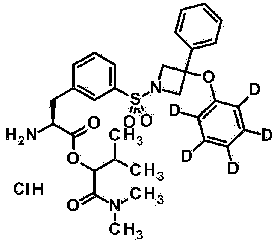 | ¹H-NMR (CD₃OD) δ: 7.87-7.63 (4H, m), 7.36-7.23 (5H, m). 5.27 (0.5H, d, J - 5.9 Hz), 5.18 (0.5H, d, J - 5.9 Hz), 4.49-4.42 (1H, m), 4.30-4.26 (2H, m), 4.16-4.09 (2H, m), 3.45-3.20 (2H, m), 3.13-3.09 (3H, m), 2.96-2.92 (3H, m), 2.19-2.05 (1H, m), 0.99-0.84 (6H, m). MS (m/z): 585 (M+H)⁺. |
| 26-1 | 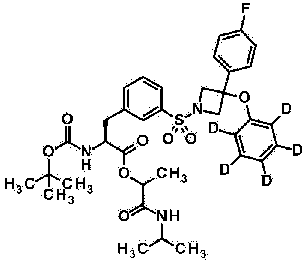 | ¹H-NMR (CDCl₃) δ: 7.78-7.72 (1H, m), 7.67-7.63 (1H, m), 7.55-7.46 (2H, m), 7.36-7.28 (2H, m), 7.03-6.96 (2H, m), 6.54-6.34 (NH, 1H, m), 5.18-4.43 (2H, m), 4.28-4-07 (5H, m), 3.33-3.06 (2H, m), 1.57-1.29 (12H, m), 1.18-1.09 (6H, m). |
| 26 | 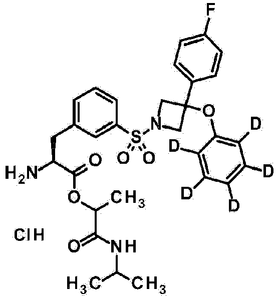 | ¹H-NMR (CD₃OD) δ: 7.90-7.61 (4H, m), 7.39-7.34 (2H, m), 7.04 (2H, t, J = 8.8 Hz), 5.04 (0.5H, q, J = 7.0 Hz), 4.99 (0.5H, q, J = 7.0 Hz), 4.41 (0.5H, t, J = 6.8 Hz), 4.37 (0.5H, t, J = 6.8 Hz), 4.31-4.23 (2H, m), 4.15-4.09 (2H, m), 3.98-3.88 (1H, m), 3.47-3.13 (2H, m), 1.41 (1.5H, d, J = 6.8 Hz), 1.34 (1.5H, d, J = 6.8 Hz), 1.15-1.07 (6H, m). MS (m/z): 589 (M+H)⁺. |
| 27 | 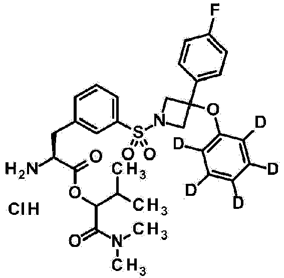 | ¹H-NMR (CD₃OD) δ: 7.87-7.62 (4H, m), 7.39-7.33 (2H, m), 7.07-6.99 (2H, m), 5.27 (0.5H, d, J = 5.4 Hz), 5.18 (0.5H, d, J = 5.9 Hz), 4.47-4.42 (1H, m), 4.30-4.24 (2H, m), 4.15-4.08 (2H, m), 3.45-3.19 (2H, m), 3.13-2.91 (6H, m), 2.20-2.06 (1H, m), 0.99-0.84 (6H, m). MS (m/z): 603 (M+H)⁺. |

**[Table 2-9]**

| | | |
|---|---|---|
| 28 | 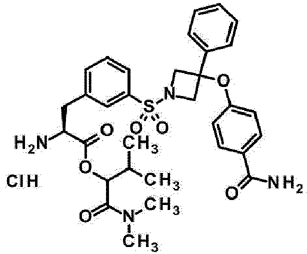 | ¹H-NMR (CD₃OD) δ: 7.89-7.60 (6H, m), 7.36-7.24 (5H, m), 6.48-6.41 (2H, m), 5.28-5.15 (1H, m), 4.50-4.41 (1H, m), 4.37-4.31 (2H, m), 4.19-4.12 (2H, m), 3.47-3.19 (2H, m), 3.13-2.91 (6H, m), 2.18-2.04 (1H, m), 0.98-0.82 (6H, m). MS (m/z): 623 (M+H)⁺. |
| 29 | 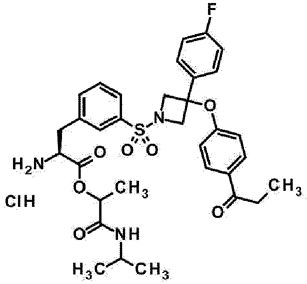 | ¹H-NMR (DMSO-d₆) δ 0.98-1.08 (9H, m), 1.18-1.30 (3H, m), 2.84-2.94 (2H, m), 3.13-3.32 (2H, m), 3.78-3.87 (1H, m), 4.07-4.17 (2H, m), 4.34-4.52 (3H, m), 4.81-5.01 (1H, m), 6.54 (2H, d, J = 8.4 Hz), 7.18 (2H, t, J = 8.8 Hz), 7.30-7.38 (2H, m), 7.60-7.68 (1H, m), 7.69-7.97 (6H, m), 8.31-8.67 (3H, m). MS (m/z) : 640.3 [M+H]⁺. |
| 30 | 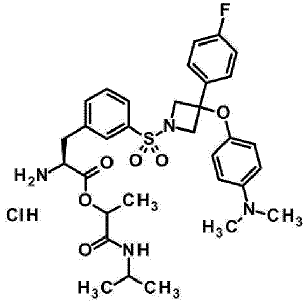 | ¹H-NMR (DMSO-d₆) δ 1.04-1.09 (6H, m), 1.17-1.29 (3H, m), 2.97 (6H, s), 3.20-3.33 (2H, m), 3.77-3.87 (1H, m), 4.03-4.12 (2H, m), 4.30-4.49 (3H, m), 4.84-4.98 (1H, m), 6.50-6.59 (2H, m), 7.14-7.21 (2H, m), 7.22-7.60 (4H, m), 7.61-7.68 (1H, m), 7.73-7.89 (3H, m), 7.95-8.10 (1H, m), 8.63-8.85 (3H, m). MS (m/z) : 627.3 [M+H]⁺. |
| 31-1 | 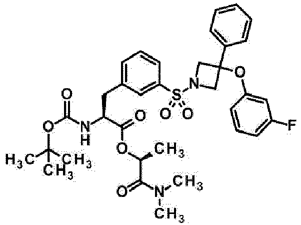 | ¹H NMR (CDCl₃) δ: 7.84-7.47 (m, 4H), 7.33-7.26 (m, 5H), 7.09-7.01 (m, 1H), 6.64-6.55 (m, 1H), 6.15 (d, J = 8.0 Hz, 2H), 5.46-5.33 (m, 1H), 4.88 (d, J = 8.4 Hz, 1H), 4.64 (d, J = 6.8 Hz, 1H), 4.26-4.11 (m, 4H), 3.48-3.13 (m, 2H), 3.12-2.69 (m, 6H), 2.01 (s, 3H), 1.55-1.27 (m, 12H). MS (m/z) : 670 [M+H]⁺. |

**[Table 2-10]**

| | | |
|---|---|---|
| 31 | 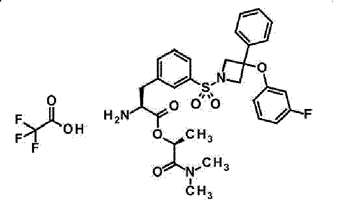 | ¹H-NMR (DMSO-d₆) δ = 8.70-8.22 (m, 3H), 7.95-7.70 (m, 3H), 7.70-7.60 (m, 1H), 7.37-7.25 (m, 5H), 7.16 (q, J = 8.0 Hz, 1H), 6.71 (m, 1H), 6.31-6.22 (m, 2H), 5.46 (d, J = 6.8 Hz, 1H), 4.45-4.31 (m, 3H), 4.10 (t, J = 10.0 Hz, 2H), 3.33 (dd, J = 7.6, 14.4 Hz, 1H), 3.20-3.07 (m, 1H), 3.05-2.95 (m, 3H), 2.91-2.77 (m, 3H), 1.27 (d, J = 6.8 Hz, 3H). MS (m/z) : 570 [M+H]⁺. |
| 32 | 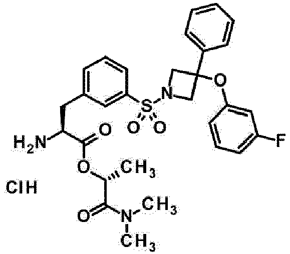 | ¹H-NMR: (DMSO-d₆) δ 1.21 (3H, d, J = 6.8 Hz), 2.82 (3H, s), 2.98 (3H, s), 3.21-3.24 (1H, m), 3.26-3.32 (1H, m), 4.08-4.12 (2H, m), 4.34-4.40 (3H, m), 5.37-542 (1 H, m), 6.27-6.31 (2H, m), 6.71-6.73 (1H, m), 7.13-7.16 (1H, m), 7.29-7.32 (5H, m), 7.33-7.35 (1H, m), 7.66-7.83 (3H, m), 8.76 (s, 3H). MS (m/z) : 570 [M+H]⁺. |
| 33 | 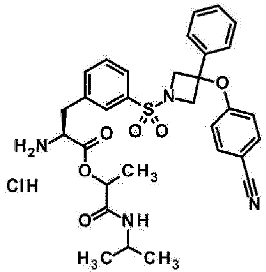 | ¹H-NMR (DMSO-d₆) δ: 8.71-8.47 (3H, m), 8.08-7.60 (7H, m), 7.38-7.22 (5H, m), 6.60 (2H, d, *J* = 33.7 Hz), 4.98-4.84 (1H, m), 4.53-4.33 (3H, m), 4.16-4.07 (2H, m), 3.90-3.77 (1H, m), 3.36-3.12 (2H, m), 1.32-1.18 (3H, m), 1.10-1.03 (6H, m). MS (m/z): 591 (M+H)⁺. |
| 34 | 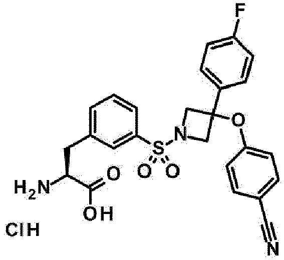 | ¹H-NMR (DMSO-d₆) δ: 13.96 (1H, br s), 8.38 (3H, m), 7.83-7.75 (2H, m), 7.74-7.68 (1H, m), 7.67-7.61 (3H, m), 7.32-7.27 (2H, m), 7.21-7.14 (2H, m), 6.63-6.58 (2H, m), 4.43-4.36 (2H, m), 4.30-4.22 (1H, m) 4.16-4.07 (2H, m), 3.27-3.15 (2H, m). MS (m/z): 496 (M+H)⁺. |

**[Table 2-11]**

| | | |
|---|---|---|
| 35 | 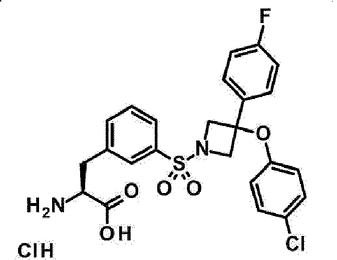 | ¹H-NMR (DMS0-d₆) δ: 13.97 (1H, br s), 8.51-8.35 (3H, m), 7.82-7.75 (2H, m), 7.75-7.69 (1H, m), 7.67-7.61 (1H, m), 7.33-7.26 (2H, m), 7.22-7.14 (4H, m), 6.49-6.43 (2H, m), 4.37-4.29 (2H, m), 4.29-4.22 (1H, m), 4.12-4.05 (2H, m), 3.29-3.18 (2H, m). MS (m/z): 505 (M+H)⁺. |
| 36 | 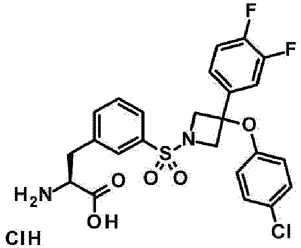 | ¹H-NMR (DMSO-d₆) δ: 13.97 (1H, br s), 8.52-8.27 (3H, m), 7.81-7.74 (2H, m), 7.73-7.68 (1H, m), 7.68-7.60 (1H, m), 7.46-7.25 (2H, m), 7.23-7.09 (3H, m), 6.50-6.43 (2H, m), 4.44-4.33 (2H, m), 4.30-4.22 (1H, m), 4.13-4.05 (2H, m), 3.28-3.15 (2H, m). MS (m/z): 523 (M+H)⁺. |
| 37 | 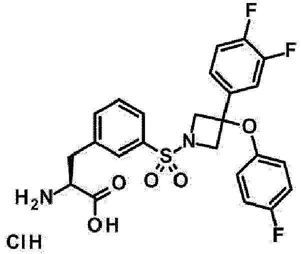 | ¹H-NMR (DMSO-d₆) δ: 13.98 (1H, br s), 8.55-8.29 (3H, m), 7.81-7.74 (2H, m), 7.74-7.68 (1H, m), 7.68-7.60 (1H, m), 7.46-7.36 (1H, m), 7.36-7.26 (1H, m), 7.20-7.10 (1H, m), 7.03-6.94 (2H, m), 6.50-6.42 (2H, m), 4.42-4.31 (2H, m), 4.30-4.22 (1H, m), 4.12-4.04 (2H, m), 3.29-3.16 (2H, m). MS (m/z): 507 (M+H)⁺. |
| 38 | 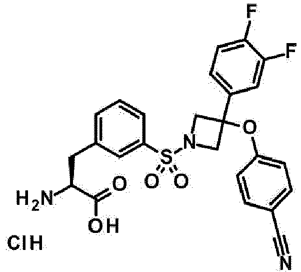 | ¹H-NMR (DMSO-d₆) δ: 14.00 (1H, br s), 8.52-8.32 (3H, m), 7.83-7.75 (2H, m), 7.74-7.69 (1H, m), 7.69-7.60 (3H, m), 7.47-7.37 (1H, m), 7.35-7.26 (1H, m), 7.18-7.08 (1H, m), 6.65-6.58 (2H, m), 4.50-4.38 (2H, m), 4.31-4.21 (1H, m), 4.17-4.08 (2H, m), 3.27-3.15 (2H, m). MS (m/z): 514 (M+H)⁺. |

**[Table 2-12]**

| | | |
|---|---|---|
| 39 | 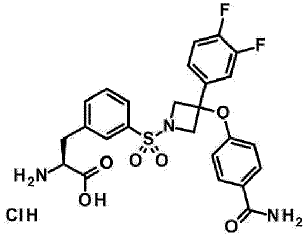 | ¹H-NMR (DMSO-d₆) δ: 14.01 (1H, br s), 8.34-8.32 (3H, m), 7.84-7.75 (3H, m), 7.73-7.61 (4H, m), 7.45-7.36 (1H, m), 7.32-7.23 (2H, m), 7.16-7.09 (1H, m), 6.50 (2H, d, *J* = 8.6 Hz), 4.46-4.38 (2H, m), 4.32-4.23 (1H, m), 4.16-4.07 (2H, m), 3.26-3.15 (2H, m). MS (m/z): 532 (M+H)⁺. |
| 4 0 | 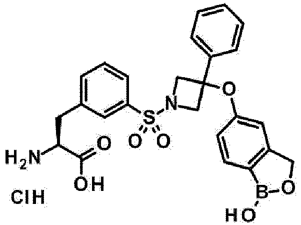 | ¹H-NMR (DMSO-d₆) δ: 13.98 (1H, br s), 9.53-8.96 (1H, m), 8.49-8.25 (3H, m), 7.84-7.20 (10H, m), 6.60-6.15 (2H, m), 4.81-4.49 (2H, m), 4.39-4.22 (3H, m), 4.14-4.04 (2H, m), 3.27-3.14 (2H, m). MS (m/z): 509 (M+H)⁺. |
| 41 | 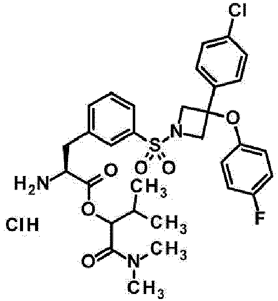 | ¹H-NMR (DMSO-d₆) δ: 8.65-8.38 (3H, m), 7.91-7.71 (3H, m), 7.70-7.59 (1H, m), 7.45-7.37 (2H, m), 7.35-7.26 (2H, m), 7.03-6.94 (2H, m), 6.47-6.39 (2H, m), 5.21-5.09 (1H, m), 4.58-4.47 (1H, m), 4.37-4.28 (2H, m), 4.11-4.04 (2H, m), 3.33-3.14 (2H, m), 3.08-3.02 (3H, m), 2.87-2.82 (3H, m), 2.09-1.97 (1H, m), 0.92-0.69 (6H, m). MS (m/z): 632 (M+H)⁺. |
| 42 | 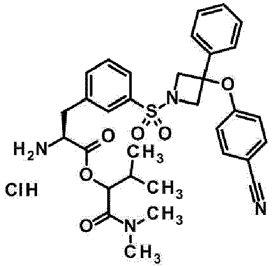 | ¹H-NMR (DMSO-d₆) δ: 8.69-8.39 (3H, m), 7.94-7.72 (3H, m), 7.70-7.59 (3H, m), 7.39-7.22 (5H, m), 6.64-6.56 (2H, m), 5.21-5.09 (1H, m), 4.57-4.46 (1H, m), 4.45-4.35 (2H, m), 4.18-4.08 (2H, m), 3.33-3.12 (2H, m), 3.09-3.01 (3H, m), 2.89-2.81 (3H, m), 2.09-1.97 (1H, m), 0.92-0.69 (6H, m). MS (m/z): 605 (M+H)⁺. |

**[Table 2-13]**

| | | |
|---|---|---|
| 43 | 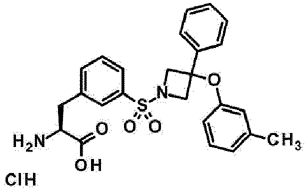 | ¹H-NMR (CD₃OD) δ: 7.86-7.84 (2H, m), 7.72-7.65 (2H, m), 7.33-7.30 (5H, m), 6.94 (1H, t, *J* = 8.0 Hz), 6.68 (1H, d, *J* = 8.0 Hz), 6.25 (1H, s), 6.11 (1H, dd, *J* = 8.6, 2.5 Hz), 4.28 (2H, dd, *J* = 9.2, 3.7 Hz), 4.23 (1H, t, J= 6.4 Hz), 4.13 (2H, dd, *J* = 9.2, 3.7 Hz), 3.35 (1H, dd, *J* = 14.1, 6.1 Hz), 3.22 (1H, dd, *J* = 14.4, 7.1 Hz), 2.17 (3H, s). MS (m/z): 467 (M+H)⁺. |
| 44 | 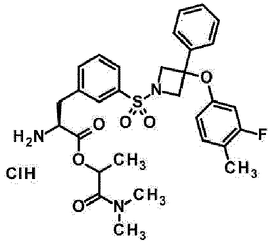 | ¹H-NMR (CD₃OD) δ: 7.92-7.65 (4H, m), 7.36-7.26 (5H, m), 6.93 (1H, t, *J* = 8.6 Hz), 6.14-6.07 (2H, m), 5.59-5.45 (1H, m), 4.45-4.39 (1H, m), 4.30 (2H, d, *J* = 9.8 Hz), 4.14 (2H, dd, *J* = 9.2, 3.7 Hz), 3.48 (1H, dd, *J* = 14.7, 6.7 Hz), 3.34-3.22 (1H, m), 3.11-3.07 (3H, m), 2.97-2.94 (3H, m), 2.10-2.07 (3H, m), 1.45-1.34 (3H, m). MS (m/z): 584 (M+H)⁺ |
| 45 | 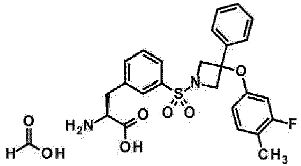 | ¹H-NMR (DMSO-d₆) δ: 7.78 (1H, s), 7.71-7.69 (2H, m), 7.58 (1H, t, *J* = 7.7 Hz), 7.32-7.26 (5H, m), 7.01 (1H, t, *J* = 8.6 Hz), 6.26 (1H, dd, J = 11.0, 2.5 Hz), 6.18 (1H, dd, *J* = 8.0, 2.5 Hz), 4.33 (2H, d, *J* = 9.8 Hz), 4.09 (2H, dd, *J* = 9.5, 4.0 Hz), 3.44-3.35 (1H, m), 3.21 (1H, dd, *J* = 14.1, 4.9 Hz), 2.96 (1H, dd, *J* = 14.4, 7.7 Hz), 2.05 (3H, s). MS (m/z): 485 (M+H)⁺. |
| 46 | 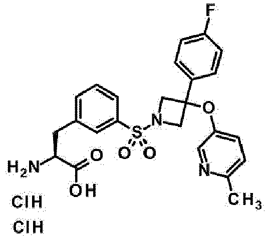 | ¹H-NMR (CD₃OD) δ: 8.07-8.04 (1H, m), 7.89-7.86 (2H, m), 7.74-7.59 (3H, m), 7.55-7.51 (1H, m), 7.40-7.34 (2H, m), 7.13-7.07 (2H, m), 4.41 (2H, dd, *J* = 9.5, 3.4 Hz), 4.36-4.31 (3H, m), 3.42-3.28 (2H, m), 2.60 (3H, s). MS (m/z): 486 (M+H)⁺. |

**[Table 2-14]**

| | | |
|---|---|---|
| 47 | 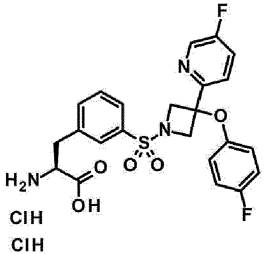 | ¹H-NMR (CD₃OD) δ: 8.37 (1H, d, *J* = 3.1 Hz), 7.87-7.81 (2H, m), 7.71-7.62 (2H, m), 7.53-7.46 (1H, m), 7.39-7.33 (1H, m), 6.90-6.83 (2H, m), 6.42-6.37 (2H, m), 4.54 (2H, dd, *J* = 9.2, 6.1 Hz), 4.28 (1H, t, *J* = 6.7 Hz), 4.15 (2H, dd, *J* = 9.2, 4.3 Hz), 3.36 (1H, dd, *J* = 14.4, 6.4 Hz), 3.25 (1H, dd, *J* = 14.4, 7.1 Hz). MS (m/z): 490 (M+H)⁺. |
| 48 | 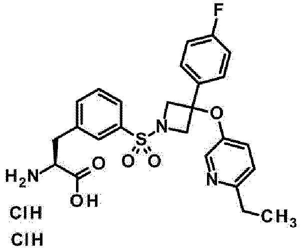 | ¹H-NMR (CD₃OD) δ: 8.07 (1H, d, *J* = 2.5 Hz), 7.90-7.86 (2H, m), 7.73-7.64 (3H, m), 7.58-7.54 (1H, m), 7.40-7.35 (2H, m), 7.13-7.08 (2H, m), 4.41 (2H, dd, *J* = 9.8, 4.3 Hz), 4.36-4.31 (3H, m), 3.39 (1H, dd, *J* = 14.1, 6.7 Hz), 3.34-3.28 (1H, m), 2.92 (2H, q, *J* = 7.6 Hz), 1.30 (3H, t, *J* = 7.7 Hz). MS (m/z): 500 (M+H)⁺ |
| 49 | 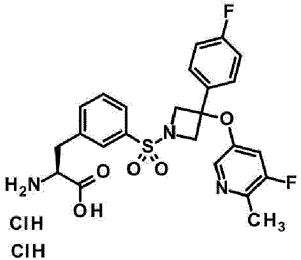 | ¹H-NMR (CD₃OD) δ: 7.89-7.85 (2H, m), 7.78-7.64 (3H, m), 7.40-7.34 (2H, m), 7.17-7.07 (3H, m), 4.39 (2H, d, *J* = 9.8 Hz), 4.36-4.31 (1H, m), 4.27 (2H, dd, *J* = 9.5, 4.6 Hz), 3.41-3.26 (2H, m), 2.44 (3H, s). MS (m/z): 504 (M+H)⁺ |
| 50 | 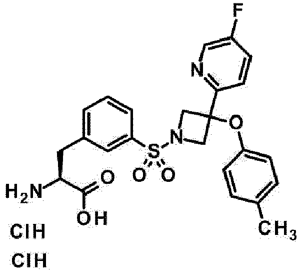 | ¹H-NMR (CD₃OD) δ: 8.37 (1H, d, *J* = 2.5 Hz), 7.86-7.81 (2H, m), 7.71-7.63 (2H, m), 7.50-7.44 (1H, m), 7.35-7.31 (1H, m), 6.92 (2H, d, *J* = 8.6 Hz), 6.28-6.23 (2H, m), 4.55-4.49 (2H, m), 4.26 (1H, t, *J* = 6.7 Hz), 4.13 (2H, dd, *J* = 8.9, 4.0 Hz), 3.35 (1H, dd, *J* = 14.7, 6.1 Hz), 3.23 (1H, dd, *J* = 14.4,7.1 Hz), 2.17 (3H, s). MS (m/z): 486 (M+H)⁺ |

**[Table 2-15]**

| | | |
|---|---|---|
| 51 | 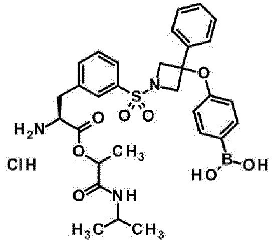 | ¹H-NMR (CD₃OD) δ: 7.92-7.64 (4H, m), 7.44-7.26 (7H, m), 6.43-6.32 (2H, m), 5.11-4.96 (1H, m), 4.46-4.37 (1H, m), 4.32 (2H, d, *J* = 8.6 Hz), 4.19-4.13 (2H, m), 4.01-3.92 (1H, m), 3.52-3.20 (2H, m), 1.46-1.34 (3H, m), 1.18-1.11 (6H, m). MS (m/z): 586 (M+H)' |
| 52 | 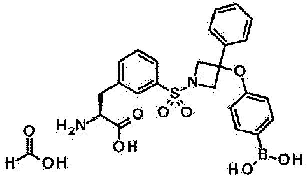 | ¹H-NMR (CD₃OD) δ: 7.87-7.80 (2H, m), 7.74-7.62 (2H, m), 7.54-7.25 (7H, m), 6.43-6.32 (2H, m), 4.30 (2H, d, *J* = 9.2 Hz), 4.15 (2H, d, *J* = 9.2 Hz), 4.04-3.99 (1H, m), 3.36 (1H, dd, *J* = 14.4, 5.2 Hz), 3.18 (1H, dd, *J* = 14.7, 7.4 Hz). MS (m/z): 497 (M+H)⁺ |
| 53 | 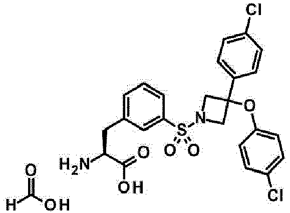 | ¹H-NMR (DMSO-d₆) δ: 7.80 (1H, s), 7.70 (2H, t, *J* = 7.7 Hz), 7.57 (1H, t, *J* = 7.7 Hz), 7.39 (2H, d, *J* = 8.6 Hz), 7.26 (2H, d, *J* = 8.6 Hz), 7.26 (2H, d, *J* = 8.6 Hz), 7.19 (2H, d, *J* = 9.2 Hz), 6.48 (2H, d, *J* = 8.6 Hz), 4.35-4.28 (2H, m), 4.10 (2H, dd, *J* = 9.8, 3.1 Hz), 3.49 (1H, t, *J* = 6.1 Hz), 3.22 (1H, dd, *J* = 14.1, 5.5 Hz), 3.01 (1H, dd, *J* = 14.1, 7.4 Hz). MS (m/z): 521 (M+H)⁺ |
| 54 | 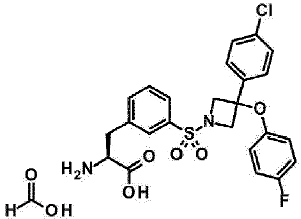 | ¹H-NMR (CD₃OD) δ: 7.87-7.85 (1H, m), 7.82-7.79 (1H, m), 7.72-7.69 (1H, m), 7.63 (1H, t, *J* = 7.7 Hz), 7.34-7.32 (4H, m), 6.88-6.83 (2H, m), 6.43-6.39 (2H, m), 4.27 (2H, d, *J* = 9.8 Hz), 4.17-4.13 (2H, m), 3.83 (1H, dd, *J* = 8.0, 4.9 Hz), 3.37 (1H, dd, *J* = 14.1, 4.9 Hz), 3.13 (1H, dd, *J* = 14.7, 8.0 Hz). MS (m/z): 505 (M+H)⁺ |
| 55 | 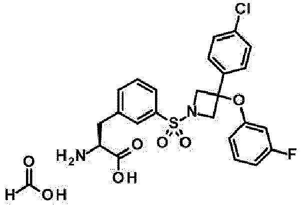 | ¹H-NMR (CD₃OD) δ: 7.87 (1H, br s), 7.82 (1H, d, *J* = 8.0 Hz), 7.71 (1H, d, *J* = 8.0 Hz), 7.64 (1H, t, *J* = 8.0 Hz), 7.37-7.31 (4H, m), 7.14-7.07 (1H, m), 6.66-6.60 (1H, m), 6.25-6.18 (2H, m), 4.31 (2H, d, *J* = 9.8 Hz), 4.19-4.14 (2H, m), 3.82 (1H, dd, *J* = 8.0, 4.9 Hz), 3.36 (1H, dd, *J* = 14.7, 4.9 Hz), 3.12 (1H, dd, *J* = 14.7, 8.0 Hz). MS (m/z): 505 (M+H)⁺ |

**[Table 2-16]**

| | | |
|---|---|---|
| 56 | 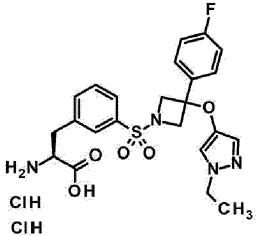 | ¹H-NMR (CD₃OD) δ: 7.87-7.83 (2H, m), 7.69-7.66 (2H, m), 7.38-7.33 (2H, m), 7.11-7.05 (2H, m), 6.98 (1H, s), 6.80 (1H, s), 4.33 (1H, t, *J* = 6.7 Hz), 4.21-4.13 (4H, m), 3.95 (2H, q, *J* = 7.2 Hz), 3.39 (1H, dd, *J* = 14.4, 6.4 Hz), 3.32-3.26 (1H, m), 1.29 (3H, t, *J* = 7.4 Hz). MS (m/z): 489 (M+H)⁺ |
| 57 | 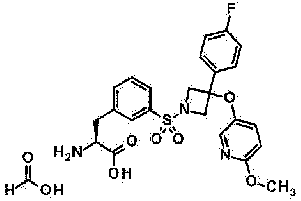 | ¹H-NMR (DMSO-d₆) δ: 7.77 (1H, br s), 7.70-7.65 (2H, m), 7.56 (2H, t, *J* = 7.7 Hz), 7.37-7.29 (3H, m), 7.19-7.13 (2H, m), 6.98-6.93 (1H, m), 6.62 (1H, d, *J* = 9.2 Hz), 4.32 (2H, dd, *J* = 9.2, 4.3 Hz), 4.12 (2H, dd, *J* = 9.8, 4.9 Hz), 3.70 (3H, s), 3.53-3.47 (1H, m), 3.21 (1H, dd, *J* = 14.1, 5.5 Hz), 2.98 (1H, dd, *J* = 14.1, 7.4 Hz). MS (m/z): 502 (M+H)⁺ |
| 58 | 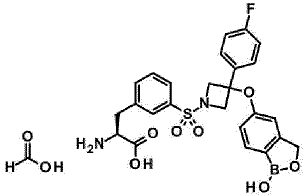 | ¹H-NMR (CD₃OD) δ: 7.87 (1H, br s), 7.83-7.79 (1H, m), 7.72-7.69 (1H, m), 7.63 (1H, t, *J* = 7.7 Hz), 7.44-7.35 (3H, m), 7.09-7.02 (2H, m), 6.48-6.44 (1H, m), 6.39-6.37 (1H, m), 4.86 (2H, s), 4.32 (2H, d, *J* = 9.8 Hz), 4.17 (2H, d, *J* = 9.8 Hz), 3.82 (1H, dd, *J* = 8.0, 4.9 Hz), 3.35 (1H, dd, *J* = 14.4,4.6 Hz), 3.11 (1H, dd, *J* = 14.4,8.3 Hz). MS (m/z): 527 (M+H)⁺ |
| 59 | 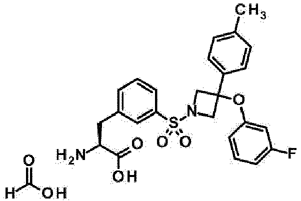 | ¹H-NMR (DMSO-d₆) δ: 7.78 (1H, br s), 7.73-7.67 (2H, m), 7.58 (1H, t, *J* = 7.7 Hz), 7.19-7.09 (6H, m), 6.74-6.67 (1H, m), 6.32-6.24 (2H, m), 4.31 (2H, dd, *J* = 9.5, 2.1 Hz), 4.09 (2H, dd, *J* = 9.8, 4.3 Hz), 3.48-3.17 (2H, m), 2.95 (1H, dd, *J* = 14.1, 7.4 Hz), 2.24 (3H, s). MS (m/z): 483 (M-H)⁺ |

**[Table 2-17]**

| | | |
|---|---|---|
| 60 | 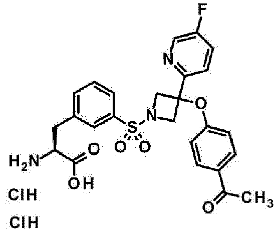 | ¹H-NMR (CD₃OD) δ: 8.40 (1H, d, *J* = 3.1 Hz), 7.88-7.79 (4H, m), 7.71-7.63 (2H, m), 7.52-7.46 (1H, m), 7.36-7.31 (1H, m), 6.53-6.48 (2H, m), 4.61 (2H, dd, *J* = 8.9, 7.7 Hz), 4.26 (1H, t, *J* = 6.7 Hz), 4.20 (2H, dd, *J* = 9.5, 5.2 Hz), 3.37-3.29 (1H, m), 3.22 (1H, dd, *J* = 14.4, 7.1 Hz), 2.48 (3H, s). MS (m/z): 514 (M+H)⁺ |
| 61 | 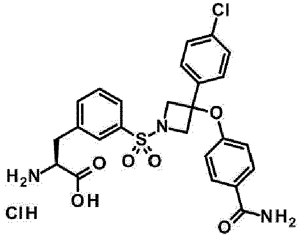 | ¹H-NMR (DMSO-d₆) δ: 14.04-13.92 (1H, br), 8.41-8.32 (2H, m), 7.83-7.75 (3H, m), 7.73-7.62 (4H, m), 7.43-7.37 (2H, m), 7.29-7.19 (3H, m), 6.48 (2H, d, *J* = 8.6 Hz), 4.37 (2H, t, *J* = 9.2 Hz), 4.31-4.24 (1H, m), 4.10 (2H, t, *J* = 9.5 Hz), 3.28-3.16 (2H, m). |
| 62 | 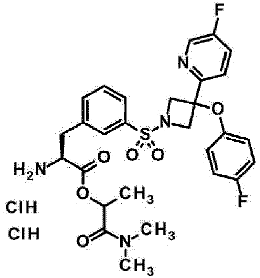 | ¹H-NMR (CD₃OD) δ: 8.37 (1H, t, *J* = 2.5 Hz), 7.91-7.63 (4H, m), 7.52-7.46 (1H, m), 7.38-7.34 (1H, m), 6.89-6.84 (2H, m), 6.42-6.36 (2H, m), 5.60-5.46 (1H, m), 4.57-4.52 (2H, m), 4.45-4.38 (1H, m), 4.18-4.13 (2H, m), 3.52-3.22 (2H, m), 3.12-3.09 (3H, m), 2.98-2.94 (3H, m), 1.46-1.35 (3H, m). MS (m/z): 589 (M+H)⁺ |
| 63 | 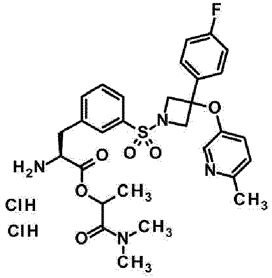 | ¹H-NMR (CD₃OD) δ: 8.09-8.07 (1H, m), 7.97-7.57 (6H, m), 7.39-7.32 (2H, m), 7.13-7.07 (2H, m), 5.61-5.47 (1H, m), 4.48-4.39 (3H, m), 4.36 (2H, d, *J* = 9.2 Hz), 3.54-3.25 (2H, m), 3.13-3.09 (3H, m), 2.98-2.94 (3H, m), 1.47-1.35 (3H, m). MS (m/z): 585 (M+H)⁺ |

**[Table 2-18]**

| | | |
|---|---|---|
| 64 | 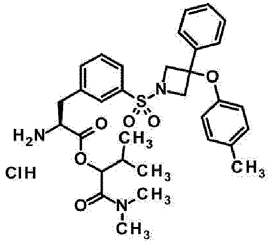 | ¹H-NMR (CD₃OD) δ: 7.89-7.64 (4H, m), 7.37-7.25 (5H, m), 6.90 (2H, d, *J* = 8.6 Hz), 6.29-6.24 (2H, m), 5.31-5.20 (1H, m), 4.53-4.46 (1H, m), 4.27 (2H, d, *J* = 9.2 Hz), 4.16-4.10 (2H, m), 3.49-3.24 (2H, m), 3.15-3.11 (3H, m), 2.99-2.95 (3H, m), 2.22-2.07 (4H, m), 1.01-0.88 (6H, m). MS (m/z): 594 (M+H)⁺ |
| 65 | 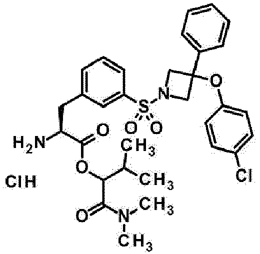 | ¹H-NMR (CD₃OD) δ: 7.90-7.60 (4H, m), 7.37-7.27 (5H, m), 7.12-7.07 (2H, m), 6.42-6.36 (2H, m), 5.31-5.20 (1H, m), 4.53-4.46 (1H, m), 4.31 (2H, d, *J* = 9.2 Hz), 4.18-4.12 (2H, m), 3.49-3.24 (2H, m), 3.16-3.11 (3H, m), 2.99-2.95 (3H, m), 2.22-2.09 (1H, m), 1.01-0.87 (6H, m). MS (m/z): 615 (M+H)⁺ |
| 66 | 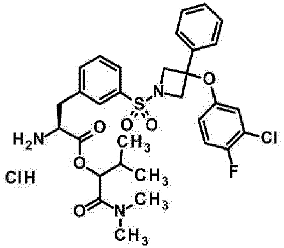 | ¹H-NMR (CD₃OD) δ: 7.90-7.65 (4H, m), 7.38-7.29 (5H, m), 7.01-6.95 (1H, m), 6.53-6.49 (1H, m), 6.34-6.28 (1H, m), 5.32-5.20 (1H, m), 4.53-4.45 (1H, m), 4.33 (2H, d, *J* = 9.2 Hz), 4.19-4.12 (2H, m), 3.49-3.25 (2H, m), 3.16-3.12 (3H, m), 2.99-2.95 (3H, m), 2.23-2.08 (1H, m), 1.02-0.89 (6H, m). MS (m/z): 633 (M+H)⁺ |
| 67 | 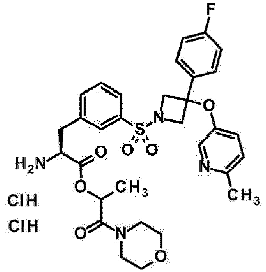 | ¹H-NMR (CD₃OD) δ: 8.04-7.56 (7H, m), 7.42-7.36 (2H, m), 7.10 (2H, t, *J* = 8.6 Hz), 5.61-5.48 (1H, m), 4.47-4.35 (5H, m), 3.75-3.29 (10H, m), 2.62 (3H, s), 1.47-1.36 (3H, m). MS (m/z): 627 (M+H)⁺ |

**[Table 2-19]**

| | | |
|---|---|---|
| 68 | 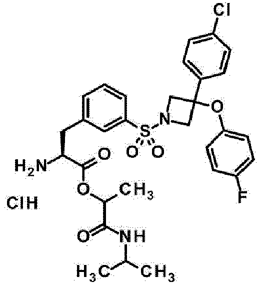 | ¹H-NMR (CD₃OD) δ: 7.92-7.65 (4H, m), 7.36-7.33 (4H, m), 6.89-6.83 (2H, m), 6.42-6.37 (2H, m), 5.10-4.98 (1H, m), 4.48-4.40 (1H, m), 4.29 (2H, d, *J* = 9.8 Hz), 4.16-4.11 (2H, m), 4.01-3.91 (1H, m), 3.53-3.21 (2H, m), 1.46-1.34 (3H, m), 1.17-1.11 (6H, m). MS (m/z): 619 (M+H)⁺ |
| 69 - 1 | 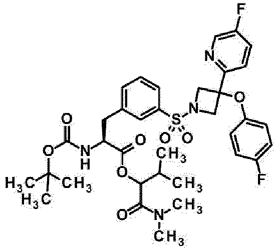 | ¹H-NMR (CDCl₃) δ: 8.32-8.27 (1H, m), 7.75-7.21 (6H, m), 6.84-6.78 (2H, m), 6.36-6.29 (2H, m), 5.12-4.39 (5H, m), 4.21-4.15 (2H, m), 3.43-2.95 (8H, m), 2.27-2.16 (1H, m), 1.43-1.35 (9H, m), 1.09-0.98 (6H, m). |
| 69 | 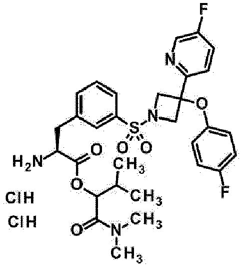 | ¹H-NMR (CD₃OD) δ: 8.38 (1H, t, *J* = 2.8 Hz), 7.88-7.63 (4H, m), 7.53-7.47 (1H, m), 7.39-7.34 (1H, m), 6.90-6.83 (2H, m), 6.42-6.36 (2H, m), 5.32-5.20 (1H, m), 4.57-4.46 (3H, m), 4.19-4.13 (2H, m), 3.50-3.23 (2H, m), 3.16-3.13 (3H, m), 3.00-2.95 (3H, m), 2.23-2.08 (1H, m), 1.02-0.89 (6H, m). MS (m/z): 617 (M+H)⁺ |
| 70 | 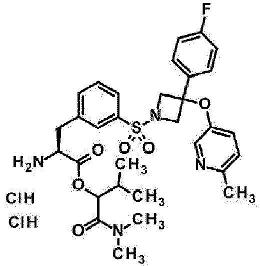 | ¹H-NMR (CD₃OD) δ: 8.11-8.07 (1H, m), 7.94-7.58 (6H, m), 7.40-7.33 (2H, m), 7.10 (2H, t, *J* = 8.6 Hz), 5.33-5.21 (1H, m), 4.56-4.47 (1H, m), 4.45-4.34 (4H, m), 3.51-3.36 (2H, m), 3.17-3.13 (3H, m), 2.99-2.95 (3H, m), 2.62 (3H, s), 2.23-2.08 (1H, m), 1.02-0.88 (6H, m). MS (m/z): 613 (M+H)⁺ |

**[Table 2-20]**

| | | |
|---|---|---|
| 71 | 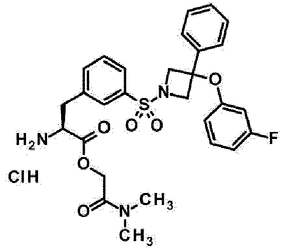 | ¹H-NMR (CDCl₃) δ: 1.36 (9H, d, J = 1.2 Hz), 2.93-2.98 (6H, m), 3.07-3.50 (3H, m), 4.11-4.22 (4H, m), 4.62-4.67 (1H, m), 4.87 (1H, d, J = 14.4 Hz), 5.18 (1H, s), 6.03-6.18 (2H, m), 6.55 (1H, t, J = 8.4 Hz), 7.01 (1H, m), 7.19-7.35 (5H, m), 7.45-7.54 (1H, m), 7.57-7.81 (3H, m). |
| 72 | 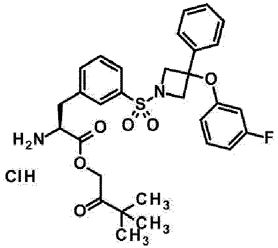 | ¹H-NMR (DMSO-d₆) δ: 1.13 (9H, s), 3.24-3.35 (2H, m), 4.09 (2H, t, J = 8.8 Hz), 4.32-4.39 (2H, m), 4.49 (1H, t, J = 6.0 Hz), 5.01-5.14 (1H, m), 5.15-5.29 (1H, m), 6.23-6.30 (2H, m), 6.68-6.75 (1H, m), 7.13-7.19 (1H, m), 7.25-7.36 (5H, m), 7.62-7.68 (1H, m), 7.76-7.82 (2H, m), 7.86 (1H, s), 8.65 (3H, s). MS (m/z): 569 [M+H]⁺. |
| 73 | 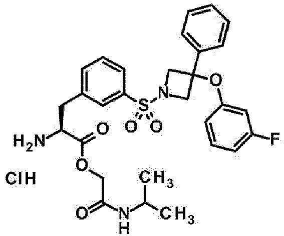 | ¹H-NMR: (DMSO-d₆) δ: 1.03-1.10 (6H, m), 3.20-3.28 (2H, m), 3.82-3.92 (1H, m), 4.05-4.12 (2H, m), 4.32-4.39 (2H, m), 4.43-4.59 (3H, m), 6.23-6.31 (2H, m), 6.68-6.75 (1H, m), 7.12-7.20 (1H, m), 7.25-7.37 (5H, m), 7.61-7.67 (1H, m), 7.75-7.81 (2H, m), 7.84-7.87 (1H, m), 8.02 (1H, d, J = 7.6 Hz), 8.52-8.64 (3H, br s). MS (m/z): 570.2 [M+H]⁺. |
| 74 | 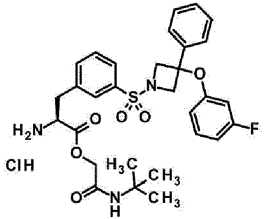 | ¹H-NMR: (DMSO-d₆) δ: 1.26 (9H, s), 3.20-3.26 (1H, m), 3.34-3.38 (1H, m), 4.05-4.11 (2H, m), 4.32-4.38 (2H, m), 4.41-4.58 (3H, m), 6.23-6.31 (2H, m), 6.67-6.75 (1H, m), 7.12-7.20 (1H, m), 7.25-7.38 (5H, m), 7.60-7.67 (1H, m), 7.73 (1H, s), 7.75-7.82 (2H, m), 7.87 (1H, s), 8.45-8.63 (3H, brs). MS (m/z): 584.2 [M+H]⁺. |

**[Table 2-21]**

| | | |
|---|---|---|
| 75 | 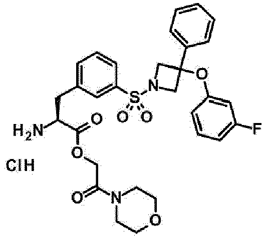 | ¹H-NMR (DMSO-d₆) δ: 3.20-3.36 (4H, m), 3.42 (3H, s), 3.55 (3H, s), 4.04-4.14 (2H, m), 4.25-4.49 (3H, m), 4.73-5.10 (2H, m), 6.21-6.31 (2H, m), 6.71 (1H, t, J = 7.6 Hz), 7.15 (1H, d, J = 7.2 Hz), 7.23-7.35 (5H, m), 7.59-7.90 (4H, m), 8.71 (3H, s). MS (m/z): 598 [M+H]⁺. |
| 76 | 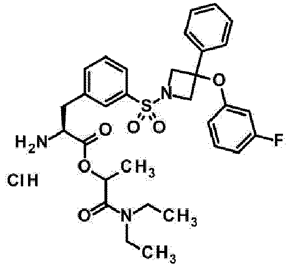 | ¹H-NMR (DMSO-d₆) δ: 0.96-1.04 (3H, m), 1.11-1.19 (3H, m), 1.20-1.29 (3H, m), 3.05-3.38 (6H, m), 4.05-4.16 (2H, m), 4.29-4.41 (3H, m), 5.22-5.36 (1H, m), 6.22-6.33 (2H, m), 6.66-6.77 (1H, m), 7.12-7.20 (1H, m), 7.23-7.37 (5H, m), 7.59-7.68 (1H, m), 7.73-8.03 (3H, m), 8.60-8.85 (3H, m). MS (m/z): 598.2 [M+H]⁺. |
| 77 | 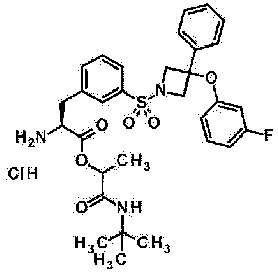 | ¹H NMR (DMSO-d₆) δ: 1.18-1.30 (12H, m), 3.17-3.31 (2H, m), 4.03-4.18 (2H, m), 4.30-4.47 (3H, m), 4.83-5.00 (1H, m), 6.20-6.35 (2H, m), 6.66-6.75 (1H, m), 7.12-7.20 (1H, m), 7.26-7.38 (5H, m), 7.55-7.96 (5H, m), 8.63 (3H, d, *J* = 2.4 Hz). MS (m/z): 598.2 [M+H]⁺. |
| 7 8 | 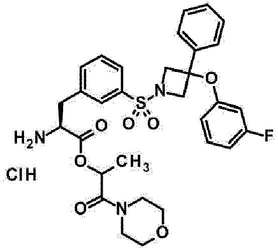 | ¹H-NMR (DMSO-d₆) δ: 1.19-1.29 (3H, m), 3.18-3.31 (2H, m), 3.42-3.59 (8H, m), 4.07-4.13 (2H, m), 4.32-4.42 (3H, m), 5.39-5.52 (1H, m), 6.25-6.31 (2H, m), 6.68-6.75 (1H, m), 7.13-7.19 (1H, m), 7.27-7.36 (5H, m), 7.61-7.68 (1H, m), 7.74-7.95 (3H, m), 8.57-8.75 (3H, m). MS (m/z): 612.2 [M+H]⁺. |

**[Table 2-22]**

| | | |
|---|---|---|
| 79 | 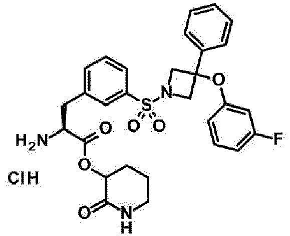 | ¹H-NMR (DMSO-d₆) δ: 1.61-1.83 (3H, m), 1.91-2.07 (1H, m), 3.03-3.16 (2H, m), 3.19-3.36 (2H, m), 4.03-4.15 (2H, m), 4.28-4.44 (3H, m), 5.00-5.26 (1H, m), 6.20-6.33 (2H, m), 6.65-6.77 (1H, m), 7.11-7.20 (1H, m), 7.23-7.37 (5H, m), 7.57-7.68 (1H, m), 7.71-7.93 (4H, m), 8.52-8.90 (3 H, m). MS (m/z): 568.2 [M+H]⁺. |
| 80 | 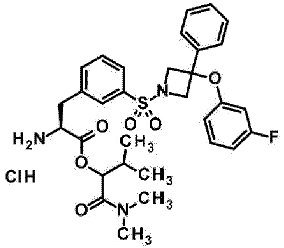 | ¹H-NMR (DMSO-d₆) δ: 0.71-0.89 (6H, m), 1.97-2.08 (1H, m), 2.84 (3H, d, *J* = 5.6 Hz), 3.04 (3H, d, *J* = 7.6 Hz), 3.19-3.30 (2H, m), 4.07-4.14 (2H, m), 4.33-4.52 (3H, m), 5.07-5.19 (1H, m), 6.24-6.30 (2H, m), 6.68-6.74 (1H, m), 7.12-7.20 (1H, m), 7.27-7.37 (5H, m), 7.60-7.69 (1H, m), 7.74-7.93 (3H, m), 8.56-8.74 (3H, m). MS (m/z): 598.3 [M+H]⁺. |
| 81 | 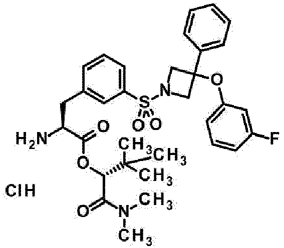 | ¹H-NMR (CD₃OD) δ: 7.85 (1H, d, J = 8.3 Hz), 7.81 (1H, s), 7.74 (1H, d, J = 7.8 Hz), 7.66 (1H, t, J = 7.6 Hz), 7.34-7.25 (5H, m), 7.10-7.04 (1H, m), 6.61-6.56 (1H, m), 6.19-6.12 (2H, m), 5.25 (1H, s), 4.49 (1H, t, J = 6.6 Hz), 4.33-4.29 (2H, m), 4.17-4.12 (2H, m), 3.43-3.20 (2H, m), 3.17 (3H, s), 2.95 (3H, s), 0.99 (9H, s). |
| 82 | 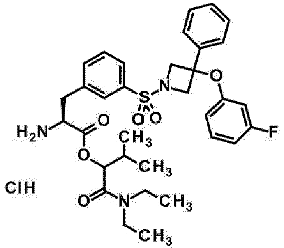 | ¹H-NMR (CD₃OD) δ: 7.87 (1H, s), 7.85 (1H, d, J = 7.8 Hz), 7.75 (1H, d, J = 7.8 Hz), 7.65 (1H, t, J = 7.8 Hz), 7.32-7.27 (5H, m), 7.10-7.02 (1H, m), 6.59 (1H, t, J = 7.1 Hz), 6.16 (2H, t, J = 10.7 Hz), 5.21 (1H, d, J = 5.4 Hz), 4.46 (1H, t, J = 6.8 Hz), 4.30 (2H, dd, J = 9.3, 4.4 Hz), 4.14 (2H, dd, J = 9.3, 3.4 Hz), 3.67-3.12 (6H, m), 2.17-2.09 (1H, m), 1.25 (3H, t, J = 7.1 Hz), 1.11 (3H, t, J = 6.8 Hz), 0.99 (3H, d, J = 6.8 Hz), 0.90 (3H, d, J = 6.8 Hz). MS (m/z): 626 (M+H)⁺. |

**[Table 2-23]**

| | | |
|---|---|---|
| 83 | 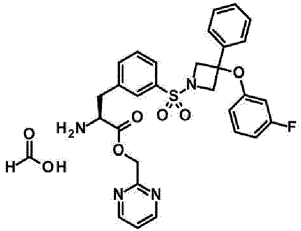 | ¹H-NMR (CD₃OD) δ: 8.80-8.72 (2H, m), 7.92-7.22 (10H, m), 7.09-7.02 (1H, m), 6.57 (1H, q, J = 8.8 Hz), 6.18-6.11 (2H, m), 5.42-5.28 (2H, m), 4.32-4.04 (5H, m), 3.51-3.16 (2H, m). MS (m/z): 563 (M+H)⁺. |
| 84 | 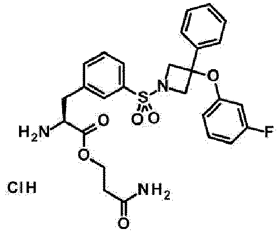 | ¹H-NMR (CD₃OD) δ: 7.88-7.83 (1H, m), 7.79 (1H, br s), 7.70-7.64 (2H, m), 7.36-7.25 (5H, m), 7.10-7.04 (1H, m), 6.62-6.56 (1H, m), 6.16 (2H, t, J = 9.0 Hz), 4.44-4.37 (2H, m), 4.36-4.29 (3H, m), 4.13 (2H, d, J = 9.3 Hz), 3.32-3.20 (2H, m), 2.57-2.52 (2H, m). MS (m/z): 542 (M+H)⁺. |
| 85 | 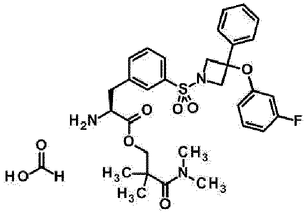 | ¹H-NMR (CD₃OD) δ: 7.92-7.55 (4H, m), 7.35-7.22 (5H, m), 7.09-7.02 (1H, m), 6.58 (1H, t, J = 8.1 Hz), 6.16 (2H, d, J = 8.3 Hz), 4.33-4.04 (7H, m), 3.39-2.87 (8H, m), 1.31-1.14 (6H, m). MS (m/z): 598 (M+H)⁺. |
| 86a | 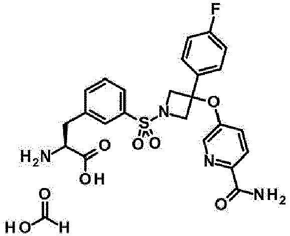 | ¹H-NMR (DMSO-d₆) δ: 7.91-7.85 (2H, m), 7.82-7.77 (2H, m), 7.72-7.68 (2H, m), 7.59-7.54 (1H, m), 7.51-7.48 (1H, m), 7.36-7.30 (2H, m), 7.19-7.13 (2H, m), 7.02-6.97 (1H, m), 4.45-4.41 (2H, m), 4.41 (2H, t, *J* = 9.8 Hz), 4.20 (2H, dd, *J* = 9.8, 4.9 Hz), 3.50 (1H, t, *J* = 6.1 Hz), 3.21 (1H, dd, *J* = 14.1, 5.5 Hz), 3.01 (1H, dd, *J* = 14.1, 7.4 Hz). MS (m/z): 515 (M+H)⁺. |

**[Table 2-24]**

| | | |
|---|---|---|
| 86b | 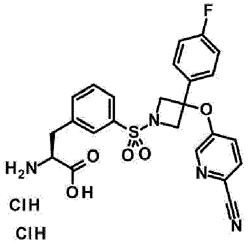 | ¹H-NMR (CD₃OD) δ: 8.39 (1H, d, *J* = 3.1 Hz), 7.87-7.83 (2H, m), 7.71-7.63 (2H, m), 7.54-7.47 (3H, m), 7.36-7.31 (1H, m), 6.60-6.56 (2H, m), 4.61 (2H, dd, *J* = 9.5, 6.4 Hz), 4.31 (1H, t, *J* = 6.7 Hz), 4.20 (2H, dd, *J* = 9.2, 5.5 Hz), 3.35 (1H, dd, *J* = 14.4, 6.4 Hz), 3.25 (1H, dd, *J* = 14.4, 7.1 Hz). MS (m/z): 497 (M+H)⁺. |
| 87 | 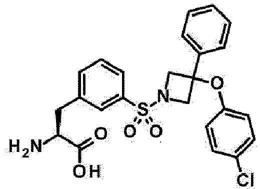 | ¹H-NMR (CD₃OD) δ: 7.84 (1H, s), 7.79 (1H, d, J = 7.8 Hz), 7.69 (1H, d, J = 7.3 Hz), 7.61 (1H, t, J = 7.8 Hz), 7.33-7.22 (5H, m), 7.06 (2H, d, J = 8.8 Hz), 6.38 (2H, d, J = 8.8 Hz), 4.27 (2H, d, J = 9.3 Hz), 4.13 (2H, d, J = 8.3 Hz), 3.81-3.76 (1H, m), 3.38-3.05 (2H, m). |
| 88 | 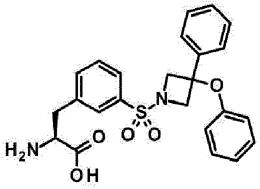 | ¹H-NMR (DMSO-d₆) δ: 2.94 (1H, dd, J = 13.9, 7.7 Hz), 3.16-3.24 (1H, m), 4.09 (2H, dd, J = 9.7, 3.6 Hz), 4.31 (2H, d, J = 9.8 Hz), 6.44 (2H, dd, J = 8.7, 0.9 Hz), 6.86 (1H, t, J = 7.4 Hz), 7.09-7.16 (2H, m), 7.23-7.34 (5H, m), 7.58 (1H, t, J = 7.8 Hz), 7.67-7.73 (2H, m), 7.78 (1H, br s), signal of one proton was overlapped with H₂O signal. MS (m/z) : 453 (M+H)⁺. |
| 89 | 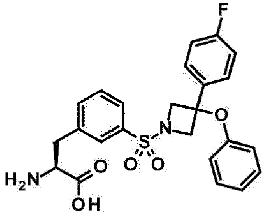 | ¹H-NMR (CD₃OD) δ: 7.83 (1H, s), 7.78 (1H, d, *J* = 7.8 Hz), 7.69-7.59 (2H, m), 7.35 (2H, dd, *J* = 8.8, 5.4 Hz), 7.08 (2H, t, *J* = 7.8 Hz), 7.03 (2H, t, *J* = 8.8 Hz), 6.84 (1H, t, *J* = 7.6 Hz), 6.37 (2H, d, *J* = 8.3 Hz), 4.26 (2H, dd, *J* = 9.3, 2.9 Hz), 4.13 (2H, d, *J* = 9.3 Hz), 3.80-3.75 (1H, m), 3.36-3.03 (2H, m). MS (m/z): 469 (M-H)⁻. |
| 90 | 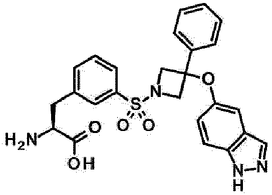 | ¹H-NMR (DMSO-d6) δ: 2.91-2.97 (m, 1H), 3.20-3.24 (m, 2H), 4.12 (m, 2H), 4.31 (d, J=10.0 Hz, 2H), 6.47 (d, J=2.0 Hz, 1H), 6.73 (dd, J=2.4, 8.8 Hz, 1H), 7.25-7.37 (m, 6H), 7.56 (t, J=7.6 Hz, 1H), 6.67-6.70 (m, 2H), 7.77 (s, 2H), 12.92-12.96 (m, 1H). MS (m/z): 493 (M+H)⁺. |

**[Table 2-25]**

| | | |
|---|---|---|
| 91 | 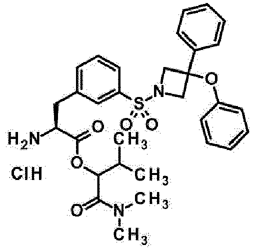 | ¹H NMR (DMSO-D₆) δ: 8.62-8.38 (3H, m), 7.92-7.60 (4H, m), 7.38-7.24 (5H, m), 7.17-7.09 (2H, m), 6.90-6.83 (1H, m), 6.45-6.39 (2H, m), 5.22-5.09 (1H, m), 4.59-4.46 (1H, m), 4.38-4.29 (2H, m), 4.13-4.04 (2H, m), 3.33-3.12 (2H, m), 3.09-3.00 (3H, m), 2.90-2.81 (3H, m), 2.10-1.97 (1H, m), 0.94-0.68 (6H, m). MS (m/z): 580 (M+H)⁺. |
| 92 | 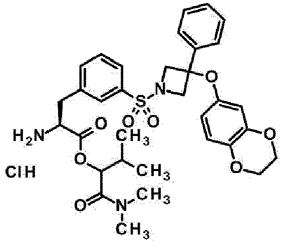 | ¹H-NMR (DMSO-D₆) δ: 8.67-8.40 (3H, m), 7.93-7.71 (3H, m), 7.71-7.61 (1H, m), 7.39-7.24 (5H, m), 6.63-6.56 (1H, m), 5.92-5.85 (2H, m), 5.21-5.09 (1H, m), 4.57-4,49 (1H, m), 4.31-4.23 (2H, m), 4.15-4.08 (4H, m), 4.07-4.01 (2H, m), 3.34-3.13 (2H, m), 3.07-3.02 (3H, m), 2.88-2.82 (3H, m), 2.10-1.97 (1H, m), 0.93-0.69 (6H, m). MS (m/z): 638 (M+H)⁺. |
| 93-1 | 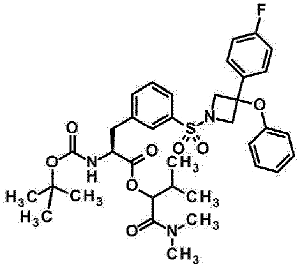 | ¹H-NMR (CDCl₃) δ: 7.75-7.22 (6H, m), 7.14-7.08 (2H, m), 7.03-6.96 (2H, m), 6.92-6.86 (1H, m), 6.39-6.33 (2H, m), 5.10-4.39 (3H, m), 4.19-4.13 (4H, m), 3.44-2.95 (8H, m), 2.26-2.14 (1H, m), 1.42-1.38 (9H, m), 1.09-0.97 (6H, m). |
| 93 | 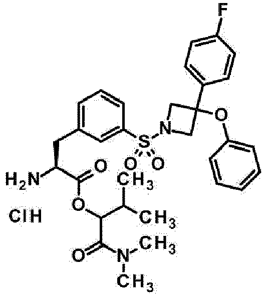 | ¹H-NMR (CD₃OD) δ: 7.89-7.65 (4H, m), 7.42-7.35 (2H, m), 7.15-7.03 (4H, m), 6.87 (1H, t, *J* = 7.4 Hz), 6.41-6.36 (2H, m), 5.31-5.20 (1H, m), 4.52-4.45 (1H, m), 4.33-4.28 (2H, m), 4.184.12 (2H, m), 3.48-3.23 (2H, m), 3.16-3.12 (3H, m), 2.99-2.95 (3H, m), 2.22-2.08 (1H, m), 1.02-0.89 (6H, m). MS: m/z 598 [M+H]⁺ |

**[Table 2-26]**

| | | |
|---|---|---|
| 94 | 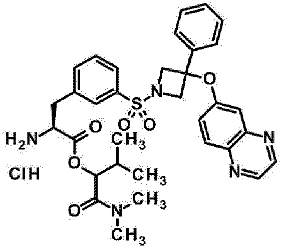 | ¹H-NMR (DMSO-D₆) δ: 8.80-8.73 (2H, m), 8.58-8.39 (3H, m), 8.01-7.59 (5H, m), 7.42-7.25 (6H, m), 6.65-6.59 (1H, m), 5.20-5.07 (1H, m), 4.58-4.45 (3H, m), 4.29-4.19 (2H, m), 3.33-3.09 (2H, m), 3.08-2.98 (3H, m), 2.89-2.80 (3H, m), 2.07-1.96 (1H, m), 0.91-0.67 (6H, m). MS (m/z): 632 (M+H)⁺. |
| 95 | 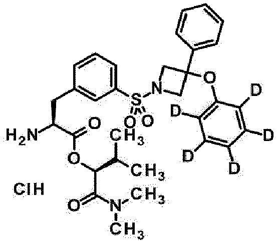 | ¹H-NMR (CD₃OD) δ: 7.86-7.82 (2H, m), 7.77-7.72 (1H, m), 7.67-7.62 (1H, m), 7.35-7.23 (5H, m), 5.27 (1H, d, *J* = 5.9 Hz), 4.46 (1H, t, *J* = 6.6 Hz), 4.28 (2H, dd, *J* = 9.0, 3.2 Hz), 4.13 (2H, dd, *J* = 93, 2.4 Hz), 3.45-3.20 (2H, m), 3.11 (3H, s), 2.95 (3H, s), 2.19-2.09 (1H, m), 1.00-0.82 (6H, m). MS (m/z): 585 (M+H)⁺. |
| 96 | 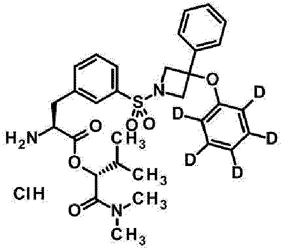 | ¹H-NMR (CD₃OD) δ: 7.87-7.82 (1H, m), 7.75-7.73 (1H, m), 7.67 (2H, d, *J* = 4.9 Hz), 7.37-7.23 (5H, m), 5.18 (1H, d, *J* = 5.9 Hz), 4.45 (1H, t, *J* = 6.8 Hz), 4.28 (2H, d, *J* = 9.3 Hz), 4.12 (2H, dd, *J* = 9.3, 2.9 Hz), 3.34-3.20 (2H, m), 3.10 (3H, s), 2.93 (3H, s), 2.14-2.06 (1H, m), 0.91-0.84 (6H, m). MS (m/z): 585 (M+H)⁺. |
| 97 | 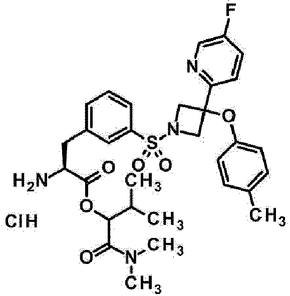 | ¹H-NMR (DMSO-D₆) δ: 8.63-8.41 (4H, m), 7.90-7.55 (5H, m), 7.38-7.30 (1H, m), 6.97-6.89 (2H, m), 6.32-6.24 (2H, m), 5.21-5.08 (1H, m), 4.57-4.43 (3H, m), 4.11-4.03 (2H, m), 3.34-3.11 (2H, m), 3.08-3.01 (3H, m), 2.89-2.81 (3H, m), 2.14 (3H, s), 2.10-1.96 (1H, m), 0.92-0.70 (6H, m). MS (m/z): 613 (M+H)⁺. |

**[Table 2-27]**

| | | |
|---|---|---|
| 98 | 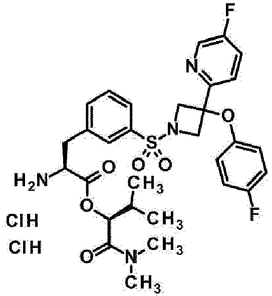 | ¹H-NMR (CD₃OD) δ: 8.37 (1H, d, *J* =2.5Hz), 7.88-7.83 (2H, m), 7.78-7.74 (1H, m), 7.66 (1H, t, *J* = 7.7 Hz), 7.52-7.46 (1H, m), 7.39-7.34 (1H, m), 6.90-6.83 (2H, m), 6.42-6.37 (2H, m), 5.31 (1H, d, *J* = 5.5 Hz), 4.55 (2H, d, *J* = 9.2 Hz), 4.51 (1H, t, *J* = 6.4 Hz), 4.16 (2H, dd, *J* = 9.2, 3.7 Hz), 3.50-3.24 (2H, m), 3.15 (3H, s), 2.99 (3H, s), 2.23-2.14 (1H, m), 1.02-0.91 (6H, m). MS: m/z 617 [M+H]⁺ |
| 99 | 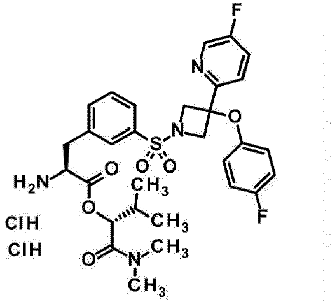 | ¹H-NMR (CD₃OD) δ: 8.38 (1H, d, *J* = 2.5 Hz), 7.88-7.83 (1H, m), 7.78-7.75 (1H, m), 7.71-7.67 (2H, m), 7.53-7.47 (1H, m), 7.39-7.34 (1H, m), 6.90-6.83 (2H, m), 6.42-6.36 (2H, m), 5.22 (1H, d, *J* = 6.1 Hz), 4.55 (2H, dd, *J* = 9.2,4.9 Hz), 4.48 (1H, t, *J* = 7.1 Hz), 4.15 (2H, dd, *J* = 9.2, 3.7 Hz), 3.37-3.26 (2H, m), 3.14 (3H, s), 2.96 (3H, s), 2.17-2.08 (1H, m), 0.95-0.88 (6H, m). MS: m/z 617 [M+H]⁺ |

**[Table 3]**

| Structure |
|---|
| 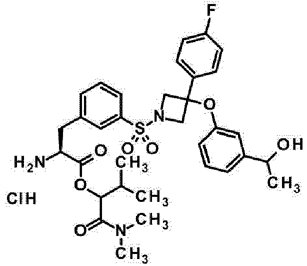 |
| 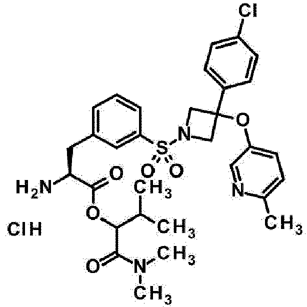 |

### [Test example 1] Effect of test substance on incorporation of ³H-thymidine into activated T cells

Effect of a test substance on incorporation of ³H-thymidine was evaluated using T cells derived from the mouse spleen. The spleen was excised out from a C57BL/6J mouse euthanized, cut into pieces, ground on a cell strainer (40 µm mesh) and centrifuged to collect spleen cells. CD4+ T cells were isolated from the spleen cells by use of EasySep Mouse CD4+ T cell isolation kit (STEMCELL Technologies). CD4+ T cells were seeded to a 96-well round-bottom plate. Test substance dissolved in DMSO (dimethyl sulfoxide) or vehicle (DMSO) was added to culture medium and Dynabeads Mouse T-Activator CD3/CD28 (Thermo Fisher Scientific Inc.) was added. Culture was carried out at 37°C in 5% CO₂. After culture was carried out for 24 hours, ³H-thymidine (PerkinElmer) diluted with the culture medium was added to individual wells. Culture was further carried out overnight at 37°C in 5% CO₂. In order to measure ³H-thymidine incorporated in nuclei of the cells, the cells were collected on a filter plate UniFilter-96 GF/B (PerkinElmer) by use of a FilterMate Universal Harvester (PerkinElmer). After the filter plate was placed in a dryer and allowed to stand still for 2 hours or more, scintillation cocktail MicroScint-O (PerkinElmer) was added and radioactivity was measured by TopCount NXT (PerkinElmer). With the radioactivity of a group not containing a test substance being specified as 100% and the radioactivity of a group not activated being specified as 0%, relative radioactivity values of individual wells to which the test substance was added (proliferation rate, % of vehicle) were calculated. The values of "% of vehicle" were entered in analysis software Prism (Version 4.03, GraphPad Software). "Nonlinear regression Log (inhibitor) vs reaction variable tilt" was selected and sigmoid type-fitting (Bottom = 0, Top = 100) was carried out to calculate 50 % inhibition concentration (IC₅₀). The results are shown in Table 4. The compound of the present invention suppresses ³H-thymidine incorporation in activated T cells. Since incorporation of ³H-thymidine indicates proliferation of cells, the results demonstrate that the compound of the present invention suppresses proliferation of activated T cells.

**[Table 4]**

| Example No. | IC₅₀ (*µ* M) | Example No. | IC₅₀ (*µ* M) | Example No. | IC₅₀ (*µ* M) |
|---|---|---|---|---|---|
| 1 | 0.020 | 19 | 0.030 | 49 | 0.036 |
| 2 | 0.012 | 20 | 0.0088 | 50 | 0.024 |
| 3 | 0.035 | 21 | 0.0037 | 52 | 0.0066 |
| 4 | 0.012 | 22 | 0.0048 | 53 | 0.0087 |
| 5 | 0.030 | 24 | 0.012 | 54 | 0.0048 |
| 6 | 0.0066 | 31 | 0.021 | 55 | 0.0078 |
| 7 | 0.016 | 32 | 0.018 | 56 | 0.24 |
| 8 | 0.013 | 34 | 0.0033 | 57 | 0.016 |
| 9 | 0.0076 | 35 | 0.024 | 58 | 0.016 |
| 10 | 0.010 | 36 | 0.019 | 59 | 0.018 |
| 11 | 0.026 | 37 | 0.024 | 60 | 0.014 |
| 12 | 0.021 | 38 | 0.017 | 61 | 0.0099 |
| 13 | 0.0091 | 39 | 0.014 | 86a | 0.023 |
| 14 | 0.0077 | 43 | 0.018 | 86b | 0.017 |
| 15 | 0.014 | 45 | 0.023 | 87 | 0.019 |
| 16 | 0.010 | 46 | 0.031 | 88 | 0.015 |
| 17 | 0.023 | 47 | 0.034 | 90 | 0.0034 |
| 18 | 0.014 | 48 | 0.022 | | |

### [Test Example 2] Effect of test substance on IFN-α production in mouse spleen cells

IFN-α production by stimulation with TLR9 ligand CpG-ODN D35 activating pDC was evaluated. Spleen cells were prepared from a C57BL/6J mouse euthanized in the same manner as in Test Example 1 and seeded to a 96-well round-bottom plate. Test substance dissolved in DMSO or vehicle (DMSO) was added to culture medium. After preincubation was carried out for 30 minutes, CpG-ODN D3 (final concentration 1 µM) was added. Culture was carried out at 37°C in 5% CO₂. After culture was carried out for 24 hours, the culture supernatant was recovered. The concentration of IFN-α in the supernatant was measured by ELISA kit (Mouse IFN-alpha Platinum ELISA Kit, eBioscience). With the IFN-α production of a group not containing a test substance being specified as 100% and the IFN-α production of a group not activated being specified as 0%, relative values of IFN-α production of individual wells (IFN-α production rate, % of vehicle) were calculated. The values of "% of vehicle" of IFN-α production were entered in analysis software Prism (Version 5.0, GraphPad Software). "Nonlinear regression Log (inhibitor) vs reaction variable tilt" was selected and sigmoid type-fitting (Bottom = 0, Top = 100) was carried out to form a concentration reaction curve (graph) to calculate 50 % inhibition concentration (IC₅₀). The results are shown in Figure 1. The compound of Example 1 showed a concentration-dependent suppressive action and IC₅₀ of the compound was 13 nM.

### [Test Example 3] Effect of test substance in mouse acute GVHD model

A mouse acute GVHD (graft-versus-host disease) model was prepared in accordance with a publication (Puliaev R et al., Differential requirement for IFN-gamma in CTL maturation in acute murine graft-versus-host disease; J Immunol. 173, 910-9 (2004)). Spleen cells were prepared from a donor mouse (C57BL/6N) euthanized, suspended in PBS without hemolyzing/removing red blood cells and administered to a host mouse, BDF1, from the tail vein (number of the cells transplanted were controlled so as to be as equal as 7 to 9 × 10⁷ cells/0.2 mL/mouse between individuals in the same test). To mice of Normal group, 0.2 mL of PBS was administered in place of the cells. Test substance was mushed in an agate mortar and suspended in a 0.5% aqueous methyl cellulose 400 solution (0.5 w/v% Methyl Cellulose 400 Solution, sterilized, WAKO, 133-17815, hereinafter referred to as "0.5% MC", herein) to prepare a solution (test substance solution) for administration. To mice of Vehicle group, 0.5% MC was administered. To the host mouse to which cells were transplanted, test substance solution was forcibly and orally administered twice a day continuously from the morning of Day 0 (when cells were transferred) to the last day of the experiment by use of an oral sonde. On Day 7, after blood sampling, the plasma was centrifugally collected and the concentration of IFN-γ in the plasma was measured by AlphaLISA immunoassay kit (Mouse Interferon-gamma (mIFN-y) Kit, PerkinElmer, AL501C). The graph of Figure 2 shows the results when the compound of Example 25 was orally administered at a dose of 3 and 10 mg/kg twice a day (BID). Whether the data of Normal group and Vehicle group differ or not was determined by Student's t-test. Symbol "###" indicates that p value is 0.001 or less. Whether data of Vehicle group and a test substance administration group differ or not was analyzed by a parametric multiple comparison test, Dunnett test. Symbol "***" indicates that p value is 0.001 or less.

The compound of Example 25 suppressed production of IFN-γ in the plasma in a dose-dependent manner. A significant effect was exhibited at a dose of 10 mg/kg (BID) or more.

### [Test Example 4] Effect of test substance on kidney disorder of mouse acute GVH model

A mouse chronic GVH (graft-versus-host) model was prepared in accordance with a publication (Via CS. Advances in lupus stemming from the parent-into-Fl model; Trends Immunol. 31, 236-45 (2010)). Spleen cells were prepared from a donor mouse (DBA2 mouse) euthanized, suspended in PBS without hemolyzing/removing red blood cells and administered to a host mouse, BDF1 from the tail vein, twice (Day 0 and Day 4) (at a dose of 1× 10⁸ cells/0.2 mL/mouse). To mice of Normal group, 0.2 mL of PBS was administered in place of the cells. A test substance was mushed in an agate mortar and suspended in 0.5% MC to prepare a solution (test substance solution) for administration. To mice of Vehicle group, 0.5% MC was administered. To the host mouse to which cells were transplanted, test substance solution was forcibly and orally administered twice a day continuously from Day 4 (day of second transplantation of cells) to the last day of the experiment by use of an oral sonde. Day 28 or 32, mice were allowed to eat freely, and urine was collected by use of a metabolic cage. The concentration of urinary protein was measured by use of a test kit (Micro TP-test Wako, WAKO, #465-50601). The concentration of urine creatinine was measured by a creatinine test kit (Lab Assay Creatinine, WAKO, #290-65901). The ratio of urine protein and creatinine (concentration) was calculated with respect to each individual. Whether the data of Normal group and Vehicle group differ or not was determined by Wilcoxon test (nonparametric, comparison between two groups). Whether the data of Vehicle group and a test substance administration group differ or not was analyzed by Shirley-William's test (nonparametric test, multiple comparison test based on dose response).

### [Test Example 5] Effect of test substance in ConA induced mouse cytokine production model

In this test, mice were treated with concanavalin A (ConA) to activate lymphocytes and the medicinal effect of a test substance was evaluated based on the production of IFN-γ. A system in which cytokine production based on expression of LAT1 is induced, by treating mice with ConA twice at optimal timing, was established.

To female Balb/c mice of 7 weeks old, a ConA solution (0.4 mg/mL, Concanavalin A, WAKO, 037-08774) prepared with PBS (D-PBS (-), WAKO, 049-29793) was administered (4 mg/kg, twice at an interval of 16 hours) through the tail vein. Four hours later, under anesthesia by isoflurane inhalation, blood was taken from the posterior vena cava of each of the mice. The blood was centrifuged to collect the plasma. The concentration of IFN-γ in the in plasma was measured by AlphaLISA immunoassay kit (Mouse Interferon-gamma (mIFN-y) Kit, PerkinElmer, AL501C). Test substance was dissolved in 0.5% MC or mushed in an agate mortar, suspended in 0.5% MC to prepare a solution to be administered (1 or 3 mg/mL) and orally administered one hour before the second treatment with ConA so as to be 10 mL/kg (10 or 30 mg/kg. To Vehicle group, 0.5% MC was administered similarly so as to be 10 mL/kg).

Test substances were evaluated. The results are shown in Table 5. Suppression rates (%) were calculated in accordance with the following expression.

Suppression rate (%) = 100-[(average value of test substance administration group - average value of Normal group)/(average value of Vehicle group - average value of Normal group) × 100]

Whether the data of two groups, Normal group and Vehicle group, differ or not was determined by Student's or Welch's t test. Whether the data of Vehicle group and a test substance administration group are different or not was analyzed by parametric multiple comparison test, Dunnett test. The compounds (test substances) suppressed IFN-γ production in a dose-dependent manner.

**[Table 5]**

| | **Percent suppression (%)** | |
|---|---|---|
| Example No. | 10mg/kg | 30mg/kg |
| 25 | 31 | 51 |
| 27 | 47 | 58 |
| 62 | 43 | 54 |
| 69 | 33 | 44 |
| 91 | 55 | 53 |
| 93 | 46 | 54 |

### [Test Example 6] Pharmacokinetic test of prodrug in mice

Compounds of Examples 31, 32 and 80 (prodrug forms of an active substance, the compound of Example 1) and the compound of Example 33 (prodrug form of an active substance, the compound of Example 18) were orally administered to mice. Thereafter, the concentrations of the active substances in the plasma were measured to calculate various PK parameters in accordance with the following method.

### (Method)

To C57BL/6J mice (female, not fasted), compounds of Examples were orally administered in the following conditions and blood was sampled.

Dose (number of subjects, dose vehicle): 10 mg/kg (n = 2, 0.5% methyl cellulose)
The time of sampling blood: 0.25, 0.5, 1, 2, 4, 7 and 24 h

After blood was sampled, plasma was obtained by centrifugation. To 10 µL of the plasma, the same volume of a 50 v/v% acetonitrile solution was added, and thereafter and an internal standard (IS) solution (200 µL) was added. After stirring, the whole amount of the sample was filtered by MultiScreen Solvinert Filter Plate. The filtrate was collected and used as a measurement sample. To blank plasma, 50 v/v% acetonitrile solution of a test substance prepared so as to be 0.0005, 0.002, 0.005, 0.02, 0.05, 0.2, 0.5, 2, 5, 20 or 50 µM was added. The resultant solutions were subjected to the same treatment as above and used as samples for preparing a calibration curve.

From the measured concentration values of the compounds serving as an active substance in the plasma, the following PK parameters were calculated by use of Moment (Microsoft Excel).

AUClast: area under the curve of plasma concentration versus time up to a quantitable final point Cmax: maximum plasma concentration
Table 6 shows PK parameters of an active substance (compound of Example 1) after PO administration of the compounds (prodrug forms) of Example 31, 32 or 80 to mice at a dose of 10 mg/kg.

**[Table 6]**

| **Example No.** | AUClast (ng· h/mL) | Cmax (ng/mL) |
|---|---|---|
| 31 | 3625. 1 | 2044.4 |
| 32 | 3344.9 | 1253. 9 |
| 80 | 3104.2 | 1564. 5 |

Table 7 shows PK parameters of an active substance (compound of Example 18) after PO administration of the compound (prodrug forms) of Example 33, to mice at a dose of 10 mg/kg.

**[Table 7]**

| **Example No.** | AUClast (ng· h/mL) | Cmax (ng/mL) |
|---|---|---|
| 33 | 647.0 | 721.8 |

As described above, it was demonstrated that the prodrug forms of the present invention have sufficient bioavailability of an active substance and have satisfactory PK profiles.

### Industrial Applicability

A compound represented by formula (I) of the present invention or a pharmaceutically acceptable salt thereof is useful as a therapeutic or prophylactic agent for a disease the symptom of which can be prevented, ameliorated or mitigated by suppressing activation of T cells and pDC, and production of IFN-α**.** Examples of the disease include systemic lupus erythematosus; a renal disease associated with systemic lupus erythematosus, central nervous system disorder, lung disorder or vasculitis; polymyositis; ulcerative colitis; Sjogren's syndrome; rejection associated with organ transplantation, graft-versus-host disease (GVHD); dry eye; rheumatoid arthritis; Crohn's disease; psoriasis; interstitial cystitis; dermatomyositis; atopic dermatitis; systemic scleroderma; and type I diabetes. Preferably, as the disease, SLE; a renal disease associated with systemic lupus erythematosus, central nervous system disorder, lung disorder or vasculitis; polymyositis; ulcerative colitis; Sjogren's syndrome; and graft-versus-host disease (GVHD) or psoriasis is mentioned. Particularly preferably, e.g., SLE and a renal disease associated with systemic lupus erythematosus (lupus nephritis) are mentioned. A compound represented by formula (I) of the present invention or a pharmaceutically acceptable salt thereof suppresses immune cell function by inhibiting proliferation of activated T cells and production of IFN-α, thereby producing an effect on treatment and prevention of autoimmune diseases mentioned above.

## Claims

1. A compound represented by formula (I): 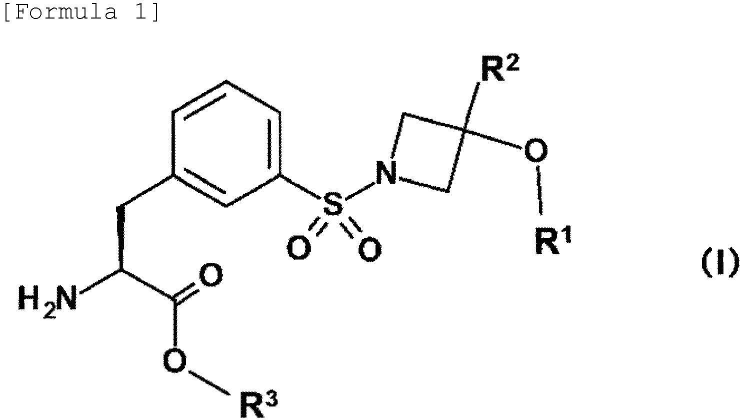 wherein
R¹ represents a phenyl group, a monocyclic aromatic heterocyclic group or a dicyclic heterocyclic group, wherein
the monocyclic aromatic heterocycle is a 5 to 6-membered aromatic heterocycle having 1 to 4 heteroatoms in the ring independently selected from oxygen atoms, sulfur atoms and nitrogen atoms,
the dicyclic heterocyclic group is represented by any one of the following formulas (R¹-1A) to (R¹-1F): 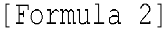 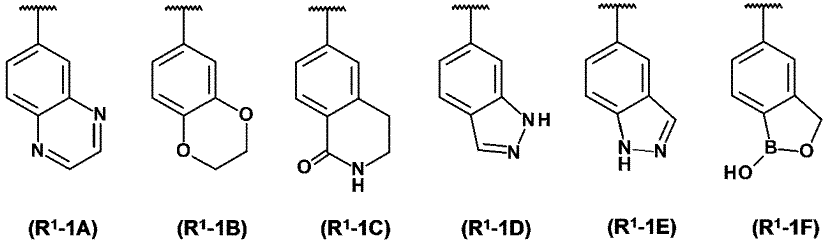 and
the phenyl group, monocyclic aromatic heterocyclic group or dicyclic heterocyclic group optionally has 1 to 5 substituents independently selected from substituent group a;
R² represents a phenyl group or a monocyclic aromatic heterocyclic group, wherein
the monocyclic aromatic heterocycle is a 5 to 6-membered aromatic heterocycle having 1 to 4 heteroatoms in the ring independently selected from oxygen atoms, sulfur atoms and nitrogen atoms, and
the phenyl group or monocyclic aromatic heterocyclic group optionally has 1 to 5 substituents independently selected from substituent group b;
R³ represents a hydrogen atom,
a group represented by formula (A): 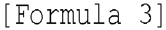 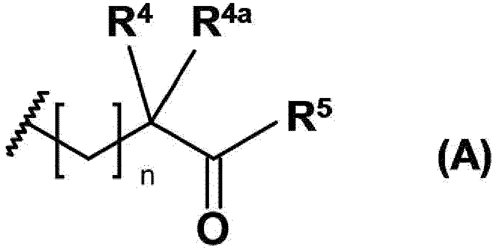 (wherein
n represents an integer 0 or 1,
R⁴ represents a hydrogen atom, a C₁-C₆ alkyl group or a C₃-C₆ cycloalkyl group,
R^{4a} represents a hydrogen atom, a C₁-C₆ alkyl group or a C₃-C₆ cycloalkyl group,
R⁵ represents a C₁-C₆ alkyl group, an amino group, a mono (C₁-C₆ alkyl) amino group, a di (C₁-C₆ alkyl) amino group or a morpholino group, wherein the C₁-C₆ alkyl group, amino group, mono (C₁-C₆ alkyl)amino group, di(C₁-C₆ alkyl)amino group or morpholino group optionally has one or two substituents independently selected from substituent group c),
a group represented by formula (B): 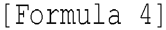 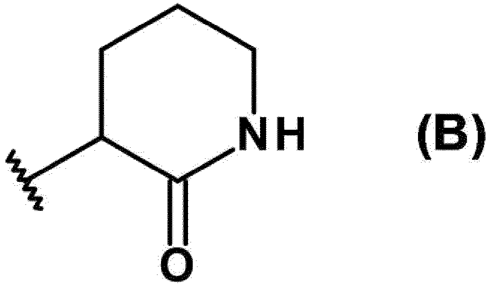 or
a C₁-C₆ alkyl group substituted with one pyrimidinyl group;
Substituent group a: a halogen atom, a C₁-C₆ alkyl group, a C₁-C₆ alkyl group substituted with one to three halogen atoms, a C₁-C₆ alkyl group substituted with one carbamoyl group, a C₁-C₆ alkyl group substituted with one hydroxy group, a cyano group, a carbamoyl group, a deuterium atom, a phenyl group (wherein the phenyl group is optionally substituted with one to three substituents independently selected from the group consisting of C₁-C₆ alkyl groups and halogen atoms), a hydroxy group, a C₁-C₆ alkoxy group, a C₁-C₆ alkoxy group substituted with one to three halogen atoms, a C₁-C₇ acyl group, a mono(C₁-C₆ alkyl)carbamoyl group, a di(C₁-C₆ alkyl)carbamoyl group, a (C₁-C₆ alkoxy)carbonyl group, a carboxy group, a mono (C₁-C₆ alkyl) amino group, di (C₁-C₆ alkyl) amino group and a B(OH)₂ group;
Substituent group b: a halogen atom, a deuterium atom and a C₁-C₆ alkyl group; and
Substituent group c: a C₁-C₆ alkoxy group and a hydroxy group;
or a pharmaceutically acceptable salt thereof.

2. The compound or a pharmaceutically acceptable salt thereof according to claim 1, wherein, in the formula (I),
R¹ is a phenyl group or a pyridyl group, wherein
the phenyl group or pyridyl group optionally has 1 to 5 substituents independently selected from substituent group al;
R² is a phenyl group or a pyridyl group, wherein
the phenyl group or pyridyl group optionally has one or two substituents independently selected from substituent group bl; and
R³ is a hydrogen atom or a group represented by formula (A), wherein
n represents an integer of 0,
R⁴ represents a hydrogen atom or a C₁-C₆ alkyl group,
R^{4a} represents a hydrogen atom, and
R⁵ represents a C₁-C₆ alkyl group, an amino group, a mono(C₁-C₆ alkyl) amino group or a di (C₁-C₆ alkyl) amino group, wherein the C₁-C₆ alkyl group, amino group, mono(C₁-C₆ alkyl)amino group or di (C₁-C₆ alkyl)amino group has no substituents;
Substituent group a1: a halogen atom, a C₁-C₃ alkyl group, a cyano group, a carbamoyl group and a deuterium atom; and
Substituent group b1: a halogen atom and a C₁-C₃ alkyl group.

3. The compound or a pharmaceutically acceptable salt thereof according to claim 2, wherein, in the formula (I), R³ is a group represented by formula (A), wherein
n represents an integer of 0,
R⁴ represents a hydrogen atom or a C₁-C₆ alkyl group,
R^{4a} represents a hydrogen atom, and
R⁵ represents a C₁-C₆ alkyl group, an amino group, a mono(C₁-C₆ alkyl) amino group or a di (C₁-C₆ alkyl) amino group, wherein the C₁-C₆ alkyl group, amino group, mono (C₁-C₆ alkyl) amino group or di (C₁-C₆ alkyl)amino group has no substituents.

4. The compound or a pharmaceutically acceptable salt thereof according to claim 2, wherein, in the formula (I), R³ is a group represented by formula (A), wherein
n represents an integer of 0,
R⁴ represents a C₃-C₆ alkyl group,
R^{4a} represents a hydrogen atom, and
R⁵ represents a mono(C₁-C₆ alkyl)amino group or a di (C₁-C₆ alkyl)amino group, wherein the mono(C₁-C₆ alkyl)amino group or di (C₁-C₆ alkyl) amino group has no substituents.

5. The compound or a pharmaceutically acceptable salt thereof according to claim 1, wherein, in the formula (I),
R¹ is a phenyl group, a pyridyl group or a dicyclic heterocyclic group, wherein
the phenyl group or pyridyl group has 1 to 5 substituents independently selected from substituent group a according to claim 1, and at least one of the substituents is independently selected from substituent group a2, and
the dicyclic heterocyclic group is represented by any one of formulas (R¹-1A) to (R¹-1F) and optionally has 1 to 5 substituents independently selected from substituent group a according to claim 1,
Substituent group a2: a C₁-C₆ alkyl group substituted with one hydroxy group, a hydroxy group, a C₁-C₆ alkoxy group, a C₁-C₆ alkoxy group substituted with one to three halogen atoms, a C₁-C₇ acyl group, a mono (C₁-C₆ alkyl)carbamoyl group, a di(C₁-C₆ alkyl)carbamoyl group, a mono (C₁-C₆ alkyl) amino group, a di(C₁-C₆ alkyl) amino group and a B(OH)₂ group.

6. The compound or a pharmaceutically acceptable salt thereof according to claim 5, wherein, in the formula (I),
R² is a phenyl group or a pyridyl group, wherein
the phenyl group or pyridyl group optionally has one or two substituents independently selected from substituent group b2,
Substituent group b2: a halogen atom and a C₁-C₃ alkyl group.

7. The compound or a pharmaceutically acceptable salt thereof according to claim 5 or 6, wherein, in the formula (I), R³ is a group represented by formula (A), wherein
n represents an integer of 0,
R⁴ represents a hydrogen atom or a C₁-C₆ alkyl group,
R^{4a} represents a hydrogen atom, and
R⁵ represents a C₁-C₆ alkyl group, an amino group, a mono(C₁-C₆ alkyl)amino group, a di(C₁-C₆ alkyl)amino group or a morpholino group, wherein the C₁-C₆ alkyl group, amino group, mono (C₁-C₆ alkyl)amino group, di(C₁-C₆ alkyl)amino group or morpholino group optionally has one or two substituents independently selected from substituent group c,
Substituent group c: a C₁-C₆ alkoxy group and a hydroxy group.

8. The compound or a pharmaceutically acceptable salt thereof according to claim 5 or 6, wherein, in the formula (I), R³ is a group represented by formula (A), wherein
n represents an integer of 0,
R⁴ represents a C₃-C₆ alkyl group,
R^{4a} represents a hydrogen atom, and
R⁵ represents a mono(C₁-C₆ alkyl)amino group, a di (C₁-C₆ alkyl)amino group or a morpholino group, wherein the mono(C₁-C₆ alkyl)amino group, di(C₁-C₆ alkyl)amino group or morpholino group has no substituents.

9. The compound or a pharmaceutically acceptable salt thereof according to claim 1, wherein, in the formula (I), R³ is a group represented by formula (A), wherein
n represents an integer of 0,
R⁴ represents a hydrogen atom or a C₁-C₆ alkyl group,
R^{4a} represents a hydrogen atom or a C₁-C₆ alkyl group, and
R⁵ represents a C₁-C₆ alkyl group, an amino group, a mono(C₁-C₆ alkyl)amino group, a di(C₁-C₆ alkyl)amino group or a morpholino group, wherein the C₁-C₆ alkyl group, amino group, mono(C₁-C₆ alkyl)amino group or di(C₁-C₆ alkyl)amino group has one or two substituents independently selected from substituent group c, and the morpholino group optionally has one or two substituents independently selected from substituent group c,
Substituent group c: a C₁-C₆ alkoxy group and a hydroxy group.

10. The compound or a pharmaceutically acceptable salt thereof according to claim 9, wherein, in the formula (I),
R¹ is a phenyl group or a pyridyl group, wherein
the phenyl group or pyridyl group optionally has 1 to 5 substituents independently selected from substituent group a3; and
R² is a phenyl group or a pyridyl group, wherein
the phenyl group or pyridyl group optionally has one or two substituents independently selected from substituent group b3),
Substituent group a3: a halogen atom, a C₁-C₃ alkyl group, a cyano group, a carbamoyl group and a deuterium atom; and
Substituent group b3: a halogen atom and a C₁-C₃ alkyl group.

11. A medicament comprising the compound or a pharmaceutically acceptable salt thereof according to any one of claims 1 to 10.

12. A suppressor of proliferation of activated T cells, comprising the compound or a pharmaceutically acceptable salt thereof according to any one of claims 1 to 10.

13. A suppressor of interferon α production, comprising the compound or a pharmaceutically acceptable salt thereof according to any one of claims 1 to 10.

14. A therapeutic or prophylactic agent for a disease a symptom of which is prevented, ameliorated or mitigated by suppressing proliferation of activated T cells or suppressing production of interferon α by activated pDC (plasmacytoid dendritic cells), comprising the compound or a pharmaceutically acceptable salt thereof according to any one of claims 1 to 10.

15. A therapeutic or prevention method for a disease a symptom of which is prevented, ameliorated or mitigated by suppressing proliferation of activated T cells or suppressing production of interferon α by activated pDC (plasmacytoid dendritic cells), comprising administering the compound or a pharmaceutically acceptable salt thereof according to any one of claims 1 to 10, to a patient.

16. The compound or a pharmaceutically acceptable salt thereof according to any one of claims 1 to 10 for use in treating or preventing a disease a symptom of which is prevented, ameliorated or mitigated by suppressing proliferation of activated T cells or suppressing production of interferon α by activated pDC (plasmacytoid dendritic cells).

17. The therapeutic or prophylactic agent according to claim 14, wherein the disease is an autoimmune disease.

18. The therapeutic or prevention method according to claim 15, wherein the disease is an autoimmune disease.

19. The compound or a pharmaceutically acceptable salt thereof according to claim 16, wherein the disease is an autoimmune disease.

20. A therapeutic or prophylactic agent for systemic lupus erythematosus; a renal disease associated with systemic lupus erythematosus, central nervous system disorder, lung disorder or vasculitis; polymyositis; ulcerative colitis; Sjogren's syndrome; graft-versus-host disease (GVHD) or psoriasis, comprising the compound or a pharmaceutically acceptable salt according to any one of claims 1 to 10.

21. A method for treating or preventing systemic lupus erythematosus; a renal disease associated with systemic lupus erythematosus, central nervous system disorder, lung disorder or vasculitis; polymyositis; ulcerative colitis; Sjogren's syndrome; graft-versus-host disease (GVHD) or psoriasis, comprising administering the compound or a pharmaceutically acceptable salt according to any one of claims 1 to 10 to a patient.

22. The compound or a pharmaceutically acceptable salt according to any one of claims 1 to 10 for use in treating or preventing systemic lupus erythematosus, a renal disease associated with systemic lupus erythematosus, central nervous system disorder, lung disorder or vasculitis; polymyositis; ulcerative colitis; Sjogren's syndrome; graft-versus-host disease (GVHD) or psoriasis.
